(19) Europäisches Patentamt
European Patent Office
Office européen des brevets

(11) Veröffentlichungsnummer: **0 272 534**
**A2**

# (12) EUROPÄISCHE PATENTANMELDUNG

(21) Anmeldenummer: **87118112.9**

(22) Anmeldetag: **08.12.87**

(51) Int. Cl.[4]: **C07C 87/457**, C07C 121/78, C07C 121/18, C07C 103/44, C07C 103/28, C07C 101/42, C07C 95/08, C07C 91/23, C07C 143/79, C07C 125/065

(30) Priorität: **22.12.86 DE 3643899**
**15.06.87 DE 3719924**

(43) Veröffentlichungstag der Anmeldung:
**29.06.88 Patentblatt 88/26**

(84) Benannte Vertragsstaaten:
**AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Junge, Bodo, Dr.**
**Spiekern 23**
**D-5600 Wuppertal 23(DE)**
Erfinder: **Richter, Bernd, Dr.**
**Am Katternbach 13**
**D-5060 Bergisch Gladbach 2(DE)**
Erfinder: **Glaser, Thomas, Dr.**
**Köslinerstrasse 21a**
**D-5064 Rösrath(DE)**
Erfinder: **Traber, Jörg, Dr.**
**Löwenburgstrasse 12**
**D-5204 Lohmar 21(DE)**
Erfinder: **Allen, George S., Prof. Dr.**
**Vanderbilt University Dpt. of Neurological Surgery**
**Nashville Tennessee 37232(US)**

## (54) 8-Substituierte 2-Aminotetraline.

(57) Neue 8-substituierte 2-Aminotetraline können aus den entsprechenden Aminotetralinen oder Tetralonen hergestellt werden. Sie können in Arzneimitteln verwendet werden.

EP 0 272 534 A2

# 8-Substituierte 2-Aminotetraline

Die Erfindung betrifft 8-substituierte 2-Aminotetraline, Verfahren zu ihrer Herstellung und ihre Verwendung in Arzneimitteln.

Aus der EP-A1 41 488 ist bekannt, daß 8-Hydroxy-2-alkylaminotetraline bzw. 8-Amino-2-dialkylaminotetraline auf das Zentralnervensystem wirken.

Es wurden nun neue 8-substituierte 2-Aminotetraline der allgemeinen Formel (I)

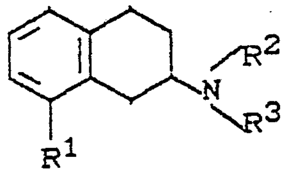

(I)

in welcher

$R^1$ -für Halogen, Cyano oder

-für eine Gruppe der Formel $-NR^4R^5$, $-COR^6$, $-(CH_2)_a-X$, $-O-(CH_2)_a-X$ oder $-CH=CH-(CH_2)_b-X$ steht,

worin

$R^4$ und $R^5$ gleich oder verschieden sind und

-Wasserstoff oder

-eine Gruppe der Formel $-COR^7$ oder $-SO_2R^8$ bedeuten,

wobei

$R^7$ -für Wasserstoff steht, oder

-für eine Gruppe $-NHR^9$ steht, oder

-für Alkoxy steht, oder

-für Aryl, Aryloxy, Aralkyl, Aralkoxy oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,

$R^8$ -für Cycloalkyl steht, oder

-für Alkyl steht, das durch Cyano, Halogen, Trifluormethyl, Trifluormethoxy oder Alkoxycarbonyl substituiert sein kann, oder

-für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder

-für eine Gruppe $-NR^{10}R^{11}$ steht,

worin

$R^{10}$ und $R^{11}$ gleich oder verschieden sind und Wasserstoff, Alkyl, Aryl oder Aralkyl bedeuten

und

$R^9$ -für Wasserstoff steht, oder

-für Cycloalkyl steht, oder

-für gegebenenfalls durch Cyano, Halogen, Trifluormethyl oder Trifluormethoxy substituiertes Alkyl steht, oder

-für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,

$R^6$ -Wasserstoff, Hydroxy, Amino, Alkoxy, Aryloxy oder Aralkoxy bedeutet,

a - eine Zahl 1 bis 10 bedeutet,

b - eine Zahl 0 bis 8 bedeutet

und

X - eine Gruppe der Formel $-NR^{12}R^{13}$, $-COR^{14}$, $-SO_2R^{15}$ oder $-OR^{16}$ bedeutet,

worin

$R^{12}$ und $R^{13}$ gleich oder verschieden sind und

-für Wasserstoff, Alkyl, Aryl oder Aralkyl stehen, wobei die Arylreste durch Halogen, Cyano, Alkyl, Alkoxy oder Trifluormethyl substituiert sein können,

oder

-für eine Gruppe der Formel $-COR^{14}$, $-SO_2R^{15}$ oder $-(CH_2)_c-NR^{12}R^{13}$ stehen,

$R^{14}$ -Wasserstoff bedeutet, oder
-eine Gruppe $-NHR^{17}$ bedeutet, oder
-Alkyl oder Alkoxy bedeutet, oder
-Aryl, Aryloxy, Aralkyl, Aralkoxy oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
$R^{15}$ -cycloalkyl bedeutet, oder
-Alkyl bedeutet, das durch Cyano, Halogen, Trifluormethyl, Trifluormethoxy oder Alkoxycarbonyl substituiert sein kann, oder
-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
-eine Gruppe $-NR^{12}R^{13}$ bedeutet,
wobei
$R^{12}$ und $R^{13}$ die oben angegebene Bedeutung haben,
$R^{16}$ - Wasserstoff, Alkyl, Aryl, Aralkyl oder eine Gruppe der Formel $-CONR^{12}R^{13}$ bedeutet,
$R^{17}$ -Wasserstoff bedeutet, oder
-Cycloalkyl bedeutet, oder
-gegebenenfalls durch Cyano, Halogen, Trifluormethyl oder Trifluormethoxy substituiertes Alkyl bedeutet, oder
-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
und
c - eine Zahl 1 bis 8 bedeutet,
oder wobei
$R^{12}$ und $R^{13}$ zusammen mit dem Stickstoffatom einen Ring der Reihe

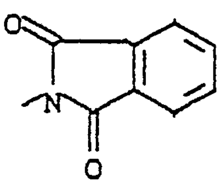 , 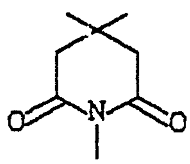 , 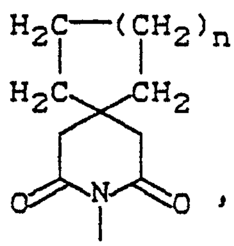 ,

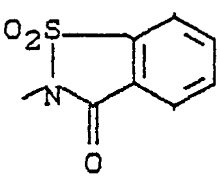 , 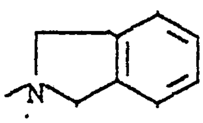 , 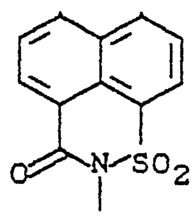 ,

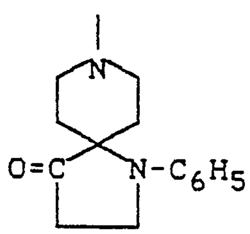 , 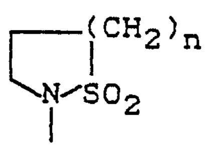 , 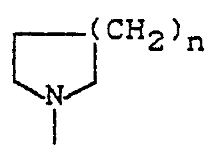

oder 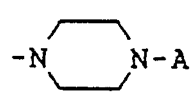 bilden,

worin
n - eine Zahl 1 oder 2 bedeutet

und
A - für Wasserstoff oder Cycloalkyl steht,
oder
-für Alkyl steht, das durch Halogen, Hydroxy, Amino, Alkylamino, Dialkylamino, Carbamoyl oder Sulfamoyl substituiert sein kann, oder
-für Aryl, Heteroaryl, Aralkyl, Alkoxycarbonyl, Alkylsulfonyl, Phenylsulfonyl, Tolylsulfonyl, Benzylsulfonyl, Formyl, Carbamoyl oder Sulfamoyl steht,
$R^2$ -für Wasserstoff oder Alkyl steht
und
$R^3$ -für Alkyl steht,
wobei jedoch
$R^1$ nicht $NH_2$ bedeutet, wenn
$R^2$ und $R^3$ Propyl bedeuten
und deren Salze gefunden.

Die erfindungsgemäßen Stoffe zeigen überraschenderweise eine überlegene Wirkung auf das Zentralnervensystem und können zu therapeutischen Behandlung von Menschen und Tieren verwendet werden.

Die erfindungsgemäßen Stoffe haben mehrere asymmetrische Kohlenstoffatome und können daher in verschiedenen stereochemischen Formen existieren.

Sowohl die einzelnen Isomeren, als auch deren Mischungen sind Gegenstand der Erfindung. Beispielsweise seien folgende isomeren Formen der substituierten basischen 2-Aminotetraline genannt:

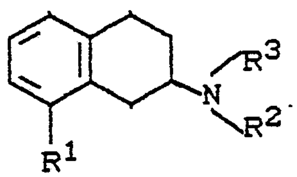

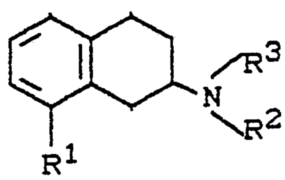

Die erfindungsgemäßen substituierten basischen 2-Aminotetraline können auch in Form ihrer Salze vorliegen. Im allgemeinen seien hier Salze mit anorganischen oder organischen Säuren genannt.

Im Rahmen der vorliegenden Erfindung werden physiologisch unbedenkliche Salze bevorzugt. Physiologisch unbedenkliche Salze der substituierten basischen 2-Aminotetraline können Salze der erfindungsgemäßen Stoffe mit Mineralsäure, Carbonsäuren oder Sulfonsäuren sein. Besonders bevorzugt sind beispielsweise Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Oxalsäure, Malonsäure, Bernsteinsäure, Maleinsäure, Fumarsäure, Äpfelsäure, Weinsäure, Zitronensäure oder Benzoesäure.

Im Rahmen der vorliegenden Erfindung haben die Substituenten im allgemeinen die folgende Bedeutung:

Alkyl steht im allgemeinen für einen verzweigten Kohlenwasserstoffrest mit 1 bis 12 Kohlenstoffatomen. Bevorzugt wird Niederalkyl mit 1 bis etwa 6 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

Alkenyl steht im allgemeinen für einen geradkettigen oder verzweigten Kohlenwasserstoffrest mit 2 bis 12 Kohlenstoffatomen und einer oder mehreren, bevorzugt mit einer oder zwei Doppelbindungen. Bevorzugt ist der Niederalkylrest mit 2 bis etwa 6 Kohlenstoffatomen und einer Doppelbindung. Besonders bevorzugt ist ein Alkenylrest mit 2 bis 4 Kohlenstoffatomen und einer Doppelbindung. Beispielsweise seien Allyl, Propenyl, Isopropenyl, Butenyl, Isobutenyl, Pentenyl, Isopentenyl, Hexenyl, Isohexenyl, Heptenyl, Isoheptenyl, Octenyl und Isooctenyl genannt.

Cycloalkyl steht im allgeinem für einen cyclischen Kohlenwasserstoffrest mit 5 bis 8 Kohlenstoffatomen. Bevorzugt ist der Cyclopentan-und der Cyclohexanring. Beispielsweise seien Cyclopentyl, Cyclohexyl, Cycloheptyl und Cyclooctyl genannt.

Aryl steht im allgemeinen für einen aromatischen Rest mit 6 bis etwa 12 Kohlenstoffatomen. Bevorzugte

Arylreste sind Phenyl, Naphthyl und Biphenyl.

Aralkyl steht im allgemeinen für einen über eine Alkylenkette gebundenen Arylrest mit 7 bis 14 Kohlenstoffatomen. Bevorzugt werden Aralkylreste mit 1 bis 6 Kohlenstoffatomen im aliphatischen Teil und 6 bis 12 Kohlenstoffatome im aromatischen Teil. Beispielsweise seien folgende Aralkylreste genannt: Benzyl, Naphthylmethyl, Phenethyl und Phenylpropyl.

Alkoxy steht im allgemeinen für einen über ein Sauerstoffatom gebundenen geradkettigen oder verzweigten Kohlenwasserstoffrest mit 1 bis 12 Kohlenstoffatomen. Bevorzugt ist Niederalkoxy mit 1 bis etwa 6 Kohlenstoffatomen. Besonders bevorzugt ist ein Alkoxyrest mit 1 bis 4 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

Aryloxy steht im allgemeinen für einen aromatischen Rest mit 6 bis etwa 12 Kohlenstoffatomen, der über ein Sauerstoffatom gebunden ist. Bevorzugte Aryloxyreste sind Phenoxy oder Naphthyloxy.

Aralkoxy steht im allgemeinen für einen Aralkylrest mit 7 bis 14 Kohlenstoffatomen, wobei die Alkylenkette über ein Sauerstoffatom gebunden ist. Bevorzugt werden Aralkoxyreste mit 1 bis 6 Kohlenstoffatomen im aliphatischen Teil und 6 bis 12 Kohlenstoffatomen im aromatischen Teil. Beispielsweise seien folgende Aralkoxyreste genannt: Benzyloxy, Naphthylmethoxy, Phenethoxy und Phenylpropoxy.

Alkylthio steht im allgemeinen für einen über ein Schwefelatom gebundenen geradkettigen oder verzweigten Kohlenwasserstoffrest mit 1 bis 12 Kohlenstoffatomen. Bevorzugt ist Niederalkylthio mit 1 bis etwa 6 Kohlenstoffatomen.

Besonders bevorzugt ist ein Alkylthiorest mit 1 bis 4 Kohlenstoffatomen. Beispielsweise seien Methylthio, Ethylthio, Propylthio, Isopropylthio, Butylthio, Isobutylthio, Pentylthio, Isopentylthio, Hexylthio, Isohexylthio, Heptylthio, Isoheptylthio, Octylthio oder Isooctylthio genannt.

Acyl steht im allgemeinen für Phenyl oder geradkettiges oder verzweigtes Niedrigalkyl mit 1 bis etwa 6 Kohlenstoffatomen, die über eine Carbonylgruppe gebunden sind. Bevorzugt sind Phenyl und Alkylreste mit bis zu 4 Kohlenstoffatomen. Beispielsweise seien genannt: Benzoyl, Acetyl, Ethylcarbonyl, Propylcarbonyl, Isopropylcarbonyl, Butylcarbonyl und Isobutylcarbonyl.

Alkoxycarbonyl kann beispielsweise durch die Formel

$$-\underset{\underset{O}{\|}}{C}-OAlkyl$$

dargestellt werden.

Alkyl steht hierbei für einen geradkettigen oder verzweigten Kohlenwasserstoffrest mit 1 bis 12 Kohlenstoffatomen. Bevorzugt wird Niederalkoxycarbonyl mit 1 bis etwa 6 Kohlenstoffatomen im Alkylteil. Insbesondere bevorzugt wird ein Alkoxycarbonyl mit 1 bis 4 Kohlenstoffatomen im Alkylteil. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Aryloxycarbonyl kann beispielsweise durch die Formel -COO-Aryl dargestellt werden. Aryl steht hierbei im allgemeinen für einen aromatischen Rest mit 6 bis 12 Kohlenstoffatomen. Beispielsweise seien zu nennen: Phenoxycarbonyl und Naphthyloxycarbonyl.

Aralkoxycarbonyl kann beispielsweise durch die Formel -COO-Aralkyl dargestellt werden. Aralkyl steht hierbei im allgemeinen für einen über eine Alkylenkette gebundenen Arylrest mit 7 bis 14 Kohlenstoffatomen, bevorzugt werden Aralkylreste mit 1 bis 6 Kohlenstoffatomen im aliphatischen Teil und 6 bis 12 Kohlenstoffatomen im aromatischen Teil. Beispielsweise seien als Aralkoxycarbonylreste genannt: Benzyloxycarbonyl, Naphthylmethyloxycarbonyl.

Heteroaryl im Rahmen der oben angebenen Definition steht im allgemeinen für einen 5-bis 6-gliedrigen aromatischen Ring, der als Heteroatome Sauerstoff, Schwefel und/oder Stickstoff enthalten kann und an den ein weiterer aromatischer Ring ankondensiert sein kann. Bevorzugt sind 5-und 6-gliedrige aromatische Ringe, die einen Sauerstoff, ein Schwefel und/oder bis zu 2 Stickstoffatome enthalten und die gegebenenfalls benzokondensiert sind. Als besonders bevorzugte Heteroarylreste seien genannt:

thienyl, Furyl, Pyridyl, Pyrimidyl, Pyrazinyl, Pyridazinyl, Chinolyl, Isochinolyl, Chinazolyl, Chinoxyalyl, Thiazolyl, Benzothiazolyl, Isothiazolyl, Oxazolyl, Benzoxazoylyl, Isoxazolyl, Imidazolyl, Benzimidazolyl, Pyrazolyl und Indolyl.

Halogen steht im allgemeinen für Fluor, Chlor, Brom oder Iod, bevorzugt für Fluor, Chlor oder Brom. Besonders bevorzugt steht Halogen für Fluor oder Chlor.

Bevorzugt werden solche Verbindungen der allgemeinen Formel (I),

in welcher

$R^1$ -für Fluor, Chlor, Brom oder Cyano steht, oder

-für eine Gruppe der Formel $-NR^4R^5$, $-COR^6$, $-(CH_2)_a-X$, $-O-(CH_2)_a-X$ oder $-CH=CH-(CH_2)_b-X$ steht,

worin

$R^4$ und $R^5$ gleich oder verschieden sind und
-Wasserstoff oder
-eine Gruppe der Formel $-COR^7$ oder $-SO_2R^8$ bedeuten,
wobei
$R^7$ -für Wasserstoff steht, oder
-für eine Gruppe $-NHR^9$ steht, oder
- für Niederalkoxy steht, oder
-für gegebenenfalls durch Niederalkyl, Niederalkoxy, Fluor, Chlor, Brom, Trifluormethyl, Dimethylamino oder Diethylamino substituiertes Phenyl, Benzyl, Benzyloxy, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl steht,
$R^8$ -für Cyclopropyl, Cyclopentyl oder Cyclohexyl steht, oder
-für gegebenenfalls durch Cyano, Fluor, Chlor, Brom, Trifluormethyl oder Niederalkoxycarbonyl substituiertes Niederalkyl steht, oder
-für gegebenenfalls durch Niederalkyl, Niederalkoxy, Fluor, Chlor, Brom, Trifluormethyl, Dimethylamino oder Diethylamino substituiertes Phenyl, Benzyl, Thienyl, Furyl, Pyrimidyl, Pyridyl, Chinolyl, Isochinolyl, Benzothiazolyl, Benzoxazolyl, Thiazolyl, Oxazolyl, Isoxazolyl oder Isothiazolyl steht, oder
-für eine Gruppe der Formel $-NR^{10}R^{11}$ steht,
worin
$R^{10}$ und $R^{11}$ gleich oder verschieden sind und Wasserstoff, Niederalkyl oder Phenyl bedeuten
und
$R^9$ -für Wasserstoff steht, oder
-für gegebenenfalls durch Cyano, Fluor, Chlor oder Brom substituiertes Niederalkyl steht, oder
-für gegebenenfalls durch Niederalkyl, Niederalkoxy, Fluor, Chlor, Brom, Trifluormethyl, Dimethylamino oder Diethylamino substituiertes Phenyl, Benzyl, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl steht,
$R^6$ -Wasserstoff, Hydroxy, Amino, Niederalkoxy oder Benzyloxy bedeutet,
a - eine Zahl 1 bis 8 bedeutet,
b - eine Zahl 0 bis 6 bedeutet
und
X - eine Gruppe der Formel $-NR^{12}R^{13}$, $-COR^{14}$, $-SO_2R^{15}$ oder $-OR^{16}$ bedeutet,
wobei
$R^{12}$ und $R^{13}$ gleich oder verschieden sind und
-für Wasserstoff, Niederalkyl, Phenyl oder Benzyl steht, wobei die genannten Reste durch Fluor, Chlor, Brom, Niederalkyl, Niederalkoxy oder Trifluormethyl substituiert sein können, oder
-für eine Gruppe der Formel $-COR^{14}$, $-SO_2R^{15}$ oder $-(CH_2)_c-NR^{12}R^{13}$ stehen,
$R^{14}$ -eine Gruppe $-NHR^{17}$ bedeutet, oder
-Niederalkyl oder Niederalkoxy bedeutet, oder
-gegebenenfalls durch Niederalkyl, Niederalkoxy, Fluor, Chlor, Brom, Trifluormethyl, Dimethylamino oder Diethylamino substituiertes Phenyl, Benzyl, Benzyloxy, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,
$R^{15}$ -Cyclopropyl, Cyclopentyl, Cyclohexyl, oder
-gegebenenfalls durch Cyano, Fluor, Chlor, Brom, Trifluormethyl oder Niederalkoxycarbonyl substituiertes Niederalkyl bedeutet, oder
-gegebenenfalls ein-oder mehrfach gleich oder verschieden durch Niederalkyl, Niederalkoxy, Fluor, Chlor, Brom, Trifluormethyl, Dimethylamino oder Diethylamino substituiertes Phenyl, Naphthyl, Benzyl, Thienyl, Furyl, Pyrimidyl, Pyridyl, Chinolyl, Isochinolyl, Benzothiazolyl, Benzoxazolyl, Thiazolyl, Oxazolyl, Isoxazolyl oder Isothiazolyl bedeutet, oder
-eine Gruppe $-NR^{10}R^{11}$ bedeutet,
wobei
$R^{10}$ und $R^{11}$ die oben angegebene Bedeutung haben,
$R^{16}$ -Wasserstoff, Niederalkyl, Phenyl oder Benzyl bedeutet,
$R^{17}$ -Wasserstoff bedeutet, oder
-gegebenenfalls durch Cyano, Fluor, Chlor oder Brom substituiertes Niederalkyl bedeutet, oder
-gegebenenfalls durch Niederalkyl, Niederalkoxy, Fluor, Chlor, Brom, Trifluormethyl, Dimethylamino oder Diethylamino substituiertes Phenyl, Benzyl, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,
und
c - eine Zahl 1 bis 6 bedeutet,

oder wobei

$R^{12}$ und $R^{13}$ zusammen mit dem Stickstoffatom einen Ring der Reihe

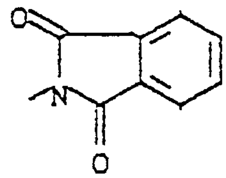 , 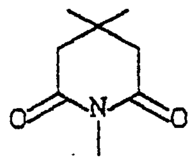 , 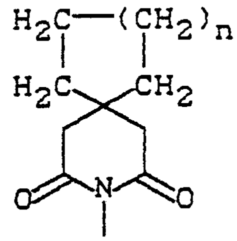 ,

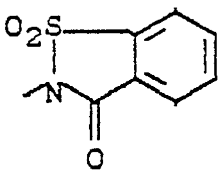 , 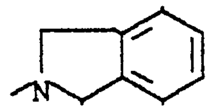 , 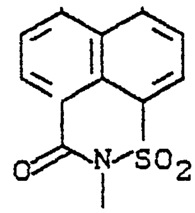 ,

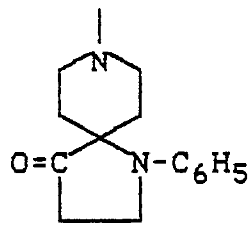 oder 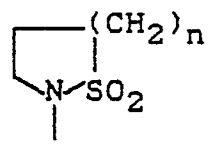 bilden,

worin

n - eine Zahl 1 oder 2 bedeutet,

$R^2$ -für Wasserstoff oder Niederalkyl steht

und

$R^3$ -für Niederalkyl steht,

wobei jedoch

$R^1$ -nicht für $NH_2$ steht, wenn

$R^2$ und $R^3$ Propyl bedeuten

und deren Salze.

Besonders bevorzugt sind solche Verbindungen der allgemeinen Formel (I) in welcher

$R^1$ -für Chlor, Brom, Cyano oder - für eine Gruppe der Formel $-NR^4R^5$, $-COR^6$, $-(CH_2)_a-X$, $-O-(CH_2)_a-X$ oder $-CH=CH-(CH_2)_b-X$ steht,

worin

$R^4$ und $R^5$ gleich oder verschieden sind und

-Wasserstoff oder

-eine Gruppe der Formel $-COR^7$ oder $-SO_2R^8$ bedeuten,

worin

$R^7$ -für Wasserstoff steht, oder

-für eine Gruppe $-NHR^9$ steht, oder

-für Methoxy, Ethoxy, Propoxy oder Isopropoxy steht, oder

-für gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl steht,

$R^8$ -für gegebenfalls durch Fluor, Chlor, Methoxycarbonyl, Ethoxycarbonyl, Propoxycarbonyl, Isopropoxycarbonyl, Butoxycarbonyl oder Isobutoxycarbonyl substituiertes Ethyl, Propyl, Isopropyl, Butyl oder Isobutyl steht, oder

- für gegebenenfalls durch Methyl, Ethyl, Propyl, Isopropyl, Methoxy, Ethoxy, Propoxy oder Isopropoxy, Fluor oder Chlor substituiertes Phenyl, Thienyl, Furyl, Pyridyl, Pyrimidyl, Chinolyl oder Isochinolyl steht, oder

-für eine Gruppe $-NR^{10}R^{11}$ steht,

worin

$R^{10}$ und $R^{11}$ gleich oder verschieden sind und Wasserstoff, Methyl, Ethyl, Propyl, Isopropyl, Butyl oder Isobutyl bedeuten

und

$R^9$ -für Wasserstoff steht, oder
-für gegebenenfalls durch Fluor oder Chlor substituiertes Methyl, Ethyl, Propyl, Isopropyl, Butyl, Isobutyl, Pentyl, Isopentyl, Hexyl oder Isohexyl steht, oder
-für Phenyl steht, das durch Fluor, Chlor, Methyl oder Methoxy substituiert sein kann,
$R^6$ -Wasserstoff, Hydroxy, Amino, Methoxy, Ethoxy, Propoxy, Butoxy, Isopropoxy oder Isobutoxy bedeutet,
a - eine Zahl 1 bis 6 bedeutet,
b - eine Zahl 0 bis 4 bedeutet
und
X - eine Gruppe der Formel $-NR^{12}R^{13}$, $-COR^{14}$, $-SO_2R^{15}$ oder $-OR^{16}$ bedeutet,
wobei
$R^{12}$ und $R^{13}$ gleich oder verschieden sind, und
-für Wasserstoff, Methyl, Ethyl, Propyl, Isopropyl, Butyl, Isobutyl, oder
-für gegebenenfalls durch Fluor, Chlor, Methyl oder Methoxy subsituiertes Phenyl, oder
-für eine Gruppe $-COR^{14}$, $-SO_2R^{15}$ oder

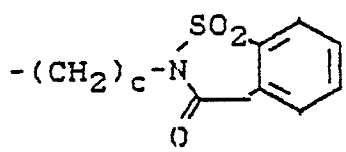

stehen,
$R^{14}$ -Wasserstoff bedeutet, oder
-eine Gruppe $-NHR^{17}$ bedeutet, oder
-Methyl, Ethyl, Propyl, Isopropyl, Methoxy, Ethoxy, Propoxy, Isopropoxy, oder
-gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl bedeutet,
$R^{15}$ -gegebenenfalls durch Fluor, Chlor, Methoxycarbonyl, Ethoxycarbonyl, Propoxycarbonyl, Isopropoxycarbonyl, Butoxycarbonyl oder Isobutoxycarbonyl substituiertes Methyl, Ethyl, Propyl, Isopropyl, Butyl oder Isobutyl bedeutet, oder
-gegebenenfalls ein-oder mehrfach gleich oder verschieden durch Methyl, Ethyl, Propyl, Isopropyl, Methoxy, Fluor oder Chlor substituiertes Phenyl, Naphthyl, Thienyl, Furyl, Pyridyl, Pyrimidyl, Chinolyl oder Isochinolyl bedeutet, oder
-eine Gruppe $-NR^{10}R^{11}$ bedeutet,
$R^{10}$ und $R^{11}$ die oben angegebene Bedeutung haben,
$R^{16}$ -Wasserstoff, Methyl, Ethyl, Propyl, Isopropyl, Phenyl oder Benzyl bedeutet,
$R^{17}$ -Wasserstoff bedeutet, oder
-gegebenenfalls durch Fluor oder Chlor substituiertes Methyl, Ethyl, Propyl, Isopropyl, Butyl, Isobutyl, Pentyl, Isopentyl, Hexyl oder Isohexyl bedeutet, oder
-Phenyl bedeutet, das durch Fluor, Chlor, Methyl oder Methoxy substituiert sein kann,
und
c - eine Zahl 1 bis 4 bedeutet,
$R^2$ -für Wasserstoff, Methyl, Ethyl, Propyl oder Isopropyl steht
und
$R^3$ -für Methyl, Ethyl, Propyl oder Isopropyl steht,
wobei jedoch
$R^1$ -nicht $NH_2$ bedeutet, wenn
$R^2$ und $R^3$ Propyl bedeuten,
und deren Salze.

Ganz besonders bevorzugt sind solche Verbindungen der allgemeinen Formel (I),
in welcher
$R^1$ -für Chlor, Brom, Cyano oder
-für eine Gruppe der Formel $-NR^4R^5$, $-COR^6$, $-(CH_2)_a-X$, $-O-(CH_2)_a-X$ oder $-CH=CH-(CH_2)_b-X$ steht,
worin
$R^4$ -Wasserstoff bedeutet,
$R^5$ -eine Gruppe der Formel $-COR^7$ oder $-SO_2R^8$ bedeuten,
worin
$R^7$ -für Wasserstoff steht, oder

-für eine Gruppe $-NHR^9$ steht, oder
-für Methoxy oder Ethoxy steht.
$R^8$ -für Trifluormethyl, Phenyl, Tolyl steht, oder
-für eine Gruppe $-NR^{10}R^{11}$ steht.
worin
$R^{10}$ und $R^{11}$ gleich oder verschieden sind und Wasserstoff, Methyl oder Ethyl bedeuten,
und
$R^9$ -für Wasserstoff steht, oder
-für Methyl, Ethyl, Propyl, Isopropyl oder Butyl, oder
-für Phenyl steht,
$R^6$ -Wasserstoff, Hydroxy, Amino, Methoxy oder Ethoxy bedeutet.
a - eine Zahl 1 bis 4 bedeutet,
b - eine Zahl 0 bis 2 bedeutet
und
X - eine Gruppe der Formel $-NR^{12}R^{13}$, $-COR^{14}$, $-SO_2R^{15}$ oder $-OR^{16}$ bedeutet,
wobei
$R^{12}$ und $R^{13}$ gleich oder verschieden sind, und
-für Wasserstoff, Methyl, Ethyl, Propyl, oder
-für eine Gruppe $-COR^{14}$, $-SO_2R^{15}$ oder

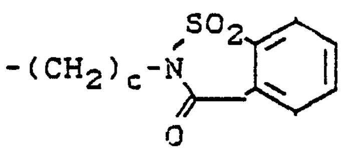

stehen,
$R^{14}$ - Wasserstoff bedeutet, oder
-eine Gruppe $-NHR^{17}$ bedeutet, oder
-Methyl, Ethyl, Propyl, Methoxy oder Ethoxy bedeutet,
$R^{15}$ -Trifluormethyl, Methyl, Ethyl, Propyl, Isopropyl, Butyl oder Isobutyl bedeutet, oder
-gegebenenfalls durch ein-oder mehrere Methyl oder Chlor substituiertes Phenyl, oder Naphthyl bedeutet,
-eine Gruppe $-NR^{10}R^{11}$ bedeutet,
wobei
$R^{10}$ und $R^{11}$ die oben angegebene Bedeutung haben,
$R^{16}$ -Wasserstoff, Methyl, Ethyl oder Propyl, bedeutet,
$R^{17}$ -Wasserstoff bedeutet, oder
-Methyl, Ethyl, Propyl, Isopropyl, Butyl oder Isobutyl bedeutet, oder
-Phenyl bedeutet
und
c - eine Zahl 2 bis 4 bedeutet,
und
$R^2$ und $R^3$ für Propyl stehen
und deren Salze.

Beispielhaft seien folgende 8-substituierte 2-Aminotetraline genannt:
2-Dipropylamino-8-chloro-1,2,3,4-tetrahydronaphthalin,
2-Dipropylamino-8-bromo-1,2,3,4-tetrahydronaphthalin,
2-Dipropylamino-8-cyano-1,2,3,4-tetrahydronaphthalin,
2-Dipropylamino-8-(3-butyl-ureido)-1,2,3,4-tetrahydronaphthalin,
2-Dipropylamino-8-formamido-1,2,3,4-tetrahydronaphthalin,
2-Dipropylamino-8-carbamoyl-1,2,3,4-tetrahydronaphthalin,
2-Dipropylamino-1,2,3,4-tetrahydronaphthalin-8-carbonsäure,
2-Dipropylamino-8-ethoxycarbonyl-1,2,3,4-tetrahydronaphthalin,
2-Dipropylamino-8-formyl-1,2,3,4-tetrahydronaphthalin,
2-Dipropylamino-8-hydroxymethyl-1,2,3,4-tetrahydronaphthalin,
2-Dipropylamino-8-aminomethyl-1,2,3,4-tetrahydronaphthalin,
2-Dipropylamino-8-aminomethyl-1,2,3,4-tetrahydronaphthalin Hydrochlorid,
2-Dipropylamino-8-sulfonamidomethyl-1,2,3,4-tetrahydronaphthalin,

2-Dipropylamino-8-butylsulfonamidomethyl-1.2.3.4-tetrahydronaphthalin.
2-Dipropylamino-8-ethoxycarbonylamidomethyl-1.2.3.4-tetrahydronaphthalin.
2-Dipropylamino-8-ethoxycarbonylamidomethyl-1.2.3,4-tetrahydronaphthalin Hydrochlorid.
2-Dipropylamino-8-(3,3-diethylureido)methyl-1,2,3,4-tetrahydronaphthalin.
2-Dipropylamino-8-ureidomethyl-1.2,3.4-tetrahydronaphthalin.
2-Dipropylamino-8-(3-methylureido)methyl-1.2,3,4-tetrahydronaphthalin.
2-Dipropylamino-8-formamidoethyl-1,2,3,4-tetrahydronaphthalin.
2-Dipropylamino-8-(2-hydroxyethoxy)-1,2,3,4-tetrahydronaphthalin Hydrochlorid.
2-Dipropylamino-8-carbamoylethoxy-1,2,3,4-tetrahydronaphthalin.
2-Dipropylamino-8-(2-aminoethoxy)-1,2,3,4-tetrahydronaphthalin.
2-Dipropylamino-8-(2-methansulfonamido-methoxy)-1,2,3,4-tetrahydronaphthalin,
2-Dipropylamino-8-(2-butylsulfonamido-ethoxy)-1.2.3,4-tetrahydronaphthalin
2-Dipropylamino-8-(2-propionylamido-ethoxy)-1,2,3,4-tetrahydronaphthalin,
2-Dipropylamino-8-(2-ethoxycarbonylamido-ethoxy)-1,2,3,4-tetrahydronaphthalin,
2-Dipropylamino-8-[2-(3.3-diethylureido)ethoxy]-1.2.3,4-tetrahydronaphthalin.
2-Dipropylamino-8-ureidoethoxy-1,2,3,4-tetrahydronaphthalin,
2-Dipropylamino-8-[2-(3-methylureido)ethoxy]-1,2,3,4-tetrahydronaphthalin.
2-Dipropylamino-8-[2-(3-butylureido)ethoxy]-1,2,3,4-tetrahydronaphthalin.
2-Dipropylamino-8-(2-formylamido-ethoxy)-1,2,3,4-tetrahydronaphthalin,
2-Dipropylamino-8-[2-(N,N-dimethylaminosulfonyl)ethenyl]-1,2.3,4-tetrahydronaphthalin,
2-Dipropylamino-8-[2-(N.N-dimethylaminosulfonyl)ethyl]-1,2,3.4-tetrahydronaphthalin,
2-Dipropylamino-8-(2-nitro-ethenyl)-1,2,3,4-tetrahydronaphthalin,
2-Dipropylamino-8-(2-amino-ethyl)-1,2,3,4-tetrahydronaphthalin,
2-Dipropylamino-8-(2-methansulfonamido-ethyl)-1,2,3,4-tetrahydronaphthalin Hydrochlorid,
2-Dipropylamino-8-(2-butansulfonamido-ethyl)-1.2,3,4-tetrahydronaphthalin.
2-Dipropylamino-8-[2-(p-chlorbenzolsulfonamido)ethyl]-1,2,3,4-tetrahydronaphthalin,
2-Dipropylamino-8-(2-ethoxycarbonylamido-ethyl)-1,2,3,4-tetrahydronaphthalin.
2-Dipropylamino-8-(2-benzyloxycarbonylamido-ethyl)-1,2,3,4-tetrahydronaphthalin.
2-Dipropylamino-8-(2-ureido-ethyl)-1,2,3,4-tetrahydronaphthalin,
2-Dipropylamino-8-[2-(3-methylureido)ethyl]-1,2,3,4-tetrahydronaphthalin.
2-Dipropylamino-8-(2-formylamido-ethyl)-1.2,3,4-tetrahydronaphthalin.

Im Rahmen der vorliegenden Erfindung entsprechen die 8-Halogen-aminotetraline (Ia) der allgemeinen Formel

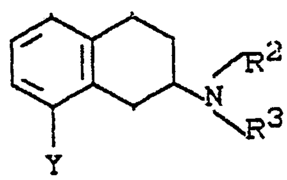

(Ia),

in welcher
$R^2$ und $R^3$ die angegebene Bedeutung haben
und
Y - für Halogen oder Cyano steht.

Im Rahmen der vorliegenden Erfindung entsprechen die Diaminotetraline (Ib) der allgemeinen Formel

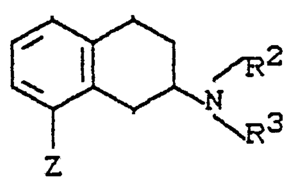

(Ib),

in welcher
Z - für eine Gruppe der Formel $-NR^4R^5$ steht,
worin
$R^4$ und $R^5$ -gleich oder verschieden sind und

-Wasserstoff oder
-eine Gruppe der Formel $-COR^7$ oder $-SO_2R^8$ bedeuten,
wobei
$R^7$ -für Wasserstoff, oder
-für Alkoxy steht, oder
-für Aryl, Aryloxy, Aralkoxy, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
und
$R^8$ -die angegebene Bedeutung hat und
$R^2$ und $R^3$ die angegebene Bedeutung haben.

Im Rahmen der vorliegenden Erfindung entsprechen die 8-Ureido-aminotetraline (Ic) der allgmeinen Formel

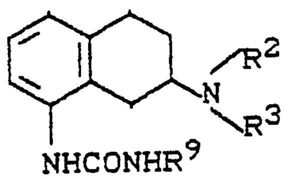

(Ic),

in welcher
$R^2$, $R^3$ und $R^9$ die angegebene Bedeutung haben.

Im Rahmen der vorliegenden Erfindung entsprechen die 8-Acyl-aminotetraline (Id) der allgemeinen Formel

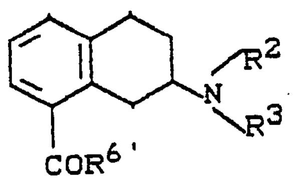

(Id)

in welcher
$R^{6'}$- für Hydroxy, Amino, Alkoxy, Aryloxy oder Aralkoxy steht
und
$R^2$ und $R^3$ die angegebene Bedeutung haben.

Im Rahmen der vorliegenden Erfindung entsprechen die 8-Formyl-aminotetraline (Ie) der allgemeinen Formel

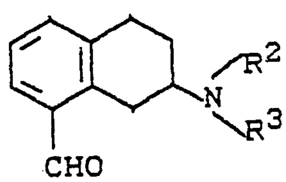

(Ie),

in welcher
$R^2$ und $R^3$ die angegebene Bedeutung haben,

Im Rahmen der vorliegenden Erfindung entsprechen die 8-Methylen-aminotetraline (If) der allgemeinen Formel

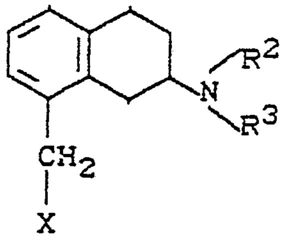

(If),

in welcher $R^2$, $R^3$ und X die oben angegebene Bedeutung haben.

Im Rahmen der vorliegenden Erfindung ensprechen die 8-Alkylen-aminotetraline (Ig) der allgemeinen Formel

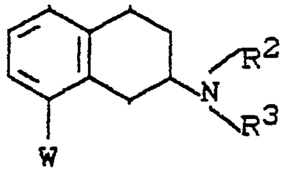

(Ig),

in welcher
W - für eine Gruppe der Formel $-(CH_2)_{a'}-X$ oder $-CH=CH-(CH_2)_b-X$ steht,
$R^2$, $R^3$, X und b die oben angegebene Bedeutung haben
und
a' eine Zahl 2 bis 10 bedeutet.

Im Rahmen der vorliegenden Erfindung entsprechen die 8-Ethylen-aminotetraline (Ih) der allgemeinen Formel

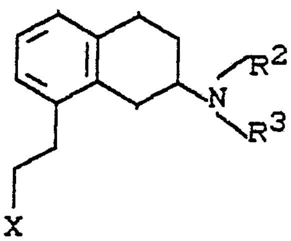

(Ih),

in welcher $R^2$, $R^3$ und X die angegebene Bedeutung haben.

Es wurde ein Verfahren zur Herstellung der erfindungsgemäßen 8-Halogen-aminotetraline der allgemeinen Formel (Ia)

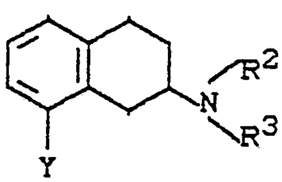

(Ia),

in welcher
Y - für Halogen oder Cyano steht,
$R^2$ -für Wasserstoff oder Alkyl steht,
und
$R^3$ -für Alkyl steht,
und deren Salze gefunden,
das dadurch gekennzeichnet ist, daß man
[A] 8-Aminotetraline der allgemeinen Formel (II)

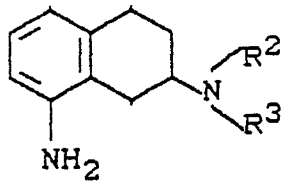

(II),

in welcher
$R^2$ und $R^3$ die angegebene Bedeutung haben,
in inerten Lösemitteln in Anwesenheit von Säuren mit Nitriten umsetzt,
dann die erhaltenen Diazoniumsalze mit Kupfersalzen der allgemeinen Formel (III)
CuY (III),
in welcher
Y die angegebene Bedeutung hat,
gegebenenfalls in Anwesenheit von Hilfsstoffen umsetzt
und im Fall der Herstellung der Salze mit den entsprechenden Säuren umsetzt,
oder, daß man
[B] Tetralone der allgmeinen Formel (IV)

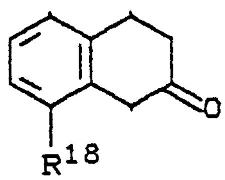

(IV),

in welcher
$R^{18}$ -für Chlor oder Brom steht,
zunächst mit Aminen der allgemeinen Formel (V)

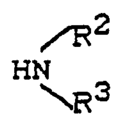

(V),

in welcher
$R^2$ und $R^3$ die angegebene Bedeutung haben,
in inerten Lösemitteln, gegebenenfalls in Anwesenheit von Hilfsstoffen umsetzt,
dann die Zwischenverbindungen in inerten Lösemitteln reduziert,
dann gegebenenfalls Halogen gegen Cyano austauscht
und dann im Fall der Herstellung der Salze mit den entsprechenden Säuren umsetzt.

Das erfindungsgemäße Verfahren kann durch folgendes Formelschema erläutert werden:

Verfahrensvariante A:

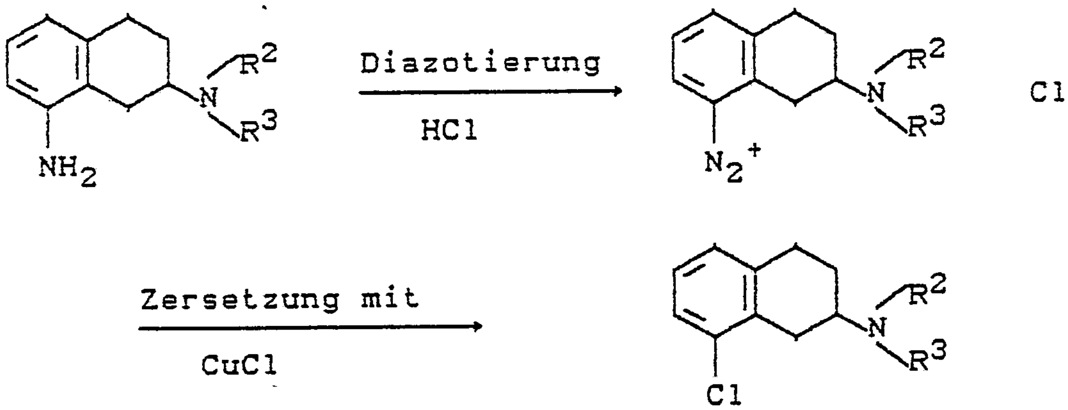


Verfahrensvariante B:

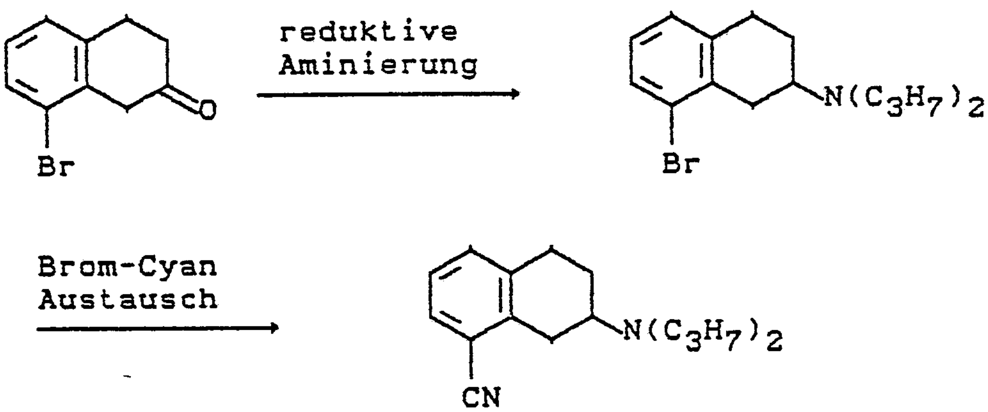


Verfahrensvariante A:

Bei der Durchführung des erfindungsgemäßen Verfahrens A entstehen im allgemeinen die Diazoniumsalze als Zwischenprodukte, die isoliert werden können. Es hat sich jedoch als zweckmäßig erwiesen, das Verfahren ohne Isolierung der Zwischenprodukte durchzuführen.

Als inerte Lösemittel eignen sich hierbei Wasser oder Alkohole wie Methanol, Ethanol, Propanol oder Isopropanol, oder Amide wie Formamid oder Dimethylformamid, oder Säuren wie Mineralsäuren oder Carbonsäuren. Bevorzugte sind Wasser und/oder Säuren wie Salzsäure, Bromwasserstoffsäure, Schwefelsäure, Phosphorsäure oder Essigsäure. Ebenso ist es möglich, Gemische der genannten Lösemitel einzusetzen.

Als Säuren werden im allgemeinen Mineralsäuren eingesetzt, Bevorzugt werden hierbei Chlorwasserstoffsäure, Bromwasserstoffsäure, Schwefelsäure, Phosphorsäure oder aber Gemische der genannten Säuren eingesetzt.

Als Nitrite werden im allgemeinen Alkalinitrite, wie Natrium-oder Kaliumnitrit verwendet. Bevorzugt wird Natriumnitrit eingesetzt.

Als Hilfsstoffe werden im allgemeinen Alkali-oder Erdalkalihalogenide bzw. -cyanide eingesetzt. Bevorzugt sind Natriumchlorid, Natriumbromid oder Natriumcyanid.

Die Umsetzung wird im allgemeinen in einem Temperaturbereich von -10°C bis ±150°C, bevorzugt von 0°C bis +100°C durchgeführt.

Die Umsetzung wird im allgemeinen bei Normaldruck durchgeführt. Es ist ebenso möglich, die Umsetzung bei erhöhtem oder erniedrigtem Druck durchzuführen (z.B. von 0,5 bis 5 bar).

Das erfindungsgemäße Verfahren wird im allgemeinen derart durchgeführt, daß man zunächst das 8-Aminotetralin in konzentrierten wässrigen Säuren mit einer Lösung von Nitrit in Wasser versetzt und anschließend die Reaktionslösung mit Kupfer(I)halogeniden bzw. Kupfer(I)cyanid, gegebenenfalls in Wasser

gelöst, behandelt. Das Diazoniumsalz wird im allgemeinen nicht isoliert. Bevorzugt verwendet man bei der Einführung des Chloratoms (Y = Cl) Salzsäure und Kupfer(I)chlorid, bei der Einführung des Bromatoms (Y = Br) Bromwasserstoffsäure und Kupfer(I)bromid und bei der Einführung der Nitrilfunktion (Y = CN) Schwefelsäure und Kupfer(I)cyanid, gegebenenfalls in Anwesenheit von Natriumcyanid.

Die Aufarbeitung erfolgt im allgemeinen durch Neutralisation der Reaktionsmischung mit Alkalihydroxiden oder -carbonaten, sowie Extraktion, Kristallisation und/oder Chromatographie der so erhaltenen freien Basen, aus denen durch Umsetzung mit entsprechenden Säuren deren Salze erhalten werden.

Die als Ausgangsverbindungen eingesetzten 8-Aminotetraline sind bekannt [EP-A1 41 488].

Beispielhaft seien folgende 8-Aminotetraline genannt:

8-Amino-2-dimethylamino-1,2,3,4-tetrahydronaphthalin,
8-Amino-2-diethylamino-1,2,3,4,-tetrahydronaphthalin,
8-Amino-2-dipropylamino-1,2,3,4-tetrahydronaphthalin,
8-Amino-2-(N-ethyl-N-methyl)amino-1,2,3,4-tetrahydronaphthalin,
8-Amino-2-(N-methyl-N-propyl)amino-1,2,3,4-tetrahydronaphthalin,
8-Amino-2-(N-ethyl-N-propyl)amino-1,2,3,4-tetrahydronaphthalin.

Verfahrensvariante B:

Die Herstellung der Zwischenverbindungen durch Umsetzung der Tetralone (IV) mit Aminen (V) erfolgt in inerten organischen Lösemitteln, gegebenenfalls in Anwesenheit eines Katalysators und gegebenenfalls in Anwesenheit eines wasserbindenden Mittels.

Im Fall der Reaktion mit primären Aminen sind die Zwischenverbindungen Schiff'sche Basen und im Fall der Reaktion mit sekundären Aminen sind die Zwischenverbindungen Enamine oder Immonium-Salze.

Das erfindungsgemäße Verfahren kann in zwei Schritten, d.h. mit Isolierung der Zwischenverbindungen durchgeführt werden. Ebenso ist es möglich das erfindungsgemäße Verfahren als Eintopfverfahren durchzuführen.

Als inerte Lösemittel eignen sich hierbei die üblichen organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Butylmethylether, Dioxan, Tetrahydrofuran, Glykoldimethylether oder Diethylenglykoldimethylether, oder Halogenkohlenwasserstoffe wie beispielsweise Methylenchlorid, Chloroform oder Tetrachlorkohlen stoff, oder Kohlenwasserstoffe wie Benzol, Toluol, Xylol, oder Erdölfraktionen, oder Amide wie Dimethylformamid oder Hexamethylphosphorsäuretriamid, oder Essigsäure.

Außerdem ist es möglich, Gemische der genannten Lösemittel zu verwenden.

Als Katalysatoren werden im allgemeinen Protonensäuren verwendet. Hierzu gehören bevorzugt anorganische Säuren wie beispielsweise Salzsäure oder Schwefelsäure, oder organische Carbonsäuren mit 1 bis 6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit $C_1$-$C_4$-Alkylresten oder mit Arylresten wie beispielsweise Methansulfonsäure, Ethansulfonsäure, Benzolsulfonsäure oder Toluolsulfonsäure.

Das bei der Reaktion gebildete Wasser kann gegebenenfalls im Gemisch mit dem verwendeten Lösemittel während oder nach der Reaktion z.B. durch Destillation oder durch Zugabe von wasserbindenden Mitteln, wie beispielsweise Phosphorpentoxid, oder bevorzugt durch Molekularsieb, entfernt werden.

Die Reaktion wird im allgemeinen in einem Temperaturbereich von 0°C bis +200°C, bevorzugt von +20°C bis +150°C durchgeführt.

Bei dem azeotropen Abdestillieren des bei der Reaktion gebildeten Wassers mit den verwendeten Lösemitteln, arbeitet man bevorzugt bei der Siedetemperatur des Azeotrops.

Die Umsetzung kann bei normalem, erhöhtem und bei erniedrigtem Druck durchgeführt werden (z.B. 0,5 - 5 bar). Im allgemeinen arbeitet man bei Normaldruck.

Bei der Durchführung der Reaktion werden im allgemeinen die Ausgangsstoffe in einem molaren Verhältnis von Tetralon (IV) zu Amin (V) von 0,5 : 2 bis 1 : 2 eingesetzt. Bevorzugt verwendet man molare Mengen der Reaktanden.

Die Reduktion der Enamine erfolgt entweder durch Wasserstoff in Wasser oder inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder aber mit Hydriden in inerten Lösemitteln, gegebenenfalls in Anwesenheit eines Katalysators.

Bevorzugt wird die Reaktion mit Hydriden, wie komplexen Borhydriden oder Aluminiumhydriden,

durchgeführt. Besonders bevorzugt werden hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Diethylenglykoldimethylether, oder Amide wie Hexamethylphosphorsäuretriamid, oder Dimethylformamid oder Essigsäure. Ebenso ist es möglich, Gemische der genannten Lösemittel zu verwenden.

Als Katalysatoren bei der Reduktion werden im allgemeinen Protonensäuren verwendet. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit $C_1$-$C_4$-Alkylresten oder mit Arylresten wie beispielsweise Methansulfonsäure, Ethansulfonsäure, Benzolsulfonsäure oder Toluolsulfonsäure.

Bei der Durchführung des erfindungsgemäßen Verfahrens hat es sich als günstig erwiesen, die Umsetzung der Tetralone (IV) mit den Aminen (V) in einem inerten Lösemittel, bevorzugt in Essigester oder in Alkoholen wie beispielsweise Methanol, Ethanol, Propanol oder Isopropanol, oder deren Gemische in Anwesenheit von anorganischen oder organischen Säuren, wie beispielsweise Chlorwasserstoffsäure oder Essigsäure, und in Anwesenheit eines Reduktionsmittels, bevorzugt von komplexen Hydriden wie beispielsweise Natriumborhydrid oder Natriumcyanoborhydrid, gegebenenfalls in Anwesenheit eines wasserentziehenden Mittels, bevorzugt von Molsieb, als Eintopfverfahren durchzuführen.

In diesem Fall wird die Reaktion in einem Temperaturbereich von 0° C bis +150° C, bevorzugt von 0° C bis +100° C bei Normaldruck durchgeführt. Es ist ebenso möglich die Reaktion bei Unterdruck oder bei Überdruck (z.B. im Bombenrohr) durchzuführen.

Wird das erfindungsgemäße Verfahren als Eintopfreaktion durchgeführt, hat es sich als günstig erwiesen, das Amin in einem bis zu 10-fachen, bevorzugt in einem bis zu 5-fachen Überschuß über das Tetralon einzusetzen.

Der Austausch von Halogen, insbesondere Brom gegen Cyano, erfolgt im allgemeinen mit Kupfer(I)-cyanid in inerten Lösemitteln, bevorzugt Amiden wie Dimethylformamid oder Hexamethylphosphorsäuretriamid in einem Temperaturbereich von +20° C bis +200° C, bevorzugt von +50° C bis +150° C bei Normaldruck.

Die als Ausgangsstoffe eingesetzten Amine sind bekannt oder können nach bekannten Verfahren hergestellt werden [Houben-Weyls "Methoden der organischen Chemie" XI/1 und XI/2].

Beispielhaft seien folgende Amine genannt: Methylamin, Ethylamin, Propylamin, Dimethylamin, Diethylamin, Dipropylamin, Methyl-propylamin, Ethyl-methylamin, Ethyl-propylamin.

Die als Ausgangstoffe eingesetzten Tetralone sind teilweise neu und können nach an sich bekannten Methoden der Friedel-Crafts-Acylierung aus 2-Halogen-phenylessigsäurechloriden oder -bromiden, Aluminiumchlorid und Ethern hergestellt werden [Houben-Weyl's "Methoden der organischen Chemie" VII/2a, 141: G.P. Johnson, Org. Synth., Coll. Vol. IV, 900 (1963)].

Beispielhaft seien folgende Tetralone genannt:
8-Chlor-tetralon-2, 8-Brom-tetralon-2.

Außerdem wurde ein Verfahren zur Herstellung der erfindungsgemäßen Diaminotetraline der allgemeinen Formel (Ib)

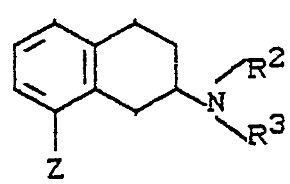

(Ib)

worin
Z - für eine Gruppe der Formel $-NR^4R^5$ steht,
worin
$R^4$ und $R^5$ gleich oder verschieden sind und
-für Wasserstoff, oder
-eine Gruppe der Formel $-COR^{7'}$ oder $-SO_2R^8$ bedeuten,
wobei
$R^{7'}$ -für Wasserstoff steht, oder

- für Alkoxy steht, oder

-für Aryl, Aryloxy, Aralkyl, Aralkoxy oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,

und

$R^8$ -für Cycloalkyl steht, oder

-für Alkyl steht, das durch Cyano, Halogen, Trifluormethyl, Trifluormethoxy oder Alkoxycarbonyl substituiert sein kann, oder

-für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder

-für eine Gruppe $-NR^{10}R^{11}$ steht,

worin

$R^{10}$ und $R^{11}$ gleich oder verschieden sind und Wasserstoff, Alkyl, Aryl oder Aralkyl bedeuten.

$R^2$ -für Wasserstoff oder Alkyl steht,

und

$R^3$ -für Alkyl steht,

wobei jedoch

$R^1$ nicht $NH_2$ bedeutet, wenn

$R^2$ und $R^3$ Propyl bedeuten,

und deren Salze gefunden,

das dadurch gekennzeichnet ist, daß man

8-Aminotetraline der allgemeinen Formel (II)

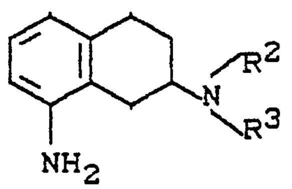

(II)

in welcher

$R^2$ und $R^3$ die oben angegebene Bedeutung haben,

mit Acylierungs-bzw. Sulfonierungsmitteln der allgemeinen Formel (VI)

$V-R^{19}$ (VI)

in welcher

$R^{19}$ für eine Gruppe der Formel $-COR^7$ oder $-SO_2R^8$ steht,

und

V - für Halogen, oder

-für den Rest $-OR^{20}$ steht,

worin

$R^{20}$ die gleiche Bedeutung hat wie $R^{19}$ und mit diesem gleich oder verschieden sein kann,

in inerten Lösemitteln gegebenenfalls in Anwesenheit von Basen umsetzt

und dann im Fall der Herstellung der Salze mit den entsprechenden Säuren umsetzt.

Das erfindungsgemäße Verfahren kann durch folgende Schemata erläutert werden:

a)

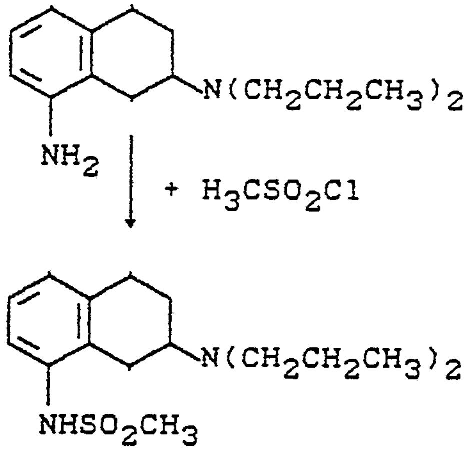


b)

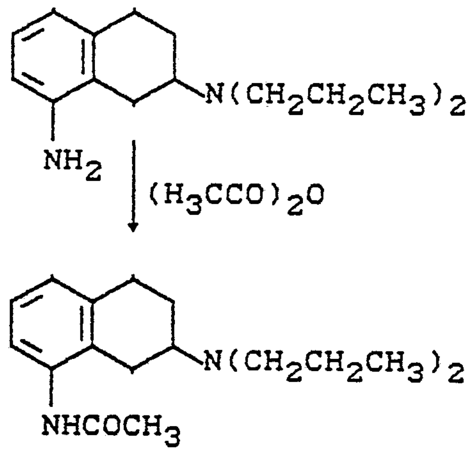


Als inerte Lösemittel eignen sich hierbei die üblichen organischen Lösemittel, die sich unter den Reaktionsbedingungen nicht verändern. Hierzu gehören bevorzugt Ether wie Diethylether, Butylmethylether, Dioxan, Tetrahydro furan, Glykoldimethylether oder Diethylenglykoldimethylether, oder Halogenkohlenwasserstoffe wie beispielsweise Methylenchlorid, Chloroform, Tetrachlorkohlenstoff, Dichlorethan, Dichlorethylen oder Trichlorethylen, oder Kohlenwasserstoffe wie Benzol, Toluol, Xylol, oder Erdölfraktionen, oder Alkohole wie Methanol, Ethanol, Propanol oder Isopropanol, oder Carbonsäuren wie Ameisensäure, Essigsäure oder Propionsäure, oder Carbonsäureanhydride wie Propionsäureanhydrid oder Acetanhydrid, oder Aceton, Essigester oder Acetonitril. Ebenso ist es möglich Gemische der genannten Lösemittel einzusetzen.

Als Basen können die üblichen basischen Verbindungen für basische Reaktionen eingesetzt werden. Hierzu gehören bevorzugt Alkali-oder Erdalkalihydroxide, oder Alkali-oder Erdalkalicarbonate wie beispielsweise Lithiumhydroxid, Natriumhydroxid, Kaliumhydroxid oder Bariumhydroxid, Natriumcarbonat, Kaliumcarbonat oder Natriumhydrogencarbonat, oder Alkalialkoholate wie beispielsweise Natriummethanolat, Natriumethanolat, Kaliummethanolat oder Kaliumethanolat, oder Alkaliamide wie Natriumamid oder Lithiumdiisopropylamid, oder organische Amine wie Triethylamin, Tripropylamin, Pyridin, Piperidin oder N,N-Dimethylaminopyridin.

Die Umsetzung erfolgt im allgemeinen in einem Temepaturbereich von -30° C bis +100° C, bevorzugt von 0C bis +80° C.

Die Umsetzung wird im allgemeinen bei Normaldruck durchgeführt. Es ist ebenso möglich die Umsetzung bei erhöhtem oder erniedrigtem Druck durchzuführen (z.B. von 0,5 bis 5 bar).

Als Acylierungsmittel (allgemeine Formel VI mit $R^{19}$ = $COR^7$) werden im allgemeinen Carbonsäurehalogenide oder -anhydride verwendet, bevorzugt Carbonsäurechloride oder -bromide, oder symmetrische oder unsymmetrische Carbonsäureanhydride, wobei bei den unsymmetrischen Anhydriden bevorzugt gemischte Anhydride mit Ameisensäure, Essigsäure oder Propionsäure verwendet werden.

Als Sulfonierungsmittel (allgemeinen Formel VI mit $R^{19}$ = $-SO_2R^8$) werden im allgemeinen Sulfonsäurehalogenide oder Sulfonsäureanhydride eingesetzt, bevorzugt Sulfonsäurechloride oder -bromide, oder symmetrische oder unsymmetrische Sulfonsäureanhydride, wobei bei den unsymmetrischen Anhydriden bevorzugt gemischte Anhydride mit Methan-Ethan-, Benzol-oder Toluolsulfonsäure verwendet werden.

Bei der Durchführung des erfindungsgemäßen Verfahrens werden die Acylierungs-bzw. Sulfonierungsmittel im allgemeinen in einer Menge von 1 bis 10 Mol, bevorzugt von 1 bis 5 Mol bezogen auf 1 Mol des

8-Aminotetralins eingesetzt. Die Base wird im allgemeinen in einer Menge von 1 bis 5, bevorzugt von 1 bis 2 Mol bezogen auf 1 Mol des Acylierungs-bzw. Sulfonierungsmittels eingesetzt.

Außerdem wurde ein Verfahren zur Herstellung der erfindungsgemäßen 8-Ureido-aminotetraline der allgemeinen Formel (Ic)

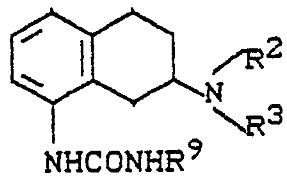
(Ic)

in welcher
$R^2$ -für Wasserstoff oder Alkyl steht,
$R^3$ -für Alkyl steht
und
$R^9$ für Wasserstoff steht, oder
-für Cycloalkyl steht, oder
-für gegebenenfalls durch Cyano, Halogen, Trifluormethyl oder Trifluormethoxy substituiertes Alkyl steht, oder
-für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
und deren Salze gefunden,
das dadurch gekennzeichnet ist, daß man
8-Aminotetraline der allgemeinen Formel (II)

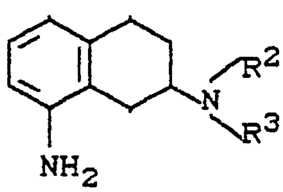
(II)

in welcher
$R^2$ und $R^3$ die angegebene Bedeutung haben,
in inerten Lösemitteln gegebenenfalls in Anwesenheit von Basen mit Isocyanaten der allgemeinen Formel (VII)
$R^9N=C=O$ (VIII)
in welcher
$R^9$ -die angegebene Bedeutung hat,
umsetzt,
und dann im Fall der Herstellung der Salze mit den entsprechenden Säuren umsetzt.

Das erfindungsgemäße Verfahren kann durch folgendes Schema erläutert werden:

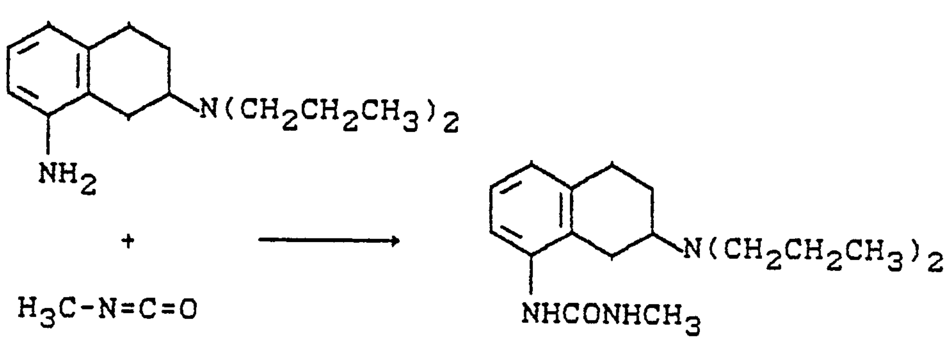

Als inerte Lösemittel eignen sich die üblichen organischen Lösemittel, die sich unter den Reaktionsbedingungen nicht verändern. Hierzu gehören bevorzugt Ether wie Diethylether, Butylmethylether, Dioxan, Tetrahydrofuran oder Glykoldimethylether, oder Halogenkohlenwasserstoffe wie beispielsweise Methylenchlorid, Chloroform, Tetrachlorkohlenstoff, Dichlorethan, Dichlorethylen oder Trichlorethylen, oder Kohlenwasserstoffe wie Benzol, Toluol, Xylol oder Erdölfraktionen, oder Amide wie Dimethylformamid oder Hexamethylphosphorsäuretriamid, oder Essigester, Acetonitril oder Pyridin. Ebenso ist es möglich, Gemische der genannten Lösemittel einzusetzen.

Als Basen eignen sich hierbei die üblichen organischen Amine. Hierzu gehören bevorzugt Trialkylamine wie beispielsweise Triethylamin oder Tripropylamin, oder tertiäre organische Basen wie beispielsweise Pyridin, N,N-Dimethylaminopyridin, Picolin, Piperidin, Morpholin oder 1,5-Diazabicyclo[4,3,0]non-5-en oder 1,5-Diazabicyclo[5,4,0]undec-5-en.

Die Umsetzung erfolgt im allgemeinen in einem Temperaturbereich von -30° C bis +100 °C, bevorzugt von 0° C bis +80° C.

Die Umsetzung wird im allgemeinen bei Normaldruck durchgeführt. Es ist ebenso möglich die Umsetzung bei erhöhtem oder erniedrigtem Druck durchzuführen (z.B. von 0,5 bis 5 bar).

Bei der Durchführung des erfindungsgemäßen Verfahrens werden die Isocyanate im allgemeinen in einer Menge von 1 bis 3, bevorzugt von 1 bis 2 Mol bezogen auf 1 Mol des 8-Aminotetralins eingesetzt. Die Base wird im allgemeinen in einer Menge von 0,01 bis 1 Mol, bevorzugt von 0,1 bis 0,5 Mol bezogen auf 1 Mol des Isocyanats eingesetzt.

Das Verfahren kann beispielsweise durchgeführt werden, indem man das 8-Aminotetralin in einem inerten Lösemittel mit Isocyanat und Base vermischt und gegebenenfalls erwärmt. Die Aufarbeitung erfolgt durch Extraktion, Chromatographie und/oder Kristallisation.

Im Fall der Herstellung der unsubstituierten 8-Ureidoaminotetraline ($R^9$ = H) setzt man Alkalicyanate, bevorzugt Natrium-oder Kaliumcyanat in Wasser und/oder Säuren wie Salzsäure, Bromwasserstoffsäure oder Schwefelsäure ein.

Außerdem wurde ein Verfahren zur Herstellung der erfindungsgemäßen 8-Acyl-aminotetraline der allgemeinen Formel (Id)

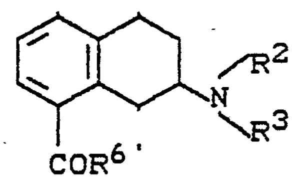

(Id)

in welcher
$R^2$ -für Wasserstoff oder Alkyl steht,
$R^3$ -für Alkyl steht
und
$R^{6'}$ für Hydroxy, Amino, Alkoxy, Aryloxy oder Aralkoxy steht,
und deren Salze gefunden,
daß dadurch gekennzeichnet ist, daß man
8-Cyanotetraline oder allgemeinen Formel (VIII)

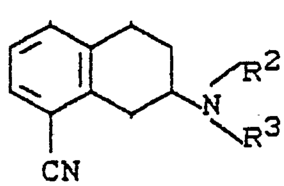

(VIII)

in welcher
$R^2$ und $R^3$ die oben angegebene Bedeutung haben hydrolysiert,
im Fall der Herstellung der Carbonsäureester die erhaltenen Carbonsäuren verestert,
und im Fall der Herstellung der Salze mit den entsprechenden Säuren umsetzt.

Das erfindungsgemäße Verfahren kann durch folgendes Schema erläutert werden:

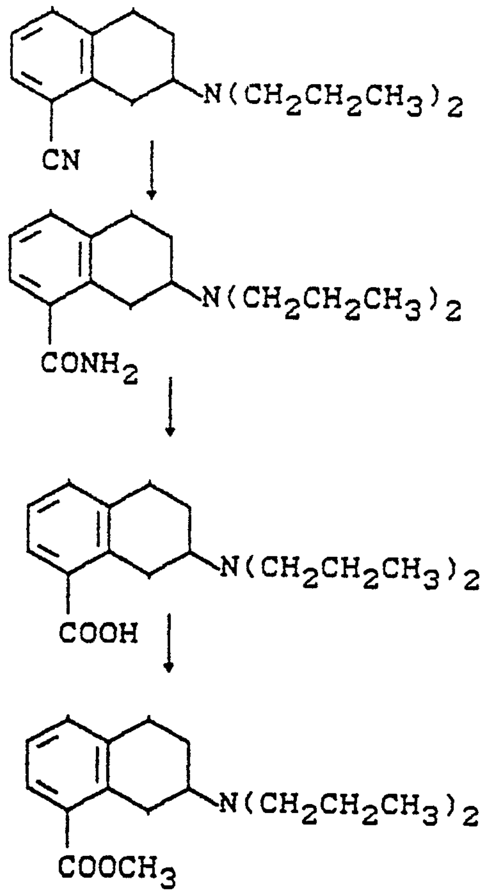

Bei der Durchführung des erfindungsgemäßen Verfahrens entstehen im allgemeinen bei der Hydrolyse zunächst die Amide und dann die Carbonsäuren. Die Carbonsäuren können auch ohne Isolierung der Amide hergestellt werden. Aus den Carbonsäuren erhält man durch Veresterung die erfindungsgemäßen Ester. Auch hier kann man ohne Isolierung der Amide bzw. der Carbonsäuren arbeiten. Bevorzugt werden die Carbonsäuren ohne Isolierung der Amide in einem Schritt hergestellt, die Carbonsäureester dagegen aus den isolierten Carbonsäuren.

Die Hydrolyse zu den erfindungsgemäßen Amiden bzw. Carbonsäuren erfolgt im allgemeinen mit Wasser in inerten Lösemitteln in Anwesenheit von Basen.

Als inerte Lösemittel eignen sich hierbei Wasser oder Alkohole wie Methanol, Ethanol, Propanol, Isopropanol, Butanol oder Glykol, oder Amide wie Dimethylformamid oder Hexamethylphosphorsäuretriamid, oder Ether wie Dioxan, Tetrahydrofuran, Glykolmonomethylether, Glykoldimethylether oder Diethylglykoldimethylether. Ebenso ist es möglich Gemische der genannten Lösemittel einzusetzen.

Als Basen eignen sich bei der Hydrolyse die üblichen basischen Verbindungen. Hierzu gehören bevorzugt Alkali-oder Erdalkalihydroxide wie Natriumhydroxid, Kaliumhydroxid oder Bariumhydroxid, oder Alkalicarbonate wie beispielsweise Natriumcarbonat, Natriumhydrogencarbonat oder Kaliumcarbonat, oder Alkalialkoholate wie beispielsweise Natriummethanolat, Natriumethanolat, Kaliummethanolat, Kaliumethanolat oder Kalium-tert.butanolat.

Die Hydrolyse wird im allgemeinen in einem Temperaturbereich von 0° C bis +200° C, bevorzugt von +20° C bis +150° C durchgeführt.

Die Hydrolyse wird im allgemeinen bei Normaldruck durchgeführt. Es ist ebenso möglich, die Umsetzung bei erhöhtem oder bei erniedrigtem Druck durchzuführen (z.B. von 0,5 bis 5 bar).

Bei der Durchführung der erfindungsgemäßen Hydrolyse werden die Basen im allgemeinen in einer Menge von 1 bis 10, bevorzugt von 1 bis 5 Mol bezogen auf 1 Mol der 8-Cyano-aminotetraline eingesetzt.

Die Veresterung der erfindungsgemäßen Carbonsäuren zu den erfindungsgemäßen Carbonsäureestern erfolgt im allgemeinen mit den entsprechenden Alkoholen in Anwesenheit von Säuren in inerten Lösemitteln.

Als inerte Lösemittel eignen sich hierbei die üblichen organischen Lösemittel, die sich unter den Reaktionsbedingungen nicht verändern. Hierzu gehören bevorzugt Alkohole wie Methanol, Ethanol, Propanol, Isopropanol oder Butanol, oder Ether wie Diethylether, Dioxan oder Tetrahydrofuran, oder Halogenkohlenwasserstoffe wie Methylenchlorid, Chloroform oder Tetrachlorkohlenstoff. Ebenso ist es möglich Gemische der genannten Lösemittel einzusetzen.

Als Säuren eignen sich bei der Veresterung die üblichen anorganischen Säuren. Hierzu gehören bevorzugt Mineralsäuren wie Chlorwasserstoff-, Bromwasserstoff-, Schwefelsäure oder Phosphorsäure.

Die Veresterung wird im allgemeinen in einem Temperaturbereich von +10° C bis + 150° C, bevorzugt von +20° C bis +100° C durchgeführt.

Die Veresterung wird im allgemeinen bei Normaldruck durchgeführt. Es ist ebenso möglich, die Veresterung bei erhöhtem oder erniedrigtem Druck durchzuführen (z.B. bei0,5 bis 5 bar).

Bei der Durchführung der Veresterung werden die Säuren im allgemeinen in einer Menge von 1 bis 50, bevorzugt von 1 bis 20 Mol bezogen auf 1 Mol der Carbonsäure eingesetzt. Die Alkohole werden im allgemeinen im Überschuß eingesetzt. Es hat sich hierbei als günstig erwiesen, die Alkohole mit denen verestert wird, gleichzeitig als Lösemittel einzusetzen.

Bevorzugt wird als Säure eine etherische oder alkoholische Chlorwasserstofflösung verwendet, wobei als Alkohol wiederum der Alkohol verwendet wird, mit dem die Carbonsäure verestert wird.

Das erfindungsgemäße Verfahren kann beispielsweise folgendermaßen durchgeführt werden:

Das 8-Cyano-aminotetralin wird zusammen mit einer Base in einem inerten Lösemittel erwärmt, wobei die Länge der Reaktion und die Höhe der Temperatur abhängig ist davon, ob das Carbonsäureamid oder die Carbonsäure hergestellt werden soll. Zur Veresterung wird die entsprechende Carbonsäure in einem inerten Lösemittel in Anwesenheit einer Säure erwärmt, wobei gegebenenfalls das entstehende Reaktionswasser mit dem Lösemittel abdestilliert werden kann.

Die als Ausgangsstoffe eingesetzten 8-Cyano-aminotetraline sind neu und können nach dem oben beschriebenen Verfahren hergestellt werden.

Als 8-Cyano-aminotetraline können beispielsweise erfindungsgemäß verwendet werden:

8-Cyano-2-dimethylamino-1,2,3,4-tetrahydronaphthalin,
8-Cyano-2-diethylamino-1,2,3,4-tetrahydronaphthalin,
8-Cyano-2-dipropylamino-1,2,3,4-tetrahydronaphthalin,
8-Cyano-2-(N-ethyl-N-methyl)amino-1,2,3,4-tetrahydronaphthalin,
8-Cyano-2-(N-ethyl-N-propyl)amino-1,2,3,4-tetrahydronaphthalin,
8-Cyano-2-(N-methyl-N-propyl)amino-1,2,3,4-tetrahydronaphthalin.

Außerdem wurde ein Verfahren zur Herstellung der erfindungsgemäßen 8-Formyl-aminotetraline der allgemeinen Formel (Ie)

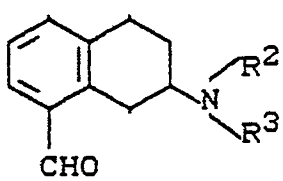

(Ie)

in welcher
$R^2$ -für Wasserstoff oder Alkyl steht,
und
$R^3$ -für Alkyl steht
und deren Salze gefunden,
das dadurch gekennzeichnet ist, daß man
8-Halogen-substituierte 2-Aminotetraline der allgemeinen Formel (IX)

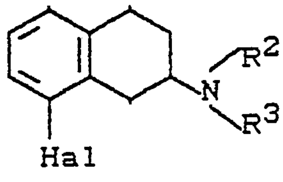

(IX)

in welcher
$R^2$ und $R^3$ die oben angegebene Bedeutung haben
und
Hal für Fluor, Chlor, Brom, Iod, bevorzugt für Chlor oder Brom steht,
in inerten Lösemitteln, gegebenenfalls in Anwesenheit von Hilfsstoffen mit Magnesium und Formamiden der

allgemeinen Formel (X)

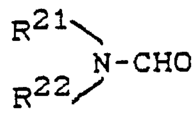

(X),

in welcher
$R^{21}$ und $R^{22}$ gleich oder verschieden sind und
-für Methyl, Ethyl, Propyl, Phenyl oder Pyridyl stehen, oder
$R^{21}$ und $R^{22}$ gemeinsam mit dem Stickstoffatom einen Piperidinring bilden,
umsetzt
und dann im Fall der Herstellung der Salze mit den entsprechenden Säuren umsetzt.

Das erfindungsgemäße Verfahren kann durch folgendes Schema erläutert werden:

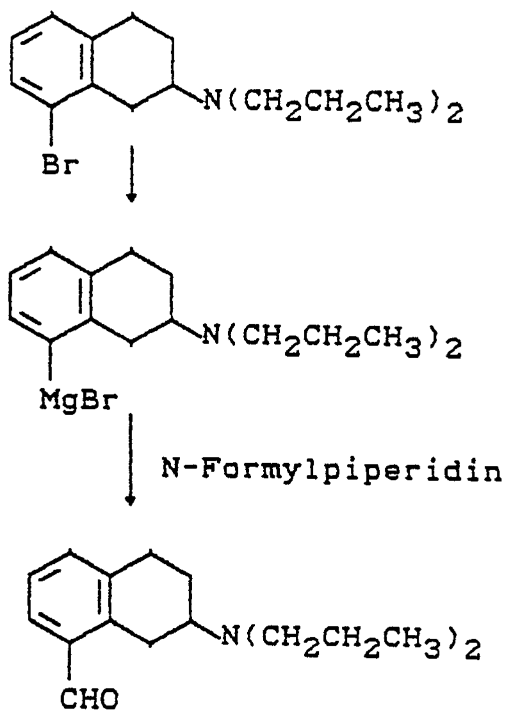


Als inerte Lösemittel eignen sich hierbei die üblichen organischen Lösemittel, die sich unter den Reaktionsbedingungen nicht verändern. Hierzu gehören bevorzugt Ether wie Diethylether, Butylmethylether, Dioxan oder Tetrahydrofuran, oder Kohlenwasserstoffe wie Benzol, Toluol oder Xylol, oder Amide wie Dimethylformamid oder Hexamethylphosphorsäuretriamid. Ebenso ist es möglich, Gemische der genannten Lösemittel einzusetzen.

Als Hilfsstoffe werden im allgemeinen Stoffe eingesetzt, wie sie für die Aktivierung einer Grignard-Reaction üblich sind. Hierzu gehören bevorzugt Iod oder iodorganische Verbindungen oder Anthracen, bevorzugt Iod oder Iodethan.

Die Umsetzung wird im allgemeinen in einem Temperaturbereich von -30° C bis +100° C, bevorzugt von 0° C bis +50° C durchgeführt.

Die Umsetzung wird im allgemeinen bei Normaldruck durchgeführt. Es ist ebenso möglich die Umsetzung bei erhöhtem oder erniedrigtem Druck durchzuführen (z.B. von 0,5 bis 5 bar).

Bei der Durchführung des erfindungsgemäßen Verfahrens werden die Formamide im allgemeinen in einer Menge von 1 bis 5 Mol, bevorzugt von 1 bis 2 Mol bezogen auf 1 Mol der Ausgangsverbindung eingesetzt.

Das erfindungsgemäße Verfahren kann beispielsweise folgendermaßen durchgeführt werden:
Magnesiumpulver oder Magnesiumspäne werden in einem geeigneten Lösemittel vorgelegt, eine Lösung des 8-Halogen-sub stituierten 2-Aminotetralins in einem geeigneten Lösemittel wird zugetropft und die Reaktionsmischung anschließend mit dem entsprechenden Formamid, gegebenenfalls gelöst in einem inertem Lösemittel, versetzt. Nach Hydrolyse der Reaktionsmischung wird durch Extraktion Chromatographie und/oder Kristallisation aufgearbeitet.

Die als Ausgangsstoffe eingesetzten 8-Halogen-substituierten 2-Aminotetraline sind neu und können nach dem oben bereits beschriebenen Verfahren hergestellt werden.

Als 8-Halogen-substituierte 2-Aminotetraline können beispielsweise erfindungsgemäß verwendet werden:

8-Brom-2-dimethylamino-1,2,3,4-tetrahydronaphthalin, 8-Brom-2-diethylamino-1,2,3,4-tetrahydronaphthalin,
8-Brom-2-dipropylamino-1,2,3,4-tetrahydronaphthalin,
8-Chlor-2-dimethylamino-1,2,3,4-tetrahydronaphthalin,
8-Chlor-2-diethylamino-1,2,3,4-tetrahydronaphthalin,
8-Chlor-2-dipropylamino-1,2,3,4-tetrahydronaphthalin,
8-Brom-2-(N-ethyl-N-methyl)amino-1,2,3,4-tetrahydronaphthalin,
8-Brom-2-(N-ethyl-N-propyl)amino-1,2,3,4-tetrahydronaphthalin,
8-Brom-2-(N-methyl-N-propyl)amino-1,2,3,4-tetrahydronaphthalin,
8-Chlor-2-(N-ethyl-N-methyl)amino-1,2,3,4-tetrahydronaphthalin,
8-Chlor-2-(N-ethyl-N-propyl)amino-1,2,3,4-tetrahydronaphthalin,
8-Chlor-2-(N-methyl-propyl)amino-1,2,3,4-tetrahydronaphthalin.

Außerdem wurde ein Verfahren zur Herstellung der erfindungsgemäßen 8-Methylen-aminotetraline der allgemeinen Formel (If)

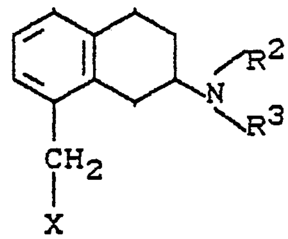

(If)

in welcher
$R^2$ -für Wasserstoff oder Alkyl steht,
$R^3$ -für Alkyl steht
und
X - eine Gruppe der Formel $-NR^{12}R^{13}$ , $-COR^{14}$ , $-SO_2R^{15}$ oder $-OR^{16}$ bedeutet,
worin
$R^{12}$ und $R^{13}$ gleich oder verschieden sind und
-für Wasserstoff, Alkyl, Aryl oder Aralkyl stehen, wobei die Arylreste durch Halogen, Cyano, Alkyl, Alkoxy oder Trifluormethyl substituiert sein können, oder
-für Gruppe der Formel $-COR^{14}$ , $-SO_2R^{15}$ oder $-(CH_2)_c-NR^{12}R^{13}$ stehen,
$R^{14}$ -Wasserstoff bedeutet, oder
-eine Gruppe $-NHR^{17}$ bedeutet, oder
- Alkyl oder Alkoxy bedeutet, oder
-Aryl, Aryloxy, Aralkyl, Aralkoxy oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
$R^{15}$ -Cycloalkyl bedeutet, oder
-Alkyl bedeutet, das durch Cyano, Halogen, Trifluormethyl, Trifluormethoxy oder Alkoxycarbonyl substituiert sein kann, oder
-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
-eine Gruppe $-NR^{10}R^{11}$ bedeutet,
wobei
$R^{10}$ und $R^{11}$ gleich oder verschieden sind und
-für Wasserstoff, Alkyl, Aryl oder Aralkyl stehen,
$R^{16}$ -Wasserstoff, Alkyl, Aryl, Aralkyl oder eine Gruppe der Formel $-COR^{10}R^{11}$ bedeutet.
$R^{17}$ -Wasserstoff bedeutet, oder
-Cycloalkyl bedeutet, oder
-gegebenenfalls durch Cyano, Halogen, Trifluormethyl oder Trifluormethoxy substituiertes Alkyl bedeutet, oder

-Aryl, Aralkyl oder Heteroaryl bedeutet, wobei die Arylrest bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
und
c - eine Zahl 1 bis 8 bedeutet,
oder wobei
$R^{12}$ und $R^{13}$ zusammen mit dem Stickstoffatom einen Ring der Reihe

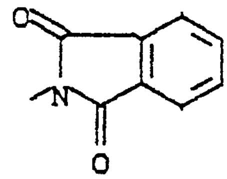 , 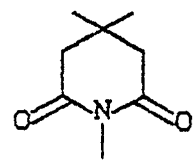 , 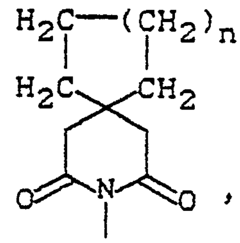 ,

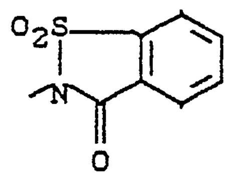 , 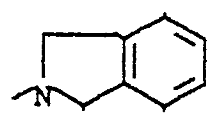 , 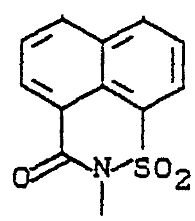 ,

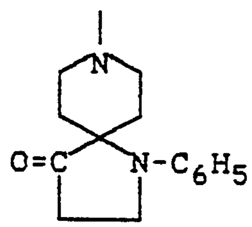 , 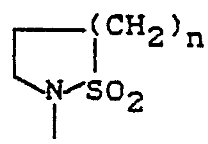 , 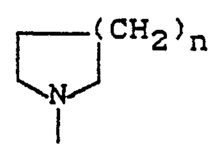

oder 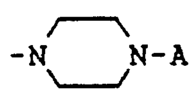

bilden,
worinn - eine Zahl 1 oder 2 bedeutet,
und
A - für Wasserstoff oder Cycloalkyl steht, oder
- für Alkyl steht, das durch Halogen, Hydroxy, Amino, Alkylamino, Dialkylamino, Carbamoyl oder Sulfamoyl substituiert sein kann, oder
-für Aryl, Heteroaryl, Aralkyl, Alkoxycarbonyl, Alkylsulfonyl, Phenylsulfonyl, Tolylsulfonyl, Benzylsulfonyl, Formyl, Carbamoyl oder Sulfamoyl steht,
$R^2$ -für Wasserstoff oder Alkyl steht,
und
$R^3$ -für Alkyl steht,
wobei jedoch
$R^1$ nicht $NH_2$ bedeutet, wenn
$R^2$ und $R^3$ Propyl bedeuten,
und deren Salze gefunden,
das dadurch gekennzeichnet ist, daß man Tetraline der allgemeinen Formel (XI)

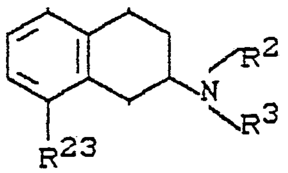

(XI)

in welcher
$R^2$ -für Wasserstoff oder Alkyl steht,
$R^3$ -für Alkyl steht
und
$R^{23}$ für eine Gruppe der Formel $-COR^{24}$ oder -CN steht,
worin
$R^{24}$ -Wasserstoff, Hydroxy, Alkoxy oder Amino bedeutet,
in inerten Lösemitteln reduziert,
dann gegebenenfalls funktionelle Gruppen durch Reduktion, Hydrolyse, Oxidation oder Umsetzung mit elektrophilen Reagenzien in andere funktionelle Gruppen überführt,
und dann im Fall der Herstellung der Salze mit der entsprechenden Säure umsetzt.

Das erfindungsgemäße Verfahren kann beispielsweise durch folgende Formelschemata erläutert werden:

a)

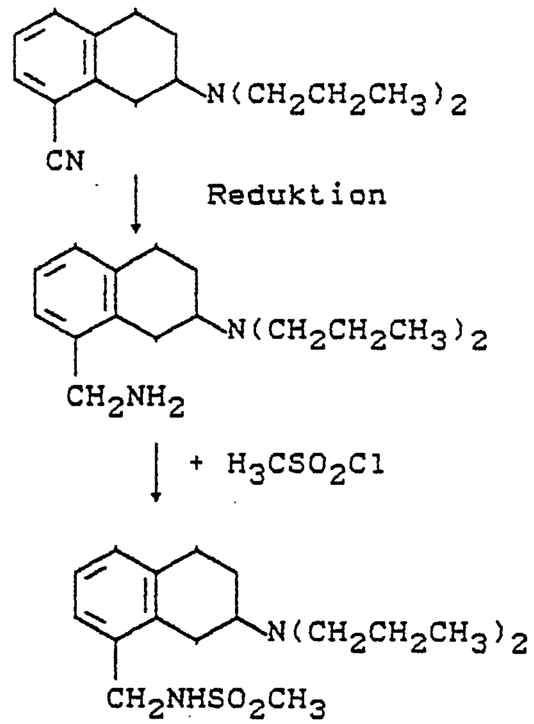


b)

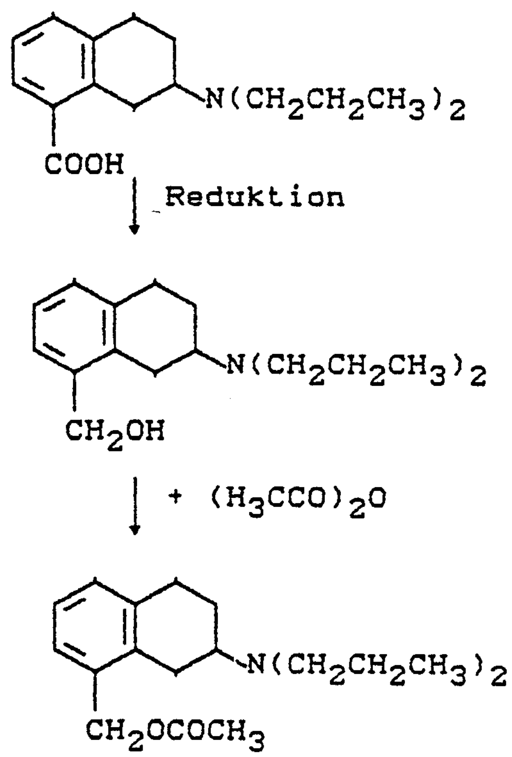


Die Reduktion erfolgt entweder durch Wasserstoff in Wasser oder inerten organischen Lösemitteln wie Alkoholen, Ethern oder Halogenkohlenwasserstoffen oder deren Gemischen, mit Katalysatoren wie Metallen oder Edelmetallen oder deren Salze, wie beispielsweise Raney-Nickel, Palladium, Palladium auf Tierkohle, Platin oder Platinoxid, oder aber mit Hydriden in inerten Lösemitteln, gegebenenfals in Anwesenheit eines Katalysators.

Bevorzugt wird die Reduktion mit Metallhydriden oder komplexen Metallhydriden wie Aluminiumhydriden oder komplexen Borhydriden oder Aluminiumhyriden durchgeführt. Besonders bevorzugt werden hierbei Natriumborhydrid, Lithiumaluminiumhydrid, Natriumaluminiumhydrid, Aluminiumhydrid, Natriumcyanoborhydrid oder Lithium-trimethoxy-hydrido-aluminat, gegebenenfalls in Anwesenheit von Aluminiumchlorid eingesetzt.

Als Lösemittel eignen sich alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Diethylenglykoldimethylether, oder Amide wie Hexamethylphosphorsäuretriamid oder Dimethylformamid, oder Essigsäure, Trichloressigsäure oder Trifluoressigsäure, oder Halogenkohlenwasserstoffe wie Methylenchlorid, Chloroform oder Tetrachlorkohlenstoff. Ebenso ist es möglich, Gemische der genannten Lösemittel zu verwenden.

Als Katalysatoren bei der Reduktion werden im allgemeinen Säuren verwendet. Hierzu gehören bevorzugt anorganische Säuren wie Salzsäure, Bromwasserstoffsäure oder Schwefel säure, oder organische Carbonsäuren mit 1 bis 4 Kohlenstoffatomen, oder Sulfonsäuren mit 1 bis 4 Kohlenstoffatomen wie beispielsweise Ameisensäure, Essigsäure, Propionsäure, Trichloressigsäure, Trifluoressigsäure, Methansulfonsäure, Ethansulfonsäure, Benzolsulfonsäure oder Toluolsulfonsäure.

Besonders bevorzugt wird die Reduktion in inerten Lösemitteln, bevorzugt in Essigester oder in Alkoholen wie beispielsweise Methanol, Ethanol, Propanol oder Isopropanol oder deren Gemischen, in Anwesenheit von anorganischen oder organischen Säuren wie beispielsweise Chlorwasserstoffsäure oder Essigsäure und in Anwesenheit eines Reduktionsmittels, bevorzugt von komplexen Hydriden wie beispielsweise Natriumborhydrid, Lithiumaluminiumhydrid oder Natriumcyanoborhydrid, durchgeführt.

Die Reduktion wird im allgemeinen in einem Temperaturbereich von -20° C bis +100° C, bevorzugt von 0° C bis +80° C durchgeführt.

Die Reduktion wird im allgemeinen bei Normaldruck durchgeführt. Es ist ebenso möglich die Umsetzung bei erniedrigtem oder erhöhtem Druck durchzuführen (z.B. von 0,5 bis 5 bar).

Bei der Durchführung der Reduktion wird im allgemeinen das Reduktionsmittel in einer Menge von 1 bis 6, bevorzugt von 1 bis 3 Mol bezogen auf 1 Mol der Ausgangsverbindung eingesetzt.

Das erfindungsgemäße Verfahren kann beispielsweise durchgeführt werden, indem man das Reduktionsmittel in einem inerten Lösemittel mit den Tetralinen, gegebenenfalls in einem inertem Lösemittel versetzt, und gegebenenfalls erwärmt. Die Aufarbeitung erfolgt in üblicher Weise durch Extraktion, Chromatographie und/oder Kristallisation.

Außerdem wurde eine Verfahren zur Herstellung der erfindungsgemäßen 8-Alkylen-aminotetraline der allgemeinen Formel (Ig)

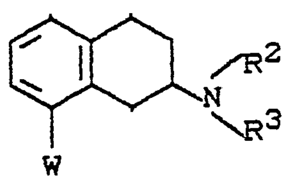

(Ig),

in welcher

$R^2$ -für Wasserstoff oder Alkyl steht,

$R^3$ -für Alkyl steht

und

W - für eine Gruppe der Formel $-(CH_2)_{a'}-X$ oder $-CH=CH-(CH_2)_b-X$ steht,

worin

X - eine Gruppe der Formel $-NR^{12}R^{13}$, $-COR^{14}$, $-SO_2R^{15}$ oder $-OR^{16}$ bedeutet,

worin

$R^{12}$ und $R^{13}$ gleich oder verschieden sind und

-für Wasserstoff, Alkyl, Aryl oder Aralkyl stehen, wobei die Arylreste durch Halogen, Cyano, Alkyl, Alkoxy oder Trifluormethyl substituiert sein können,

oder

-für eine Gruppe der Formel $-COR^{14}$, $-SO_2R^{15}$ oder $-(CH_2)_c-NR^{12}R^{13}$ stehen,

$R^{14}$ -Wasserstoff bedeutet, oder

-eine Gruppe $-NHR^{17}$ bedeutet, oder

-Alkyl oder Alkoxy bedeutet, oder

-Aryl, Aryloxy, Aralkyl, Aralkoxy oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,

$R^{15}$ -Cycloalkyl bedeutet, oder

-Alkyl bedeutet, das durch Cyano, Halogen, Trifluormethyl, Trifluormethoxy oder Alkoxycarbonyl substituiert sein kann, oder

-Aryl, Aralkyl oder Heteroaryl bedeutet, wobei die genannten Reste bis zu 3-fach gleich oder verschieden

durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
-eine Gruppe -$NR^{10}R^{11}$ bedeutet,
wobei
$R^{10}$ und $R^{11}$ die oben angegebene Bedeutung haben,
$R^{16}$ -Wasserstoff, Alkyl, Aryl, Aralkyl oder eine Gruppe der Formel -$CONR^{10}R^{11}$ bedeutet,
$R^{17}$ -Wasserstoff bedeutet, oder
-Cycloalkyl bedeutet, oder
-gegebenenfalls durch Cyano, Halogen, Trifluormethyl oder Trifluormethoxy substituiertes Alkyl bedeutet, oder
-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
und
c - eine Zahl 1 bis 8 bedeutet,
oder wobei
$R^{12}$ und $R^{13}$ zusammen mit dem Stickstoffatom einen Ring der Reihe

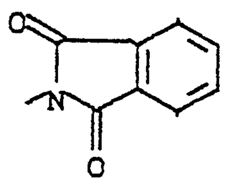 , 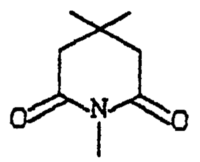 , 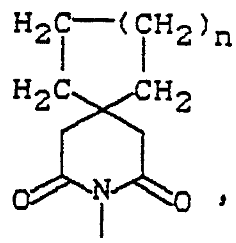 ,

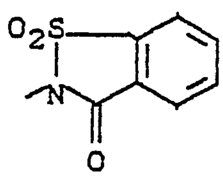 , 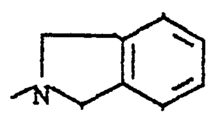 , 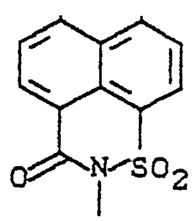 ,

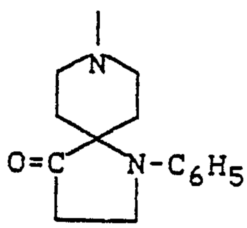 , 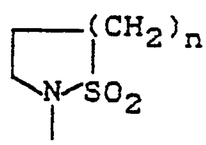 , 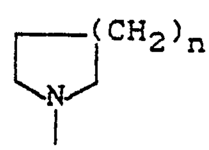

oder 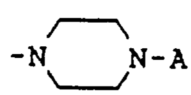

bilden,
worin
n - eine Zahl 1 oder 2 bedeutet,
und
A - für Wasserstoff oder Cycloalkyl steht,
oder
-für Alkyl steht, das durch Halogen, Hydroxy, Amino, Alkylamino, Dialkylamino, Carbamoyl oder Sulfamoyl substituiert sein kann,
oder
-für Aryl, Heteroaryl, Aralkyl, Alkoxycarbonyl, Alkylsulfonyl, Phenylsulfonyl, Tolylsulfonyl, Benzylsulfonyl, Formyl, Carbamoyl oder Sulfamoyl steht,
a' -eine Zahl 2 bis 10 bedeutet

und
b - eine Zahl 0 bis 8 bedeutet
und deren Salze gefunden,
das dadurch gekennzeichnet ist, daß man
8-Formyl-aminotetraline der allgemeinen Formel (Ie)

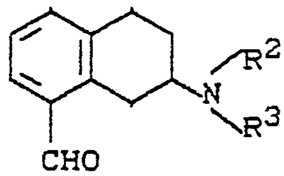

(Ie),

in welcher
$R^2$ und $R^3$ die angegebene Bedeutung haben,
in inerten Lösemitteln in Anwesenheit von Basen mit Phosphorverbindungen der allgemeinen Formel (XII)
$U-(CH_2)_{a'-1}-X'$ (XII)
in welcher
X' - die für X angegebene Bedeutung hat, oder -für Nitro steht,
a' -die oben angegebene Bedeutung hat
und
U - für eine Gruppe der Formel

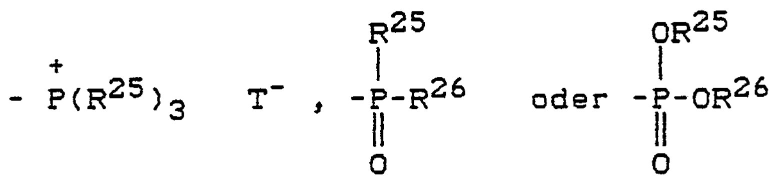

, oder steht,

wobei
$R^{25}$ und $R^{26}$ gleich oder verschieden sind und Alkyl oder Phenyl bedeuten
und
T - ein Halogenidanion, bevorzugt Chlorid, Bromid oder Iodid bedeutet,
umsetzt,
dann gegebenenfalls funktionelle Gruppen durch Reduktion, Hydrolyse, Oxidation oder Umsetzung mit elektrophilen Reagenzien in andere funktionelle Gruppen überführt und dann im Fall der Herstellung der Salze mit den entsprechenden Säuren umsetzt.

Das erfindungsgemäße Verfahren kann durch folgendes Formelschema erläutert werden:

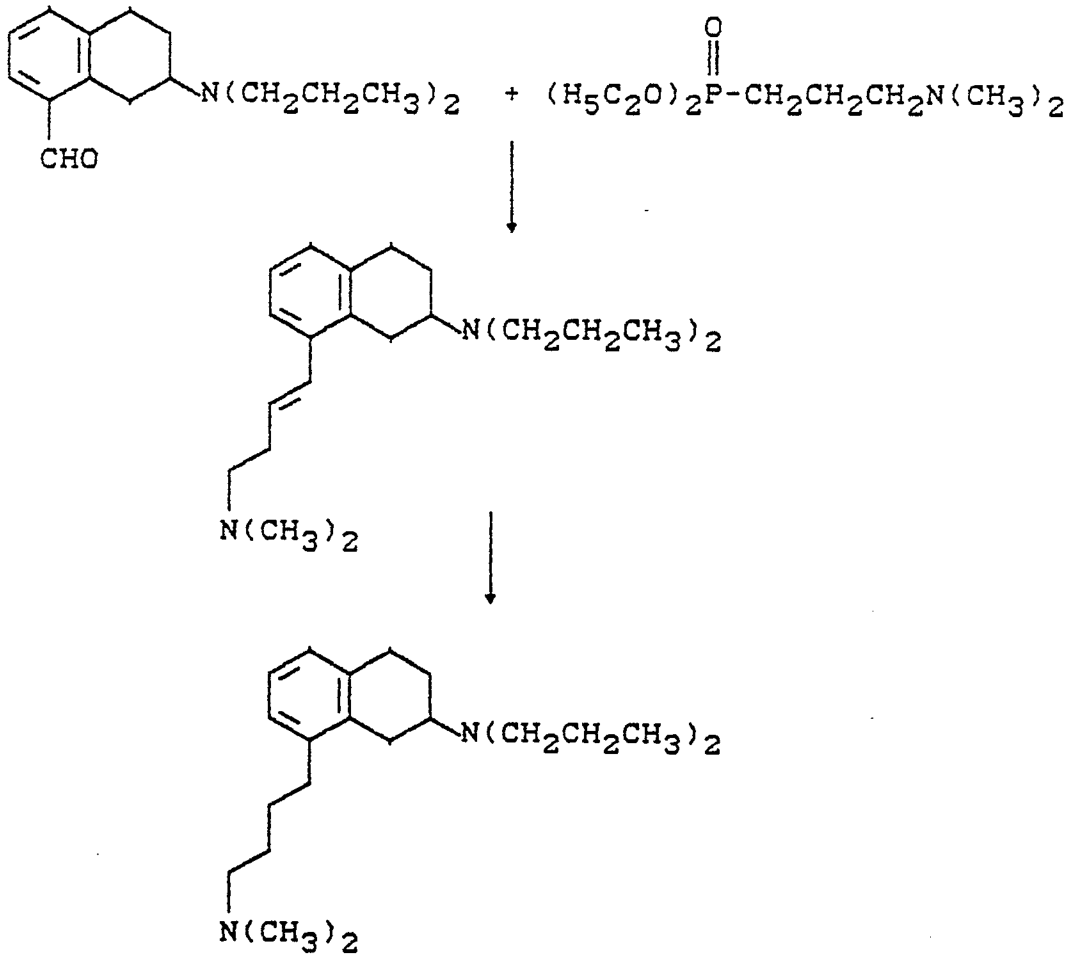

Als inerte Lösemittel eignen sich hierbei die üblichen organischen Lösemittel, die sich unter den Reaktionsbedingungen nicht verändern. Hierzu gehören bevorzugt Ether wie Diethylether, Butylmethylether, Dioxan, Tetrahydrofuran, Glykoldimethylether oder Diethylenglykoldimethylether, oder Kohlenwasserstoffe wie Benzol, Toluol, Xylol, oder Erdölfraktionen, oder Amide wie Dimethylformamid oder Hexamethylphosphorsäuretriamid, oder Alkohole wie Methanol, Ethanol, Propanol oder Isopropanol, oder Dimethylsulfoxid. Ebenso ist es möglich Gemische der genannten Lösemittel zu verwenden.

Als Basen eignen sich die üblichen basischen Verbindungen, für basische Reaktionen. Hierzu gehören bevorzugt Alkali-oder Erdalkalihydroxide wie beispielsweise Natriumhydroxid, Kaliumhydroxid oder Bariumhydroxid, oder Alkalicarbonat wie Natriumcarbonat, Natriumhydrogencarbonat oder Kaliumcarbonat, oder Alkalialkoholate wie Natriummethanolat, Natriumethanolat, Kaliummethanolat, Kaliumethanolat oder Kalium-tert.butylat, oder Amide wie Natriumamid oder Lithiumdiisopropylamid, oder lithiumorganische Verbindungen wie Phenyllithium oder Butyllithium.

Die Wahl des Lösemittel bzw. der Base richtet sich nach Stabilität, Hydrolyseempfindlichkeit bzw. CH-Azidität der entsprechenden Phosphorverbindung. Besonders bevorzugt verwendet man als Lösemittel Ether wie Diethylether, Tetrahydrofuran oder Dioxan, oder Kohlenwasserstoffe wie Benzol, Toluol oder Xylol oder Dimethylformamid. Als Basen verwendet man besonders bevorzugt Alkalialkoholate wie Natriummethanolat, Natriumethanolat, Kaliummethanolat, Kaliumethanolat oder Kalium-tert.butylat, oder lithiumorganische Verbindungen wie Phenyllithium oder Butyllithium.

Die Umsetzung wird im allgemeinen in einem Temperaturbereich von -30° C bis +150° C, bevorzugt von 0° C bis +100° C durchgeführt.

Die Umsetzung kann bei normalem, erhöhtem oder bei erniedrigtem Druck durchgeführt werden (z.B. 0,5 bis 5 bar). Im allgemeinen arbeitet man bei Normaldruck.

Bei der Durchführung der Reaktion werden im allgemeinen die Phosphorverbindungen in einer Menge von 1 bis 2 Mol, bevorzugt in molaren Mengen bezogen auf 1 Mol der 8-Formyl-aminotetraline eingesetzt. Die Basen werden im allgemeinen in einer Menge von 1 bis 5, bevorzugt von 1 bis 2 Mol bezogen auf 1 Mol der Phosphorverbindung eingesetzt.

Das erfindungsgemäße Verfahren kann beispielsweise durchgeführt werden, indem man zu den Phosphorverbindungen, in einem geeigneten Lösemittel gelöst oder suspendiert, die Base und dann die 8-Formyl-aminotetraline, gegebenenfalls in einem geeigneten Lösemittel, zugibt und gegebenenfalls erwärmt. Die Aufarbeitung erfolgt in üblicher Weise durch Extraktion, Chromatographie und/oder Kristallisation.

Bei der Durchführung des erfindungsgemäßen Verfahrens ist es ebenso möglich, anstelle der Phosphoniumsalze (U = $-P(R^{25})_3$ T ) die entsprechenden Phosphorane [$(R^{25})_3P=CH-(CH_2)_{a'-2}-X$] direkt einzusetzen, die man zuvor aus den ensprechenden Phosphoniumsalzen und Basen in einer gesonderten Umsetzung hergestellt hat. Es hat sich jedoch als günstig erwiesen, die Umsetzung mit den Phosphorverbindungen in Anwesenheit von Basen als Eintopfverfahren durchzuführen. Als besondere Variante eines Eintopfverfahrens kann die Umsetzung je nach der Stabilität der Phosphorverbindungen auch in Form einer phasentransferkatalysierten Reaktion durchgeführt werden, wobei als Lösemittel Ether, Kohlenwasserstoffe sowie Halogenkohlenwasserstoffe verwendet werden können und als Basen wäßrige Natriumhydroxid-oder Kaliumhydroxidlösungen eingesetzt werden.

Die als Ausgangstoffe eingesetzten Phosphorverbindungen der allgemeinen Formel (XII) sind bekannt oder können nach bekannten Methoden hergestellt werden [Houben-Weyl's "Methoden der organischen Chemie" Bd XII/1, 33, 167].

Außerdem wurde eine Verfahrensvariante zur Herstellung der erfindungsgemäße 8-Ethylen-aminotetraline der allgemeinen Formel (Ih)

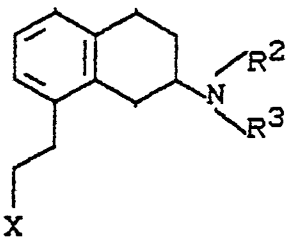

(Ih)

in welcher

$R^2$ -für Wasserstoff oder Alkyl steht,

$R^3$ -für Wasserstoff oder Alkyl steht

und

X - eine Gruppe der Formel $-NR^{12}R^{13}$, $-COR^{14}$, $-SO_2R^{15}$ oder $-OR^{16}$ bedeutet,

worin

$R^{12}$ und $R^{13}$ gleich oder verschieden sind und

-für Wasserstoff, Alkyl, Aryl oder Aralkyl stehen, wobei die Arylreste durch Halogen, Cyano, Alkyl, Alkoxy oder Trifluormethyl substituiert sein können,

oder

-für eine Gruppe der Formel $-COR^{14}$, $-SO_2R^{15}$ oder $-(CH_2)_c-NR^{12}R^{13}$ stehen,

$R^{14}$ -Wasserstoff bedeutet, oder

-eine Gruppe $-NHR^{17}$ bedeutet, oder

- Alkyl oder Alkoxy bedeutet, oder

-Aryl, Aryloxy, Aralkyl, Aralkoxy oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,

$R^{15}$ -Cycloalkyl bedeutet, oder

-Alkyl bedeutet, das durch Cyano, Halogen, Trifluormethyl, Trifluormethoxy oder Alkoxycarbonyl substituiert sein kann, oder

-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder

-eine Gruppe $-NR^{10}R^{11}$ bedeutet,

wobei

$R^{10}$ und $R^{11}$ die oben angegebene Bedeutung haben,

$R^{16}$ -Wasserstoff, Alkyl, Aryl, Aralkyl oder eine Gruppe der Formel - $CONR^{10}R^{11}$ bedeutet,

$R^{17}$ -Wasserstoff bedeutet, oder

-Cycloalkyl bedeutet, oder

-gegebenenfalls durch Cyano, Halogen, Trifluormethyl oder Trifluormethoxy substituiertes Alkyl bedeutet, oder

-Aryl, Aralkyl oder Heteroaryl bedeutet, wobei die genannten Reste bis zu 3-fach gleich oder verschieden

durch Alkyl, Alkoxy, Alkylthio, Halogen, Cyano, Trifluormethyl, Trifluormethoxy, Trifluormethylthio, Amino, Alkylamino oder Dialkylamino substituiert sein können,
und
c - eine Zahl 1 bis 8 bedeutet,
oder wobei
$R^{13}$ und $R^{12}$ zusammen mit dem Stickstoffatom einen Ring der Reihe

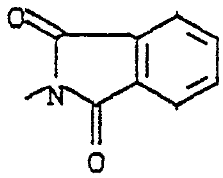 , 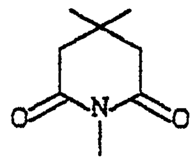 , 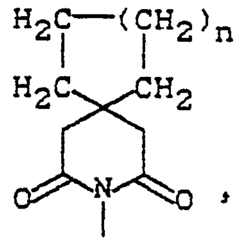 ,

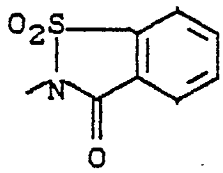 , 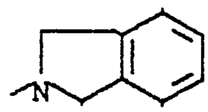 , 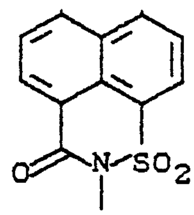 ,

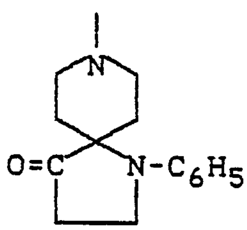 , 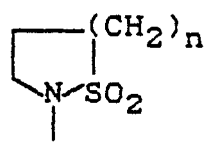 , 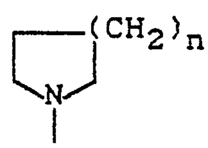

oder

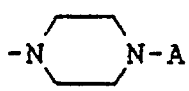

bilden,
worin
n - eine Zahl 1 oder 2 bedeutet,
und
A - für Wasserstoff oder Cycloalkyl steht,
oder
-für Alkyl steht, das durch Halogen, Hydroxy, Amino, Alkylamino, Dialkylamino, Carbamoyl oder Sulfamoyl substituiert sein kann,
oder
-für Aryl, Heteroaryl, Aralkyl, Alkoxycarbonyl, Alkylsulfonyl, Phenylsulfonyl, Tolylsulfonyl, Benzylsulfonyl, Formyl, Carbamoyl oder Sulfamoyl steht
und deren Salze gefunden,
das dadurch gekennzeichnet ist, daß man
8-Formyl-aminotetraline der allgemeinen Formel (Ie)

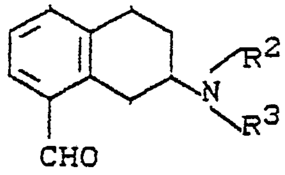

(Ie),

in welcher
$R^2$ und $R^3$ die oben angegebene Bedeutung haben,
in inerten Lösemitteln, gegebenenfalls in Anwesenheit von Kondensationsmitteln mit CH-aciden Verbindungen der allgemeinen Formel (XIII)
$H_3C\text{-}X'$ (XIII)
in welcher
X' -die für X angegebene Bedeutung hat, oder
-für Nitro steht,
umsetzt,
dann gegebenenfalls funktionelle Gruppen durch Reduktion, Hydrolyse, Oxidation oder Umsetzung mit elektrophilen Reagenzien in andere funktionelle Gruppen überführt
und dann im Fall der Herstellung der Salze mit der entsprechenden Säure umsetzt.

Das erfindungsgemäße Verfahren kann beispielsweise durch folgendes Formelschema erläutert werden:

a)

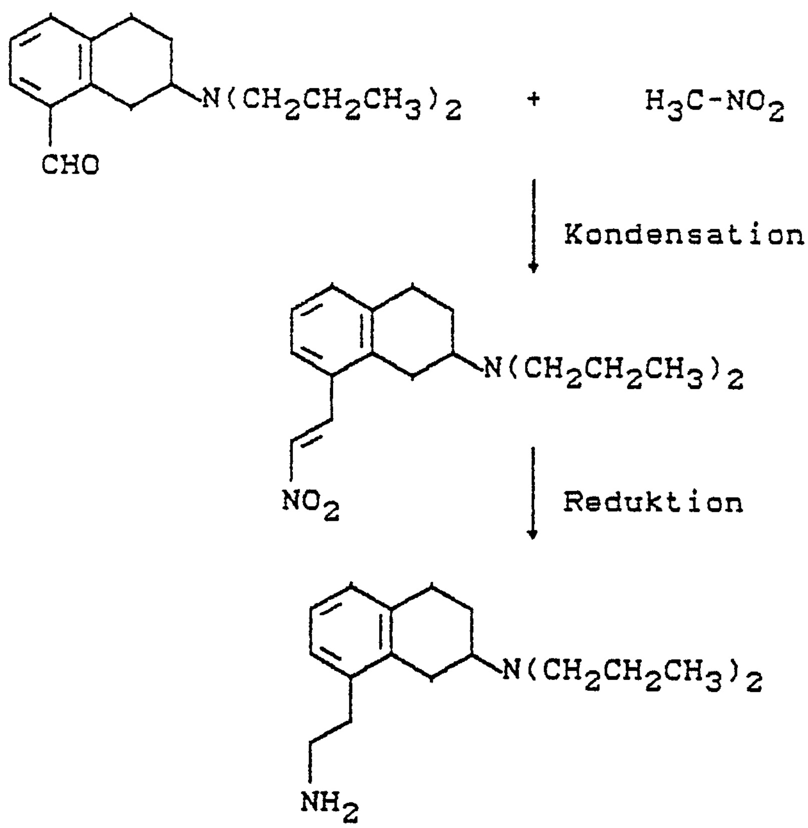


b)

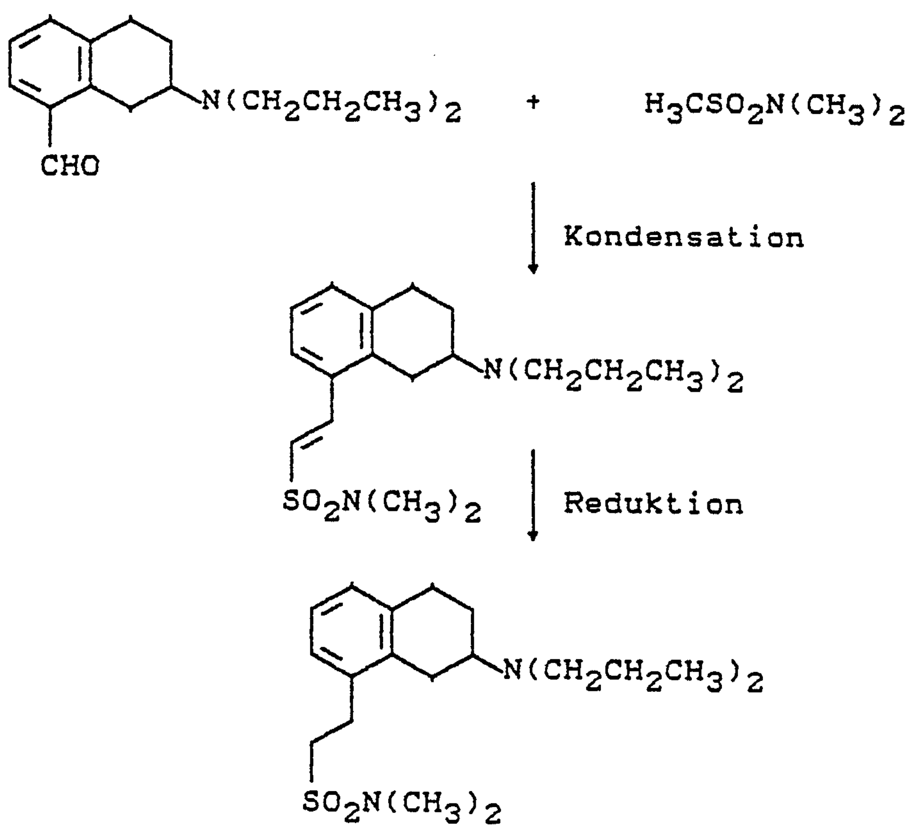


Als inerte Lösemittel eignen sich hierbei die üblichen Lösemittel, die sich unter den Reaktionsbedingungen nicht verändern. Hierzu gehören bevorzugt Wasser oder Alkohole wie Methanol, Ethanol, Propanol oder Isopropanol, oder Ether wie Diethylether, Butylmethylether, Dioxan, Tetrahydrofuran oder Glykoldimethylether, oder Halogenkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen, oder Amide wie Dimethyl-

formamid oder Hexamethylphosphorsäuretriamid, oder Dimethylsulfoxid oder Essigsäure. Ebenso ist es möglich, Gemische der genannten Lösemittel zu verwenden.

Als Kondensationsmittel werden im allgemeinen Basen verwendet. Hierzu gehören bevorzugt Alkali- oder Erdalkalihydroxide wie Natriumhydroxid, Kaliumhydroxid oder Bariumhydroxid, oder Alkalicarbonate wie Natriumcarbonat oder Kaliumcarbonat, oder Alkalialkoholate wie Natriummethanolat, Natriumethanolat, Kaliummethanolat, Kaliumethanolat, Kalium-tert.butanolat oder Ammoniak, oder Ammoniumacetat, oder organische Amine wie Diethylamin, Triethylamin, Diisopropylamin, Tripropylamin, Pyridin, Piperidin, Morpholin oder N,N-Dimethylaminopyridin oder Picolin.

Die Reaktion wird im allgemeinen in einen Temperaturbereich von 0° C bis +150° C, bevorzugt von +20° C bis +100° C durchgeführt.

Die Umsetzung kann bei normalen, erhöhtem oder erniedrigtem Druck durchgeführt werden (z.B. 0.5 bis 5 bar). Im allgemeinen arbeitet man bei Normaldruck.

Bei der Durchführung der Reaktion werden im allgemeinen die CH-acide Verbindungen in einer Menge von 0.1 bis 100, bevorzugt von 0,5 bis 50, besonders bevorzugt von 1 bis 10 Mol bezogen auf 1 Mol des 8-Formyl-aminotetralins eingesetzt.

Das erfindungsgemäße Verfahren kann beispielsweise durchgeführt werden, indem man das 8-Formylaminotetralin mit der CH-aciden Verbindung, gegebenenfalls in einem inerten Lösemittel und gegebenenfalls mit Basen mischt und gegebenenfalls erwärmt. Die Aufarbeitung erfolgt in üblicher Weise durch Extraktion, Chromatographie und/oder Kristallisation. Auch hier ist es möglich das erfindungsgemäße Verfahren nach einer phasentransferkatalysierten Variante durchzuführen.

Das Überführen von funktionellen Gruppen in andere funktionelle Gruppen in den oben aufgeführten Herstellungsverfahren erfolgt je nach Art der funktionellen Gruppe durch Oxidation, Reduktion, Hydrolyse oder durch Umsetzung mit elektrophilen Reagenzien und soll im folgenden einzeln erläutert werden:

1. Die Reduktion der Nitrilgruppe zur Aminogruppe erfolgt im allgemeinen mit Metallhydriden, bevorzugt mit Lithiumaluminiumhydrid, Aluminiumhydrid (hergestellt z.B. durch Umsetzung von Lithiumaluminumhydrid mit 100%iger Schwefelsäure oder mit Aluminiumchlorid) oder deren Gemischen in inerten Lösenmitteln wie Ethern oder Chlorkohlenwasserstoffen, bevorzugt in Ethern wie beispielsweise Tetrahydrofuran, Diethylether oder Dioxan in einem Temperaturbereich von -20° C bis +100° C, bevorzugt von 0° C bis +50° C bei Normaldruck. Die Reduktion ist außerdem durch Hydrieren der Nitrile in inerten Lösemitteln wie Alkoholen z.B. Methanol, Ethanol, Propanol oder Isopropanol in Gegenwart eines Edelmetallkatalysators wie Platin, Palladium, Palladium auf Tierkohle, oder Raney-Nickel, in einem Temperaturbereich von 0° C bis +150° C, bevorzugt von Raumtemperatur bis +100° C bei Normaldruck oder bei Überdruck möglich.

Die Reaktion kann durch folgendes Schema verdeutlicht werden:

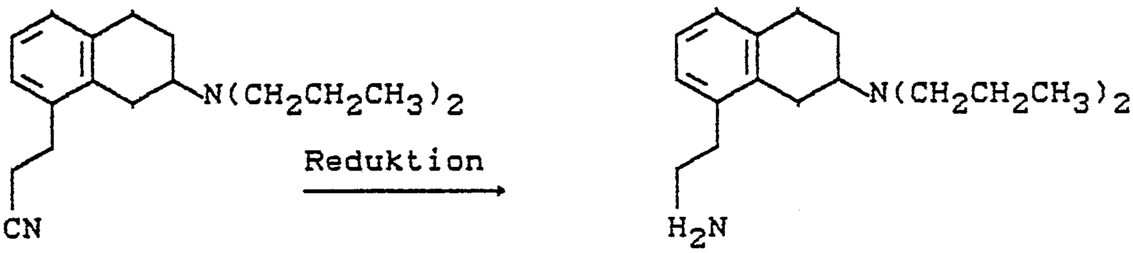


2. Die Überführung von Carbamaten in N-Methylamine erfolgt im allgemeinen durch Reduktion mit Hydriden, bevorzugt mit Lithiumaluminiumhydrid in inerten Lösemitteln wie Ethern, Kohlenwasserstoffen oder Chlorkohlenwasserstoffen, bevorzugt in Ethern wie beispielsweise Diethylether, Tetrahydrofuran oder Dioxan, in einem Temperaturbereich von 0° C bis +150° C, bevorzugt von +20° C bis +100° C bei Normaldruck.

Die Reaktion kann durch folgendes Schema verdeutlicht werden:

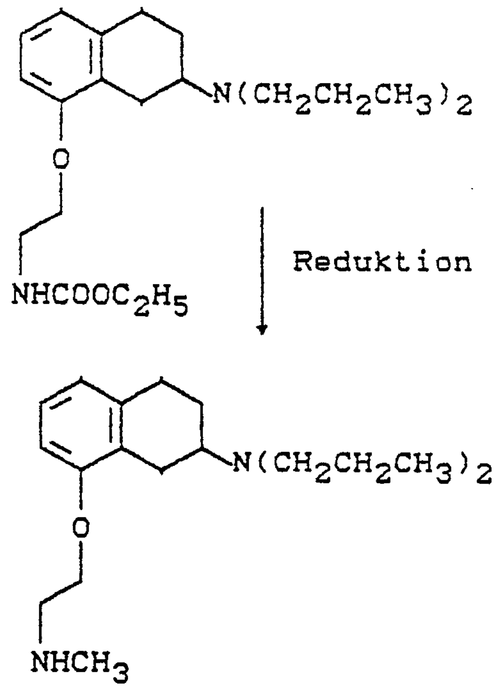


3. Die Reduktion von Alkoxycarbonylgruppen in Alkoholgruppen erfolgt im allgemeinen mit Hydriden, bevorzugt mit Lithiumaluminiumhydrid in inerten Lösemitteln wie Ethern, Kohlenwasserstoffen oder Halogenkohlenwasserstoffen oder deren Gemischen, bevorzugt in Ethern wie beispielsweise Diethylether, Tetrahydrofuran oder Dioxan in einem Temperaturbereich von 0° C bis +150° C, bevorzugt von +20° C bis +150° C bei Normaldruck.

Die Reaktion kann durch folgendes Schema verdeutlicht werden:

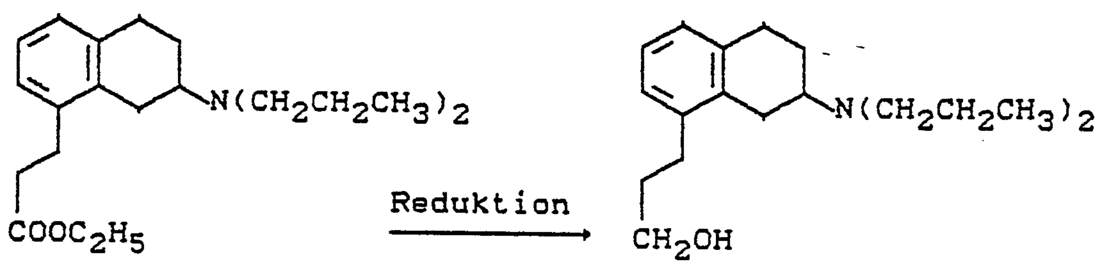


4. Die Hydrolyse der Nitrilgruppe zur Carbonamidgruppe erfolgt im allgemeinen mit Hilfe von starken Mineralsäuren, bevorzugt mit Chlorwasserstoff in inerten Lösemitteln wie Wasser und/oder Alkoholen wie beispielsweise Methanol, Ethanol, Propanol oder Isopropanol in einem Temperaturbereich von 0° C bis +150° C, bevorzugt von +20° C bis +100° C bei Normaldruck.

Die Reaktion kann durch folgendes Schema erläutert werden:

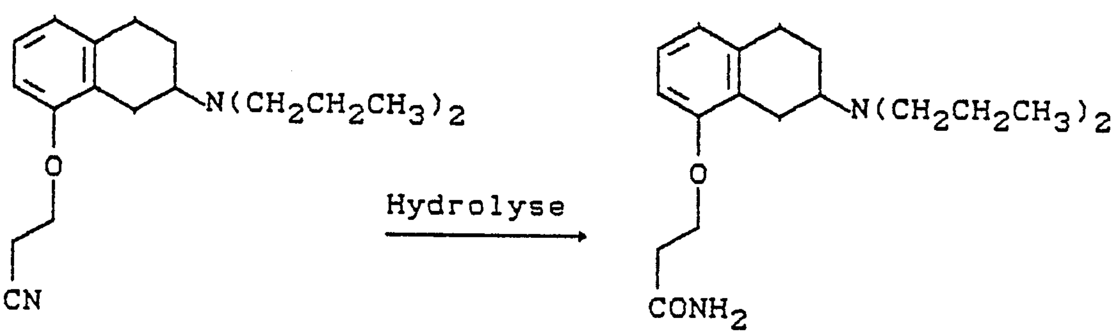


5. Durch Umsetzung von NH-oder OH-aciden Verbindungen mit elekrophilen Reagenzien erhält man eine Vielzahl weiterer erfindungsgemäßer Verbindungen:

a) Die Überführung von Aminen in Carbonamide erfolgt im allgemeinen durch Umsetzung mit Car-

bonsäureestern in inerten Lösemitteln wie Ethern oder Kohlenwasserstoffen, oder deren Gemischen, bevorzugt in Ethern wie beispielsweise Diethylether, Tetrahydrofuran oder Dioxan, gegebenenfalls in Anwesenheit von Basen wie Alkalimetalle, Alkalihydride, Alkalialkoholate oder lithiumorganischen Verbindungen, bevorzugt in Anwesenheit von Alkalimetallen wie beispielsweise Natrium, oder Alkalihydriden wie Natriumhydrid oder Kaliumhydrid in einem Temperaturbereich von +20° C bis +150° C, bevorzugt bei der Siedetemperatur des verwendeten Lösemittels bei Normaldruck.

Darüberhinaus ist es möglich, die Amide mit Carbonsäurehalogeniden oder -anhydriden, bevorzugt mit Carbonsäurechloriden in inerten Lösemitteln wie Ethern, Kohlenwasserstoffen oder Halogenkohlenwasserstoffen oder deren Gemische, bevorzugt in Ethern wie beispielsweise Diethylether, Tetrahydrofuran, oder Halogenkohlenwasserstoffen wie Methylenchlorid oder Chloroform, gegebenenfalls in Anwesenheit von Basen wie Alkalicarbonate beispielsweise Natriumcarbonat oder Kaliumcarbonat, oder organischen Aminen wie beispielsweise Triethylamin oder Pyridin, in einem Temperaturbereich von -20° C bis +100° C, bevorzugt von 0° C bis +60° C bei Normaldruck herzustellen.

Die Reaktion kann durch folgendes Schema verdeutlicht werden:

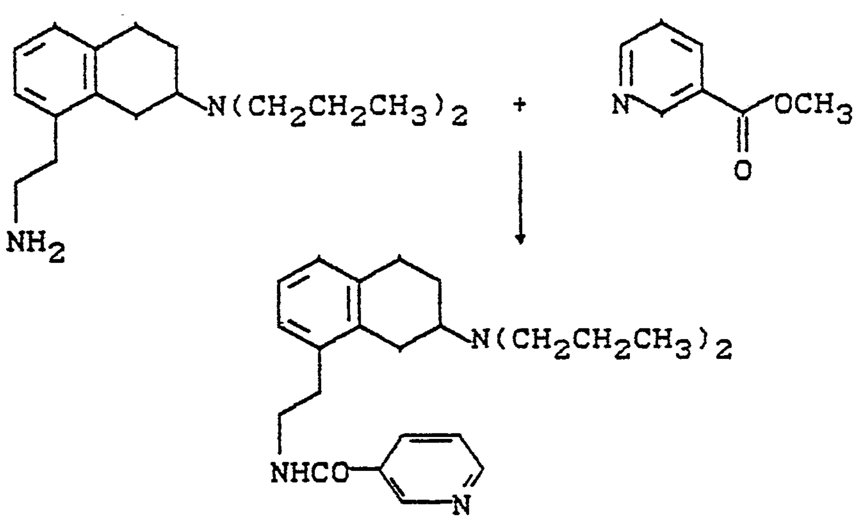

b) Die Überführung von Aminen in Carbamate erfolgt im allgemeinen mit Kohlensäureestern, bevorzugt mit unsymmetrischen Kohlensäureestern, besonders bevorzugt mit Kohlensäureestern, die einen Phenylesterrest tragen, in inerten Lösemitteln wie Ethern, Kohlenwasserstoffen oder Halogenkohlenwasserstoffen, oder deren Gemischen, bevorzugt in Ethern wie beispielsweise Diethylether, Tetrahydrofuran oder Dioxan, in einem Temperaturbereich von +20° C bis +150° C, bevorzugt von +20° C bis +100° C bei Normaldruck.

Die Reaktion kann durch folgendes Schema erläutert werden:

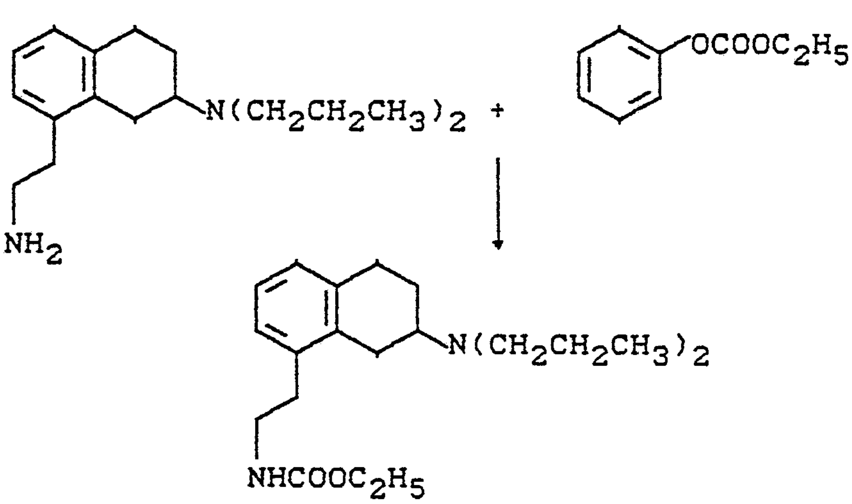

c) Die Überführung von Aminen in Harnstoffe erfolgt im allgemeinen durch Umsetzung mit Isocyanaten in inerten Lösemitteln wie Ethern, Kohlenwasserstoffen oder Halogenkohlenwasserstoffen, oder deren Gemischen, bevorzugt in Ethern wie beispielsweise Diethylether oder Tetrahydrofuran, oder in Halogenkohlenwasserstoffen wie beispielsweise Methylen chlorid oder Chloroform, in einem Temperaturbereich von -20° C bis +150° C, bevorzugt von 0° C + 100° C bei Normaldruck.

Die Reaktion kann durch folgendes Schema erläutert werden:

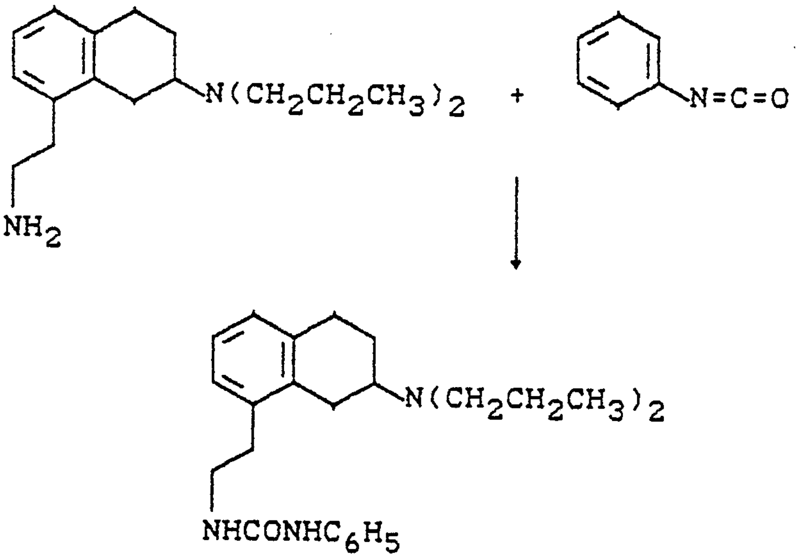

d) Die Überführung von Amiden in Sulfonamide bzw. Aminosulfamoylderivate erfolgt im allgemeinen mit Sulfonsäurehalogeniden bzw. mit Amidosulfonsäurehalogeniden, bevorzugt mit den entsprechenden Chloriden in inerten Lösemitteln wie Ethern, Kohlenwasserstoffen oder Halogenkohlenwasserstoffen, oder deren Gemischen, bevorzugt in Halogenkohlenwasserstoffen wie beispielsweise Methylenchlorid oder Chloroform, gegebenenfalls in Anwesenheit von Basen wie Alkalihydroxide, Alkalicarbonate, Alkalialkoholate oder organische Amine, bevorzugt mit Alkalihydroxiden wie Natriumhydroxid oder Kaliumhydroxid, Alkalicarbonaten wie beispielsweise Natriumcarbonat oder Kaliumcarbonat, oder organischen Aminen wie Triethyl amin oder Pyridin, in einem Temperaturbereich von -20° C bis +100° C, bevorzugt von 0° C bis +50° C bei Normaldruck.

Die Reaktion kann durch folgendes Schema verdeutlicht werden:

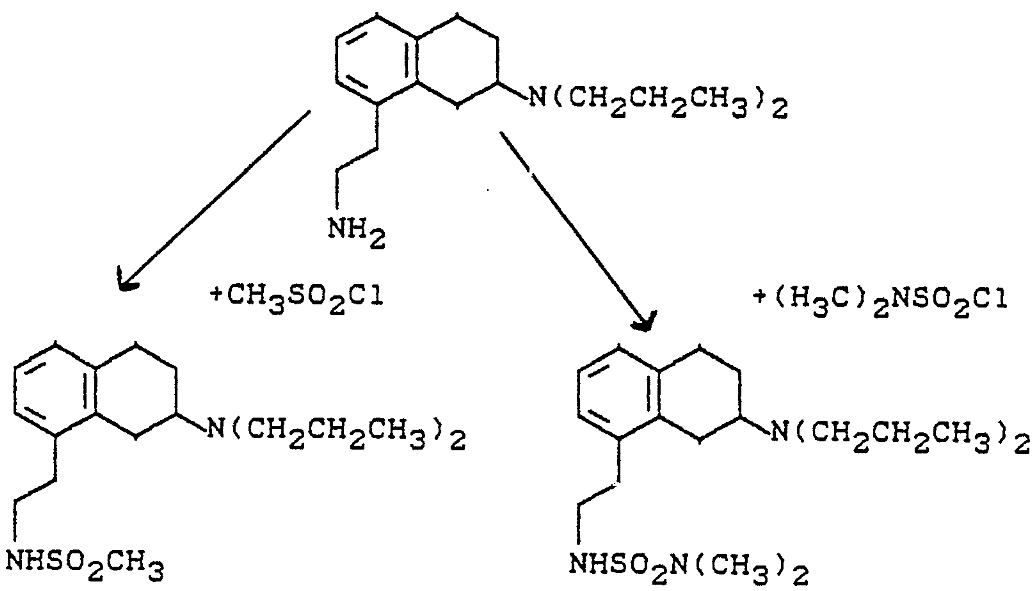


e) Die Überführung der Hydroxygruppe in Kohlensäureester erfolgt im allgemeinen durch Umsetzen mit Halogenameisensäureestern, bevorzugt mit Chlorameisensäureestern in inerten Lösemitteln wie Ethern, Kohlenwasserstoffen oder Halogenkohlenwasserstoffen, bevorzugt in Halogenkohlenwasserstoffen wie Methylenchlorid oder Chloroform, oder in Ethern wie Diethylether oder Tetrahydrofuran, gegebenenfalls in Anwesenheit von Basen wie Alkalihydroxiden, Alkalicarbonaten oder organischen Aminen, bevorzugt in Anwesenheit von organischen Dimethylaminopyridin in einem Temperaturbereich von -20° C bis +100° C,

bevorzugt von 0° C bis +30° C bei Normaldruck.

Die Reaktion läßt sich durch folgendes Schema verdeutlichen:

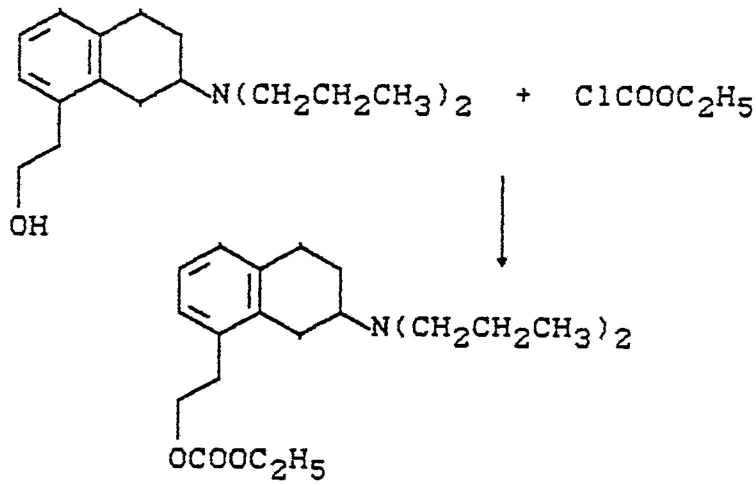

f) Cyclische Sulfonamide werden im allgemeinen durch Reaktion intramolekularer Elektrophile in inerten dipolar aprotischen Lösemitteln, bevorzugt in Dimethylformamid, Hexamethylphosphorsäuretriamid oder Dimethylsulfoxid, gegebenenfalls in Anwesenheit von Basen wie Alkalimetallen, Alkalihydriden, Alkaliamiden, Alkalialkoholaten oder lithiumorganischen Verbindungen, bevorzugt in Anwesenheit von Alkalihydriden wie Natriumhydrid oder Kaliumhydrid, oder Alkaliamiden wie Natriumamid oder Lithiumdiisopropylamid, gegebenenfalls in Gegenwart katalytischer Mengen eines Alkaliiodides, beispielsweise Natriumiodid einer Kaliumiodid, in einem Temperaturbereich von -20° C bis +100° C, bevorzugt von 0° C bis +50° C bei Normaldruck hergestellt.

Die Reaktion läßt sich durch folgendes Schema verdeutlichen:

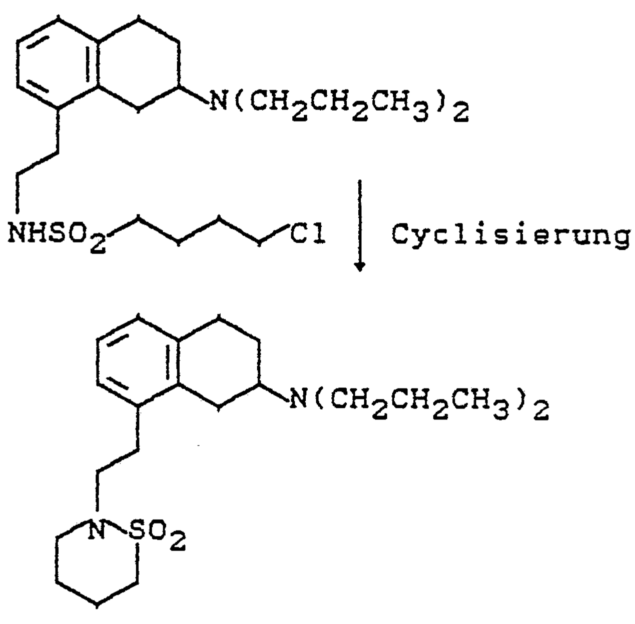


6. Eine weitere Variation des erfindungsgemäßen Verfahrens bezüglich der funktionellen Gruppen in R¹ ergibt sich durch die Reduktion der Doppelbindung in R¹ , wobei anwesende Nitrogruppen ($X' = NO_2$) gleichzeitig zur Aminogruppe reduziert werden. Die Reduktion erfolgt im allgemeinen mit Metallhydriden, bevorzugt mit Lithiumaluminiumhydrid, Aluminiumhydrid (hergestellt z.B. durch Umsetzung von Lithiumaluminiumhydrid mit Schwefelsäure und Aluminiumchlorid, Natriumborhydrid, Lithiumborhydrid oder deren Gemischen in inerten Lösemitteln wie Ether oder Chlorkohlenwasserstoffen, bevorzugt in Ethern wie beispielsweise Tetrahydrofuran, Dioxan oder Diethylether, in einem Temperaturbreich von -20° C bis +100° C, bevorzugt von 0° C bis +80° C bei Normaldruck.

Ebenso ist es möglich diese Reduktion durch Hydrieren in inerten Lösemitteln wie Alkoholen z.B.

Methanol, Ethanol oder Isopropanol, in Anwesenheit von Katalysatoren wie Platin, Platinoxid, Palladium, Palladium auf Tierkohle oder Raney-Nickel, gegebenenfalls in Anwesenheit von Säuren wie Salzsäure, Essigsäure, Trichlor-oder Trifluoressigsäure, in einem Temperaturbereich von 0° C bis +200° C, bevorzugt von +20° C bis +100° C bei Normal-oder bei Überdruck durchzuführen.

Die Reaktion kann durch folgendes Schema verdeutlicht werden:

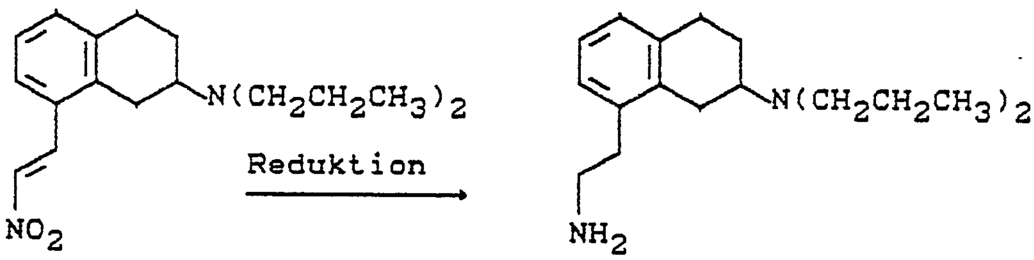


Man kann Verbindungen der Formel (I) mit R=O $(CH_2)_aX$ (in welcher a und X die spezifische Bedeutung haben) aus den entsprechenden 8-Hydroxy-2-alkylamino-tetralinen erhalten. Die 8-Hydroxy-2-alkylamino-tetraline sind bekannt (EP-A-1-41 488) oder können aus den entsprechenden Halogenverbindungen der Formel (I) hergestellt werden.

Die 8-Hydroxy-2-alkylamino-tetraline können hergestellt werden, indem man sie unter an sich bekannten Alkylierungsbedingungen mit Verbindungen der Formel

$Y-(CH_2)_a-X$

in welcher

Y für -Cl, -Br oder $-OSO_2$-Alkyl ($C_1$ bis $C_4$) steht und X und a die obengenannte Bedeutung haben, umsetzt.

Die erfindungsgemäßen Stoffe der allgemeinen Formel (I) haben eine hohe Affinität zu cerebralen 5-Hydroxy-tryptamin-Rezeptoren vom 5-$HT_1$-Typ. Hiermit verbunden sind agonistisch, partiell agonistische oder antagonistische Wirkungen am Serotonin-Rezeptor, wogegen die bekannten Stoffe der EP-A1-41 488 rein agonistische Eigenschaften besitzen.

Die in der vorliegenden Erfindung beschriebenen hochaffinen Liganden für den Serotonin-1-Rezeptor stellen somit bessere Wirkstoffe zur Bekämpfung von Krankheiten dar, die durch Störungen des serotoninergen Systems, insbesondere bei Involvierung von Rezeptoren, die hohe Affinität zu 5-Hydroxytryptamin besitzen (5-$HT_1$-Typ), gekennzeichnet sind. Sie eignen sich zur Behandlung von Erkrankungen des zentralen Nervensystems wie Angst-, Spannungs-und Depressionszuständen, zentralnervös bedingten Sexualdysfunktionen und Schlafstörungen. Weiterhin sind sie geeignet zur Beseitigung kognitiver Defizite, zur Verbesserung von Lern-und Gedächtnisleistungen und zur Behandlung der Alzheimer'schen Krankheit. Weiterhin eignen sich diese Wirkstoffe auch zur Modulierung des cardiovaskulären Systems. Sie greifen auch in die Regulation der cerebralen Durchblutung ein und stellen somit wirkungsvolle Mittel zur Bekämpfung von Migräne dar. Ebenso können die erfindungsgemäßen Verbindungen zur Bekämpfung von Schmerzzuständen eingesetzt werden. Auch eignen sie sich zur Bekämpfung von Erkrankungen des Intestinaltraktes, die durch Störungen des serotoninergen Systems gekennzeichnet sind.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.% der Gesamtmischung vorhanden sind, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. in Fall der Benutzung von Wasser als Verdünnungsmittel gegebenenfalls organische Lösungsmittel wie Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.B.: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch-und Traubenzucker), Emulgiermittel (z.B. Polyoxyethylen-Fettsäure-Ester), PolyoxyethylenFettalkohol-Ether (z.B. Lignin-Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

Beispiel 1

2-Dipropylamino-8-chloro-1,2,3,4-tetrahydronaphthalin

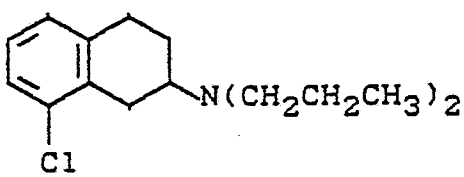

700 mg (2,8 mmol) 2-Dipropylamino-8-amino-1,2,3,4-tetrahydronaphtahlin wurden in 2,5 ml Wasser und 1,14 ml (13,7 mmol) 37%iger Salzsäure gelöst. Dazu tropfte man bei einer Innentemperatur von 0°C eine Lösung aus 195 mg Natriumnitrit und 0,45 ml Wasser. Anschließend wurde die Lösung nach 15 min bei 0°C gerührt.

Inzwischen wurden 396 mg (4 mmol) Kupfer(I)chlorid in 1,6 ml Wassr gelöst, und die Lösung auf 0°C abgekühlt. Dazu tropfte man vorsichtig die obere Reaktionslösung zu und erwärmte den Ansatz 30 min auf +95°C bis die Stickstoffentwicklung beendet war.

Die Reaktionsmischung wurde mit wässriger Kaliumhydrogencarbonatlösung neutralisiert und mit Dichlormethan extrahiert. Die organische Phase wurde über Natriumsulfat getrocknet und das Lösemittel abgetrennt. Der Rückstand wurde durch eine Säulenchromatographie auf Kieselgel gereinigt. Als Fließmittel verwendete man Dichlormethan/Methanol 20:2.

Ausbeute: 74% der Theorie (öliges Produkt)

$R_f$ : 0,64

Die Base ließ sich aus einer Petroleumbenzin-Lösung mit etherischer Chlorwasserstofflösung als Hydrochlorid ausfällen. Das Hydrochlorid war ein stark hygroskopisches, farbloses, kristallines Produkt.

Beispiel 2

Verfahrensvariante A

2-Dipropylamino-8-bromo-1.2.3,4-tetrahydronaphthalin

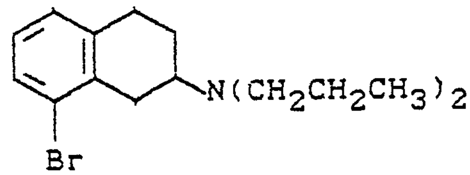

Die Reaktion ließ sich durch Einsetzen von Kupfer(I)bromid und 47%iger Bromwasserstoffsäure analog der Reaktion für 2-Dipropylamino-8-chloro-1.2.3,4-tetranydronaphthalin (Beispiel 1) durchführen.
Ausbeute: 32% der Theorie (öliges Produkt)
$R_f$ : 0,73 (Dichlormethan/Methanol 20:2)
Die Base ließ sich aus einer Petroleumbenzin-Lösung mit etherischer Chlorwasserstofflösung als Hydrochlorid ausfällen. Das Hydrochlorid war ein stark hygroskopisches, farbloses, kristallines Produkt.

Verfahrensvariante B :

1. 8-Brom-2-tetralon

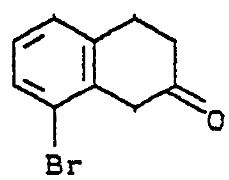

83,5 g Aluminiumchlorid (0,63 mmol) wurden in 1,8 l Methylenchlorid bei 0 - +5° C eingetragen, 105 g (0,49 Mol) 2-Bromphenyl-essigsäurechlorid (gelöst in 200 ml Methylenchlorid) wurden bei 0 - +5° C in 30 min zugetropft. Nach 30 min Nachrühren bei 0 - +5° C wurden 50 g Ethylen so eingeleitet, daß die Reaktionstemperatur +10° C nicht überschritt. Es wurde noch 2 h bei 20 - +25° C nachgerührt. Der Aluminiumkomplex wurde durch vorsichtige Zugabe von 500 ml Eiswasser zerstört, die Phasen wurden getrennt und die wäßrige Phase mit 250 ml Methylenchlorid extrahiert. Aus der getrockneten Methylenchloridphase wurde die Titelverbindung in einer Ausbeute von 109 g als gelbes Öl (GC-Gehalt 97%) erhalten und ohne weitere Reinigung eingesetzt.

2. 8-Brom-2-dipropylamino-1,2,3,4-tetrahydronaphthalin

a) Enaminsynthese: 385 g (1,71 Mol) 8-Brom-2-tetralon und 165 g Propylamin (2,8 Mol) wurden in 700 ml Benzol in Gegenwart von 1,5 g Ionenaustauscherharz Amberlyst 15 am Wasser-Abscheider 2 h unter Rückfluß gerührt. Das Ionenaustauscherharz wurde abfiltriert und das Filtrat unter reduziertem Druck eingeengt. Der verbleibende Rückstand (437 g) wurde als Rohprodukt in die reduktive Alkylierung eingesetzt.

b) Reduktive Alkylierung: 437 g rohes Enamin (a) wurden bei 0 - +5° C in 3 l Propionsäure vorgelegt. Bei 0 - +10° C wurden 310 g Natriumborhydrid portionsweise zugegeben. Nach beendeter Zugabe wurde die Reaktionstemperatur langsam auf +50° C gesteigert und 2 h gehalten. Anschließend wurde die Reaktionstemperatur auf +120° C erhöht und 3 h gehalten. Die abgekühlte Reaktionslösung wurde mit 10 l Eiswasser verdünnt und durch Zugabe von Natronlauge (45%) schwach alkalisch gestellt. Durch Extraktion mit Chloroform und fraktionierte Destillation wurden 320 g des Endproduktes (Kp 128 -135° C; 0,07 mm) erhalten (GC 90%ig).

Wird die Reaktion nach zweistündigem Nachrühren bei +50°C abgebrochen und wie beschrieben aufgearbeitet, so wird das sekundäre Amin 8-Brom-2-propylaminotetrahydronaphthalin erhalten.

Beispiel 3

Verfahrensvariante A

2-Dipropylamino-8-cyano-1.2.3,4-tetrahydronaphthalin

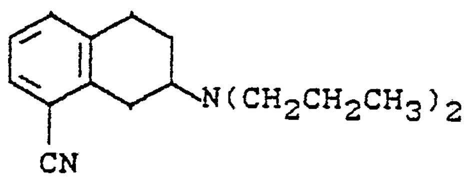

Unter Stickstoff wurden 6,0 g (24 mmol) 2-Dipropylamino-8-amino-1.2.3,4-tetrahydronaphthalin in 36 g (120 mmol) 33%iger Schwefelsäure gelöst. Danach wurde bei 0° C bis max. +5° C eine Lösung von 1.7 g (24 mmol) Natriumnitrit in 5 ml Wasser zugetropft. Anschließend wurde die Reaktionslösung noch 30 min bei der gleichen Temperatur gerührt und dann in eine auf +50° C erwärmte Lösung aus 2,9 g (32 mmol) Kupfer(I)cyanid, 8,7 g (177 mmol) Natriumcyanid und 39 ml Wasser getropft. Der Ansatz wurde danach noch 1 h bei 50° C gerührt, bis die Stickstoffentwicklung beendet war.

Anschließend wurde der Ansatz in 500 ml Eiswasser verrührt und die Mischung mit 2 N Natronlauge auf pH 10 gestellt. Danach wurde die Mischung mit Dichlormethan extrahiert und die organische Phase mit Wasser neutral gewaschen. Die Lösung wurde über Natriumsulfat getrocknet und das Lösemittel abdestilliert. Der Rückstand wurde durch eine Säulenchromatographie auf Kieselgel gereinigt. Als Fließmittel wurde Toluol/Methanol 85:15 verwendet.
Ausbeute: 48% der Theorie (Öl)
$R_f$ : 0,63 (Toluol/Methanol 83:17)

Verfahrensvariante B:

152 g 8-Brom-2-dipropylamino-1,2,3,4-tetrahydronaphthalin (Beispiel 2) (0,49 Mol) und 66 g Kupfer(I)-cyanid wurden in 450 ml Dimethylformamid 6 h bei 150° C gerührt. Der abgekühlte Reaktionsansatz wurde in eine Mischung aus 150 ml Ethylendiamin und 450 ml $H_2O$ gegeben und 1 h bei Raumtemperatur gerührt. Das durch Extraktion mit Essigester erhaltene Rohprodukt wurde fraktionell destilliert. Das Produkt wurde als farbloses Öl (Kp. 140 - 150° C; 0,1 mm) in einer Ausbeute von 73 g erhalten (GC 95%).

Beispiel 4

2-Dipropylamino-8-(3-butyl-ureido)-1,2,3,4-tetrahydronaphthalin

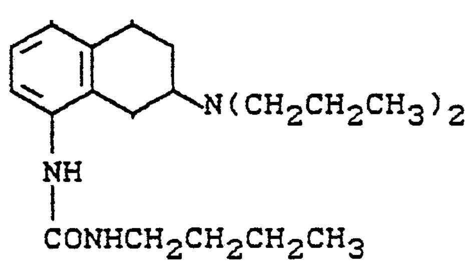

0,7 g (2,8 mmol) 2-Dipropylamino-8-amino-1,2,3,4-tetrahydronaphthalin wurden in 3 ml Toluol gelöst. Die Lösung wurde mit 0,34 g (3,4 mmol) Butylisocyanat versetzt und 17 h bei Raumtemperatur gereinigt. Anschließend wurde das Lösemittel unter Vakuum abdestilliert und der Rückstand durch eine Säulenchromatographie auf Kieselgel gereinigt. Als Fließmittel verwendete man Essigester/Methanol/Triethylamin 20:1:0,1.
Ausbeute: 51% der Theorie (ölige Substanz)

$R_f$ : 0,51

Nach Lösen in Petroleumbenzin ließ sich die Base durch Zutropfen von etherischer Chlorwasserstofflösung ins Hydrochlorid überführen. Das Hydrochlorid war eine farblose, leicht hygroskopische, kristalline Substanz.

Beispiel 5

2-Dipropylamino-8-formamido-1,2,3,4-tetrahydronaphthalin

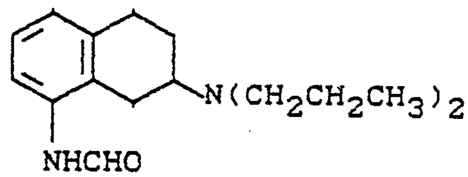

1,06 g (10,4 mmol) Acetanhydrid und 0,48 g (10,4 mmol) Ameisensäure wurden vereinigt und 3,5 h auf 50°C erwärmt. Nach Abkühlung tropfte man die Lösung aus 0,85 g (3,5 mmol) 2-Dipropylamino-8-amino-1,2,3,4-tetrahydronaphthalin und 5 ml Ameisensäure zu und rührte den Ansatz 17 h bei Raumtemperatur. Anschließend wurde das Lösemittel im Vakuum abdestilliert und der Rückstand in Dichlormethan gelöst. Die Lösung wurde dann mit wässriger Kaliumhydrogencarbonatlösung gewaschen und über Natriumsulfat getrocknet. Anschließend wurde das Lösemittel abdestilliert.
Der Rückstand wurde durch eine Säulenchromatographie auf Aluminiumoxid 90 (Aluminiumoxid der Fa. Merck, Aktivitätsstufe II-III) gereinigt. Als Fließmittel verwendete man Cyclohexan/Methanol/Triethylamin 20:0,5:0,1.
Ausbeute: 42% der Theorie (kristalline Substanz)
$R_f$ : 0,62

Die Base ließ sich, nach Lösen in Diethylether, durch Zutropfen einer etherischen Chlorwasserstofflösung ins Hydrochlorid überführen. Das Hydrochlorid war eine farblose, stark hygroskopische, kristalline Substanz.

Beispiel 6

2-Dipropylamino-8-carbamoyl-1,2,3,4-tetrahydronaphthalin

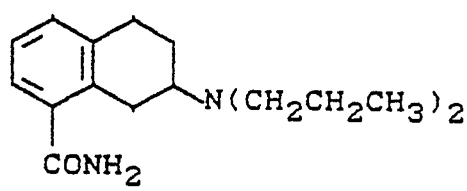

Unter Stickstoff wurden 1,0 g (4 mmol) 2-Dipropylamino-8-cyano-1,2,3,4-tetrahydronaphthalin und 0,5 g (8 mmol) Kaliumhydroxid in 10 ml tert.Butanol 6 h unter Rückfluß gekocht. Nach Abkühlung wurde die Reaktionslösung mit 20 ml gesättigter Natriumchloridlösung verdünnt und mit Dichlormethan extrahiert. Die organische Phase wurde mit Natriumsulfat getrocknet und das Lösemittel im Vakuum abdestilliert. Der Rückstand wurde aus 10 ml Diisopropylether kristallisiert.
Ausbeute: 56% der Theorie (beiges Pulver)
Fp. : 105 - 106° C
$R_f$ : 0,25 (Kieselgel; Dichlormethan/Methanol 75:25)

Beispiel 7

2-Dipropylamino-1,2,3,4-tetrahydronaphthalin-8-carbonsäure

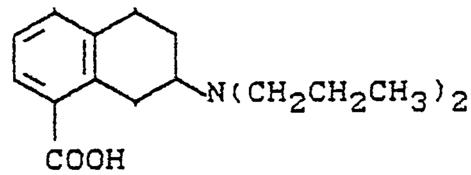

Unter Stickstoff wurden 256 mg (1 mmol) 2-Dipropylamino-8-cyano-1,2,3,4-tetrahydronaphthalin, 132 mg (2 mmol) Kaliumhydroxid und 36 mg (2 mmol) Wasser in 1 ml Ethylenglykol 20 h bei +150° C gerührt. Nach Abkühlung wurde die Reaktionslösung mit 6 ml Wasser verdünnt und mit Dichlormethan extrahiert. Die wässrige Phase wurde dann mit 2,75 ml 1N Salzsäure auf pH 1-2 eingestellt und wieder mit Dichlormethan extrahiert. Anschließend wurde die wässrige Phase mit 25%iger Ammoniaklösung auf pH 5 eingestellt. Danach wurde das Wasser im Vakuum abdestilliert und der Rückstand bei +50° C und 0,05 mb Vakuum bis zur Gewichtskonstanz getrocknet. Dann wurde der Rückstand in absolutem Tetrahydrofuran gelöst. Nachdem die unlöslichen Salze abfiltriert wurden, wurde die Lösung unter Vakuum vom Lösemittel befreit. Als Rückstand verblieb das Reaktionsprodukt als fast farbloses Öl.
Ausbeute: 44% der Theorie
$R_f$ : 0,67 (Kieselgel; Dichlormethan/Methanol/25%ige Ammoniaklösung 67:27:6)

Beispiel 8

2-Dipropylamino-8-ethoxycarbonyl-1,2,3,4-tetrahydronaphthalin

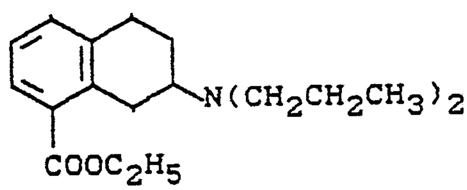

300 mg (1,1 mmol) 2-Dipropylamino-1,2,3,4-tetrahydronaphthalin-8-carbonsäure wurden in 30 ml absolutem Ethanol und 3 ml einer Lösung aus Chlorwasserstoff in Diethylether (190 mg/ml) bei Raumtemperatur gerührt, bis die Reaktion vollständig war. Anschließend wurde das Lösemittel unter Vakuum abdestilliert und der Rückstand in 10 ml Dichlormethan gelöst. Die Lösung wurde 1 mal mit 0,1 N Natronlauge und 2 mal mit Wasser gewaschen und über unter Vakuum abdestilliert. Das Reaktionsprodukt verblieb als fast farbloses Öl.
Ausbeute: 64% der Theorie
$R_f$ : 0,97 (Kieselgel; Dichlormethan/Methanol/25%ige Ammoniaklösung 67:27:6)

Beispiel 9

2-Dipropylamino-8-formyl-1,2,3,4-tetrahydronaphthalin

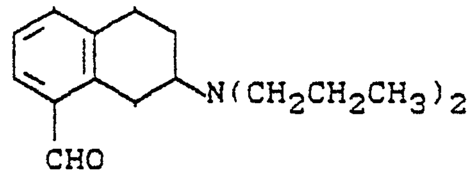

3,75 g (154,4 mmol) Magnesiumpulver wurden unter Argon in 100 ml trockenem Tetrahydrofuran vorgelegt. Man gab einige Tropfen Iodethan zu, und tropfte dann bei 0° C eine Lösung aus 31,9 g (103 mmol) 2-Dipropylamino-8-brom-1,2,3,4-tetrahydronaphthalin und 20 ml absolut trockenem Tetrahydrofuran zu. Es wurde 3 h bei +60° C nachgerührt. Dann kühlte man auf Raumtemperatur ab und tropfte bei dieser Temperatur 12,8 g (113,3 mmol) N-Formylpiperidin zu. Es wurde 30 min bei Raumtemperatur nachgerührt und dann mit 3 N Salzsäure hydrolysiert. Anschließend stellte man auf pH 12 ein und extrahierte mit Essigester. Man chromatographierte mit Cyclohexan/Essigester/Triethylamin: 80:19:1 über Kieselgel 60, 63 - 200 µm.
Ausbeute: 65,5% der Theorie (Öl)
$R_f$ : 0,342 (Diisopropylether / Triethylamin 99:1)

Beispiel 10

2-Dipropylamino-8-hydroxymethyl-1,2,3,4-tetrahydronaphthalin

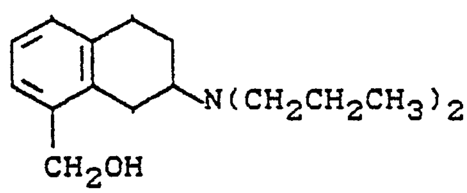

Unter Stickstoff wurden 15 mg (0,4 mmol) Lithiumaluminiumhydrid in 2 ml Diethylether suspendiert. Zur Suspension wurden vorsichtig eine Lösung aus 210 mg (0,7 mmol) 2-Dipropylamino-8-ethoxycarbonyl-1,2,3,4-tetrahydronaphthalin in 1 ml Diethylether getropft. Anschließend wurde der Ansatz noch 5 h bei Raumtemperatur gerührt.

Danach wurden erst 5 mg (0,05 mmol) Essigsäureethylester, dann 15 mg Wasser, anschließend 20 mg 15%ige Natronlauge und zum Schluß wieder 50 mg Wasser zugetropft und jeweils 0,5 h gerührt. Dann wurde der Ansatz mit 3 ml Diethylether verdünnt. Der Niederschlag wurde abfiltriert und das Filtrat mit Wasser gewaschen und vom Lösemittel befreit. Der ölige Rückstand wurde im Vakuum von 0,05 mb und einer Temperatur von 50° C bis zu Gewichtskonstanz getrocknet.
Ausbeute: 71% der Theorie
$R_f$ : 0,28 (Kieselgel; Toluol/Methanol 85:15)

Beispiel 11

8-Acetylamino-2-dipropylamino-1.2.3.4-tetrahydronaphthalin

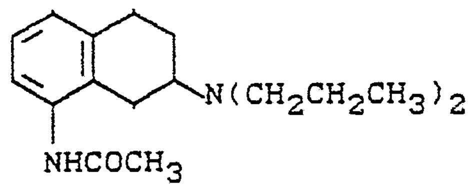

24 g (0.094 Mol) 8-Cyano-2-dipropylamino-1.2.3.4-tetrahydronaphthalin wurden in 100 ml Tetrahydrofuran (absolut) vorgelegt; 4.5 g Lithiumaluminiumhydrid (0,12 Mol) wurden bei +20 - +30° C portionsweise zugegeben. Nach zweistündigem Rühren unter Rückfluß wurde das überschüssige Lithiumaluminiumhydrid durch Zugabe von Wasser und Natriumhydroxid zerstört. Der ausgefallene anorganische Feststoff wurde abgesaugt und mit 50 ml Tetrahydrofuran gewaschen. Das nach Trocknen und Einengen des Filtrats verbleibende Rohprodukt (25 g) wurde in 200 ml Methylenchlorid gelöst und mit 10 g Triethylamin versetzt. Nach Zugabe von 0,2 g Dimethylaminopyridin als Acylierungskatalysator wurden 1 g Acetanhydrid bei +20 - +25° C zugetropft. Nach zwölfstündigem Nachrühren bei +20 - +25° C wurde die Reaktionslösung mit Natriumhydrogencarbonat und Wasser gewaschen, getrocknet und eingeengt. Das erhaltene Rohprodukt (30 g) wurde aus Hexan/Essigester (10:1) umkristallisiert. Das Produkt wurde als Feststoff in einer Ausbeute von 75% der Theorie isoliert.
Fp. : 99 - 100° C

Beispiel 12

2-Dipropylamino-8-aminomethyl-1,2,3,4-tetrahydronaphthalin

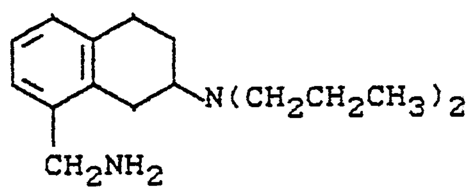

Unter Stickstoff wurden 10.6 g (35 mmol) 2-Dipropylamino-8-acetamidomethyl-1,2,3,4-tetrahydronaphthalin in 100 ml Ethanol und 23,1 g (350 mmol) Kaliumhydroxid bei 90° C 24 h zum leichtem Rückfluß erhitzt. Anschließend wurde der Ansatz in 1 l Eiswasser eingerührt und die Mischung mit Dichlormethan extrahiert. Die organische Phase wurde mit Wasser gewaschen und über Natriumsulfat getrocknet. Das Lösemittel wurde im Vakuum abdestilliert. Das als Rückstand verbliebene Reaktionsprodukt wurde durch eine Säulenchromatographie auf Kieselgel mit Methanol/Triethylamin 95:5 als Fließmittel gereinigt.
Ausbeute: 59% der Theorie (Öl)
$R_f$ : 0,62

Beispiel 13

2-Dipropylamino-8-aminomethyl-1,2,3,4-tetrahydronaphthalin Hydrochlorid

Die Verbindung des Beispiels 12 wurde in Ether gelöst. Durch Zutropfen von etherischer Chlorwasserstofflösung wurde das Hydrochlorid ausgefällt, welches dann abgesaugt und bei Raumtemperatur im Vakuum getrocknet wurde.
Ausbeute: 3,6 g
Fp. : 97 - 100°C

Beispiel 14

2-Dipropylamino-8-methylsulfonamido-methyl-1,2,3,4-tetrahydronaphthalin

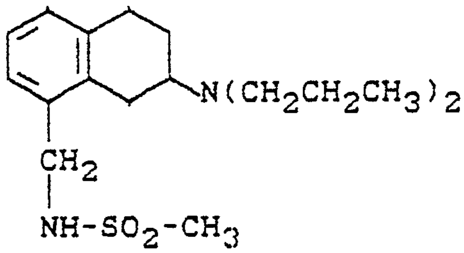

Unter Stickstoff wurden 1,6 g (6 mmol) 2-Dipropylamino-8-aminomethyl-1,2,3,4-tetrahydronaphthalin in 16 ml Dichlormethan gelöst. Bei max. +25° C wurden dann 0,7 g (6 mmol) Methansulfonsäurechlorid zugetropft. Anschließend ließ man die Reaktionlösung 18 h bei Raumtemperatur rühren. Danach wurden 16 ml Wasser zugesetzt. Die Mischung wurde mit 1 N Natronlauge auf pH 10 gestellt. Die organische Phase wurde abgetrennt und mit Wasser gewaschen. Nach Trocknung über Natriumsulfat wurde das Lösemittel im Vakuum abdestilliert. Der Rückstand wurde durch Säulenchromatographie auf Kieselgel mit Diisopropylether/Isopropanol/Triethylamin 65:70:5 als Fließmittel gereinigt.
Ausbeute: 76% der Theorie (Öl)
$R_f$ : 0,55

Beispiel 15

2-Dipropylamino-8-butylsulfonamidomethyl-1,2,3,4-tetrahydronaphthalin

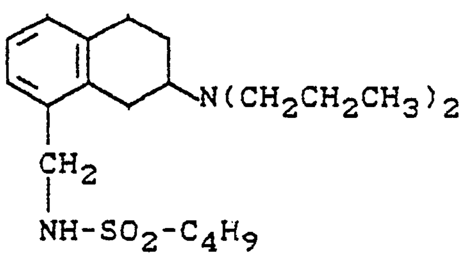

Unter Stickstoff wurden 1,6 g (6 mmol) 2-Dipropylamino-8-aminomethyl-1,2,3,4-tetrahydronaphthalin in 16 ml Dichlormethan gelöst. Bei max. +25° C wurden dann 0,9 g (6 mmol) Butansulfonsäurechlorid zugetropft. Anschließend ließ man die Reaktionslösung 18 h bei Raumtemperatur rühren. Dann wurden 16 ml Wasser zugesetzt. Die Mischung wurde mit 1N Natronlauge auf pH 10 gestellt. Die organische Phase wurde abgetrennt und mit Wasser gewaschen. Nach Trocknen über Natriumsulfat wurde das Lösemittel im Vakuum abdestilliert. Der Rückstand wurde durch Säulenchromatographie auf Kieselgel mit Diisopropylether/Triethylamin 99,5 : 0,5 als Fließmittel gereinigt. Eine weitere Reinigung erreichte man, wenn die Base in Ether gelöst wurde und durch Zutropfen in etherischer Chlorwasserstofflösung ins Hydrochlorid überführt wurde. Nach Zugabe von Wasser und Abtrennung der organischen Phase wurde die Base durch Zusatz von Na tronlauge wieder freigesetzt und durch Extraktion mit Ether isoliert.
Ausbeute: 49% der Theorie (Öl)
$R_f$ : 0,10 (Kieselgel; Diisopropylether/Triethylamin 99,5 : 0,5

Beispiel 16

2-Dipropylamino-8-ethoxycarbonylamido-methyl-1,2,3,4-tetrahydronaphthalin

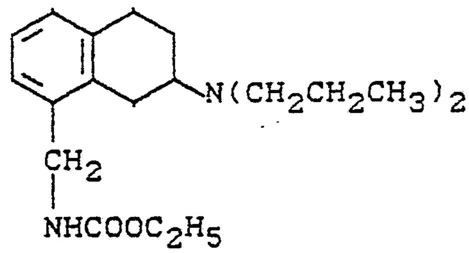

Unter Stickstoff wurden 1,6 g (6 mmol) 2-Dipropylamino-8-aminomethyl-1,2,3,4-tetrahydronaphthalin in 16 ml Diethylether gelöst. Unter Kühlung wurde 0,58 ml Chlorameisensäureethylester bei max. + 5°C zugetropft. Anschließend wurde der Ansatz noch 1 h bei +5°C gerührt und danch in Eiswasser eingerührt. Die wäßrige Phase wurde mit 1 N Natronlauge auf pH 10 gestellt und danach mit Ether extrahiert. Die nun erhaltene organische Phase wurde mit Wasser gewaschen und über Natriumsulfat getrocknet. Das Lösemittel wurde abdestilliert und der verbleibende Rückstand durch Säulenchromatographie auf Kieselgel gereinigt. Als Fließmittel wurde Diisopropylether/Triethylamin 99,5 : 0,5 verwendet.
$R_f$ : 0.20

Beispiel 17

2-Dipropylamino-8-ethoxycarbonylamido-methyl-1,2,3,4-tetrahydronaphthalin Hydrochlorid

Die freie Base der Verbindung aus Beispiel 16 wurde nach Lösen in Diisopropylether mit etherischer Chlorwasserstofflösung ins Hydrochlorid überführt. Das stark hygroskopische Hydrochlorid wurde abgesaugt und im Vakuum getrocknet.
Ausbeute: 18% der Theorie

Beispiel 18

2-Dipropylamino-8-(3,3-diethylureido)methyl-1,2,3,4-tetrahydronaphthalin

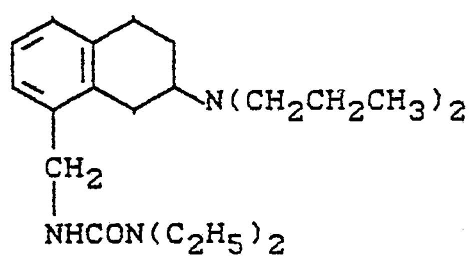

Unter Stickstoff wurden 1,6 g (6 mmol) 2-Dipropylamino-8-aminomethyl-1,2,3,4-tetrahydronaphthalin in 16 ml Dichlormethan gelöst. Bei max. +25° C wurden dann 0,81 g (6 mmol) Diethylcarbamidsäurechlorid zugetropft. Anschließend ließ man den Ansatz 26 h bei Raumtemperatur rühren. Dann wurde der Ansatz in 160 ml Eiswasser eingerührt. Die Mischung wurde mit 1 N Natronlauge auf pH 9 gestellt und mit Dichlormethan extrahiert. Die organische Phase wurde mit Wasser gewaschen und über Natriumsulfat getrocknet. Anschließend wurde das Lösemittel abdestilliert. Der Rück stand wurde durch Säulenchromatographie gereinigt. Als Fließmittel wurde Diisopropylether/Isopropanol/Triethylamin 80:19,5:0,5 verwendet. Das Endprodukt ließ sich aus Petroleumbenzin 30-50 kristallisieren und wurde bei +40° C im Vakuum getrocknet.
Ausbeute: 64% der Theorie
Fp. : 86 - 87° C

$R_f$ : 0.42

Beispiel 19

2-Dipropylamino-8-ureidomethyl-1,2,3,4-tetrahydronaphthalin

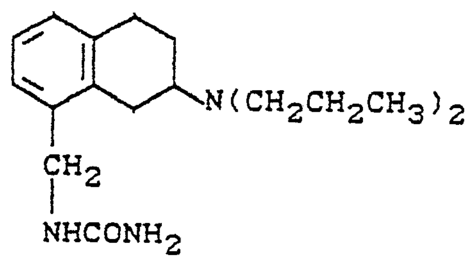

Unter Stickstoff wurden 2,6 g (10 mmol) 2-Dipropylamino-8-aminomethyl-1,2,3,4-tetrahydronaphthalin in 22 ml 1 N Salzsäure gelöst. Anschließend tropfte man eine Lösung aus 0,81 g (10 mmol) Kaliumcyanat und 6 ml Wasser bei max. +25°C zu und ließ den Ansatz 4 h bei Raumtemperatur reagieren. Danach wurden 20 ml Diethylether zugesetzt. Die wäßrige Phase wurde abgetrennt und mit 1 N Natronlauge auf pH 9 gestellt. Das ausgefällte Reaktionsprodukt wurde abgesaugt und bei +40°C im Vakuum getrocknet. Durch Umkristallisieren aus Methanol wurde das Produkt gereinigt.
Ausbeute: 38% der Theorie
Fp. : 182,5 - 183,5° C
$R_f$ : 0,55

Beispiel 20

2-Dipropylamino-8-(3-methylureido)methyl-1,2,3,4-tetrahydronaphthalin

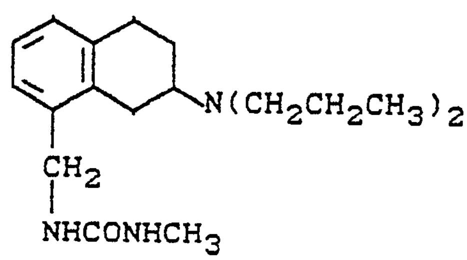

Unter Stickstoff wurden 1,6 g (6 mmol) 2-Dipropylamino-8-aminomethyl-1,2,3,4-tetrahydronaphthalin in 16 ml Toluol gelöst. Bei max. +25° C wurden 0,34 g (6 mmol) Methylisocyanat zugetropft. Anschließend ließ man den Ansatz noch 1,5 h bei Raumtemperatur rühren. Danach wurde das auskristallisierte Reaktionsprodukt abgesaugt und unter Vakuum bei +40° C getrocknet.
Ausbeute: 65% der Theorie
$R_f$ : 0,45

Beispiel 21

2-Dipropylamino-8-formamidomethyl-1,2,3,4-tetrahydronaphthalin

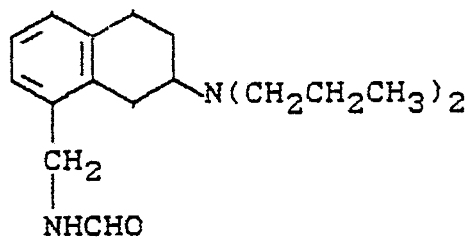

Unter Stickstoff wurden 2,04 ml (21,5 mmol) Acetanhydrid und 0,86 ml (22,8 mmol) Ameisensäure 2 h bei +60° C gerührt. Nach Abkühlung auf Raumtemperatur wurden 2,6 g (10 mmol) 2-Dipropylamino-8-aminomethyl-1,2,3,4-tetrahydronaphthalin bei max. +25° C zugetropft. Nach 15 min Nachreaktion wurden 5 ml Ether zugesetzt. Anschließend wurde der Ansatz noch 18 h bei Raumtemperatur gerührt und danach mit 20 ml Ether und 20 ml Wasser verdünnt. Die wäßrige Phase wurde mit 1 N Natronlauge auf pH 9 gestellt und mit Ether extrahiert. Die nun erhaltene organische Phase wurde mit Wasser gewaschen, über Natriumsulfat getrocknet und das Lösemittel abdestilliert. Der Rückstand wurde aus Petroleumbenzin 60/80 umkristallisiert.
Ausbeute: 46% der Theorie
Fp. : 74,5 - 75,5° C

Beispiel 22

2-Dipropylamino-8-(2-hydroxyethoxy)-1,2,3,4-tetrahydronaphthalin Hydrochlorid

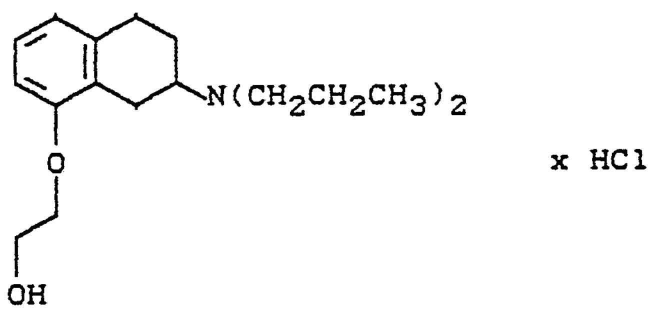

0,7 g (23 mmol) Natriumhydrid (80% in Paraffin) wurde in 5 ml Diethylenglykoldimethylether vorgelegt. Bei +25° C tropfte man eine Lösung aus 4,9 g (20 mmol) 2-Dipropylamino-8-hydroxy-1,2,3,4-tetrahydronaphthalin und 20 ml Diethylenglykoldimethylether zu. Man rührte 30 min bei +50° C nach. Nach Abkühlen auf +25° C tropfte man 2,5 g (20 mmol) 2-Bromethanol zu. Es wurde 2 h bei +100° C nachgerührt. Der abgekühlte Ansatz wurde auf Wasser gegossen und 2 mal mit Essigester extrahiert. Die organische Phase wurde über Natriumsulfat getrocknet und eingedampft. Nicht umgesetztes Ausgangsmaterial wurde durch Chromatographie mit Chloroform über basisches Aluminiumoxid Stufe I entfernt. Anschließend chromatographierte man über Kieselgel 60, 40 - 63 µm mit Diisopropylether/Ethanol 3:2. Man erhielt 700 mg eines zähen Öles. Man löste in Ether und fällte mit etherischer Salzsäure das Hydrochlorid. Man extrahierte mit Wasser und wusch noch 1 mal mit Ether. Anschließend wurde mit Methylenchlorid versetzt und mit Ammoniak auf pH 11 eingestellt. Die organische Phase wurde abgetrennt, mit Natriumsulfat getrocknet, und filtriert. Dann wurde wieder mit etherischer Salzsäure das Hydrochlorid gefällt. Man dampfte zur Trockene ein und erhielt einen glasartigen Rückstand.
Ausbeute: 6% der Theorie
$R_f$ : 0,392 (Diisopropylether : Ethanol 3:2)

Beispiel 23

2-Dipropylamino-8-carbamoylmethoxy-1,2,3,4-tetrahydronaphthalin

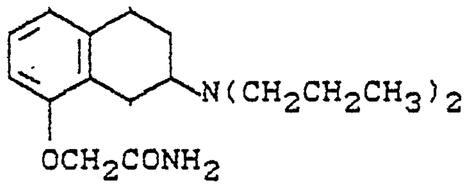

0,35 g (11 mmol) einer 80%igen Natriumhydrid-Dispersion in Öl wurden mit Petrolether gewaschen und dann in Diglyme unter Stickstoff suspendiert. Unter leichter Kühlung wurde bei +20 - +25° C eine Lösung aus 2,50 g (10 mmol) 2-Dipropylamino-8-hydroxy-1,2,3,4-tetrahydronaphthalin und 5,9 ml Diglyme innerhalb von 30 min zugetropft. Anschließend wurde der Ansatz noch 30 min gerührt und dann 15 min auf +50° C erwärmt. Danach wurden bei +20 - +25° C 0,94 g (10 mmol) Chloracetamid portionsweise eingetragen. Dann wurde der Ansatz 1 h auf +135° C erwärmt und nach Abkühlung in 100 ml Eiswasser eingerührt. Der Niederschlag wurde abgesaugt und bei +40° C unter Vakuum getrocknet. Die Rohsubstanz wurde durch zweimaliges Umkristallisieren aus Isopropanol gereinigt.
Ausbeute: 50% der Theorie
Fp. : 132-132.5° C
$R_f$ : 0,43 (Kieselgel; Toluol/Methanol 70:30)

Beispiel 24

2-Dipropylamino-8-(2-aminoethoxy)-1,2,3,4-tetrahydronaphthalin

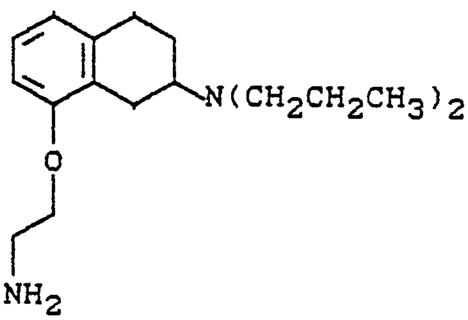

12,5 g (0,328 mol) Lithiumaluminiumhydrid wurden unter Stickstoff in 200 ml absolutem Ether vorgelegt. Dazu gab man in kleinen Portionen eine Suspension aus 25 g (0,082 mol) 2-Dipropylamino-8-(carbonamidomethoxy)-1,2,3,4-tetrahydronaphthalin in 500 ml absolutem Ether. Man rührte 4 h bei +25° C nach und zersetzte dann vorsichtig mit Wasser. Es wurde abgesaugt und der Rückstand 3 mal gründlich mit Ether nachgewaschen. Die Etherphase wurde abgetrennt, über Natriumsulfat getrocknet und dann zur Trockne eingedampft. Man chromatographierte über Kieselgel 60, 40 - 63 µm mit Isopropanol/Triethylamin 95:5.
Ausbeute: 73,6% der Theorie
$R_f$ : 0,208

Beispiel 25

2-Dipropylamino-8-(2-methansulfonamido-ethoxy)-1,2,3,4-tetrahydronaphthalin

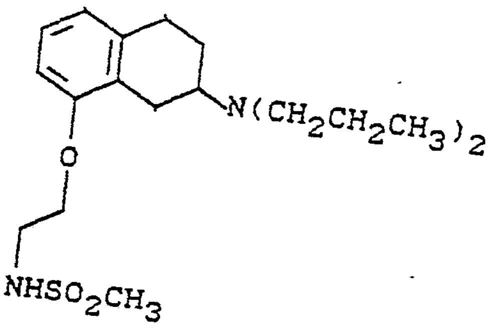

1,45 g (5 mmol) 2-Dipropylamino-8-(2-amino-ethoxy)-1,2,3,4-tetrahydronaphthalin und 1,38 g (10 mmol) Kaliumcarbonat wurden in 20 ml Toluol vorgelegt. Man tropfte 0,63 g (5,5 mmol) Methansulfonsäurechlorid zu, und rührte 3 h bei Raumtemperatur nach. Dann wurde abfiltriert und eingedampft. Der erhaltene Rückstand wurde mit Petrolether verrührt und abgesaugt. Man chromatographierte mit Diisopropylether/Ethanol 3:2 über Kieselgel 60, 40 -63 µm. Das Produkt wurde aus Petrolether mit Aktivkohle umkristallisiert.
Ausbeute: 30% der Theorie
Fp.: 76° C

<u>Beispiel 26</u>

2-Dipropylamino-8-(2-butansulfonamidoethoxy)-1,2,3,4-tetrahydronaphthalin

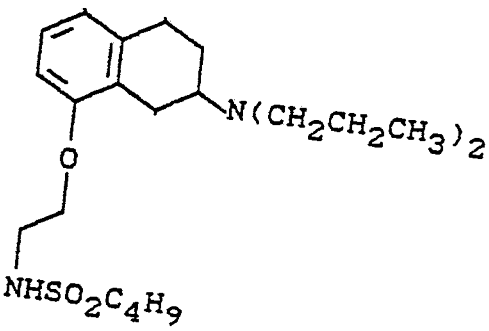

2,5 g (8,6 mmol) 2-Dipropylamino-8-(2-amino-ethoxy)-1,2,3,4-tetrahydronaphthalin und 2,4 g (17,2 mmol) Kaliumcarbonat wurden in 40 ml Toluol vorgelegt. Man tropfte 1,5 g (9,5 mmol) Butansulfonsäurechlorid zu, und rührte dann noch 2 h bei Raumtemperatur nach. Dann wurde abfiltriert und einrotiert. Man chromatographierte mit Diisopropylether/Ethanol 3:2 über Kieselgel 60, 40 - 63 µm. Es wurde aus Petrolether umkristallisiert.
Ausbeute: 27% der Theorie
$R_f$ : 0,517

<u>Beispiel 27</u>

2-Dipropylamino-8-(2-propionylamido-ethoxy)-1,2,3,4-tetrahydronaphthalin

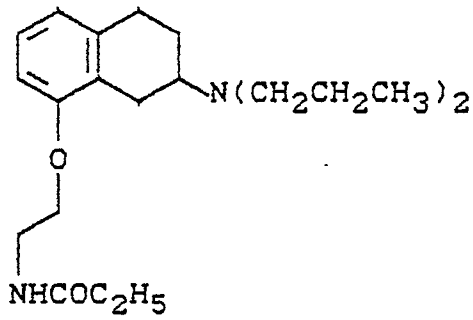

1,45 g (5 mmol) 2-Dipropylamino-8-(2-aminoethoxy)-1,2,3,4-tetrahydronaphthalin und 1,38 g (10 mmol) Kaliumcarbonat wurden in 20 ml Toluol vorgelegt. Man tropfte dann 0.5 g (5,5 mmol) Propionsäurechlorid zu und rührte anschließend 30 min unter Rückfluß nach. Dann wurde abfiltriert und eingedampft. Der Rückstand wurde mit n-Hexan verrührt und abgesaugt.
Ausbeute: 75% der Theorie
Fp. : 67 - 68° C

Beispiel 28

2-Dipropylamino-8-(2-ethoxycarbonylamido-ethoxy)-1,2,3,4-tetrahydronaphthalin

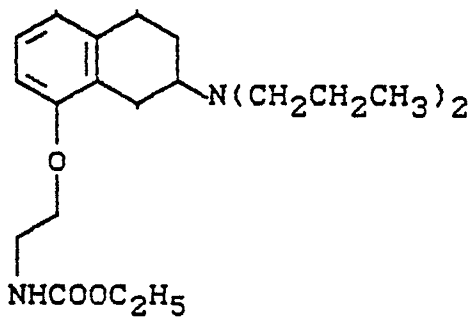

1.45 g (5 mmol) 2-Dipropylamino-8-(2-aminoethoxy)-1,2,3,4-tetrahydronaphthalin und 1,38 g (10 mmol) Kaliumcarbonat wurden in 20 ml Toluol vorgelegt. Man tropfte dann 0,53 ml (5,5 mmol) Chlorameisensäureethylester zu und rührte noch 20 min bei +25° C nach. Dann wurde abfiltriert und eingedampft. Man chromatographierte mit Diisopropylether/Ethanol 3:2 über Kieselgel 60, 40-63 µm.
Ausbeute: 71,8% der Theorie
$R_f$ : 0,492

Beispiel 29

2-Dipropylamino-8-[2-(3.3-diethylureido)-ethoxy]-1.2.3.4-tetrahydronaphthalin

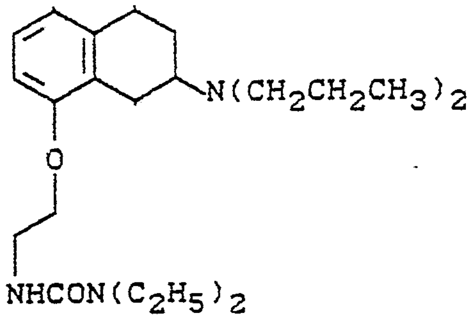

1,45 g (5 mmol) 2-Dipropylamino-8-(2-aminoethoxy)-1,2,3,4-tetrahydronaphthalin und 1,38 g (10 mmol) Kaliumcarbonat wurden in 20 ml Toluol vorgelegt. Man tropfte dann 0,77 g (5,5 mmol) Diethylcarbamidsäurechlorid zu, und rührte 2 h bei +100° C nach. Dann wurde abfiltriert und eingedampft. Man chromatographierte mit Diisopropylether/Ethanol 3:2 über Kieselgel 60, 40 - 63 µm.
Ausbeute: 56,4% der Theorie
$R_f$ : 0,408

Beispiel 30

2-Dipropylamino-8-ureidoethoxy-1,2,3,4-tetrahydronaphthalin

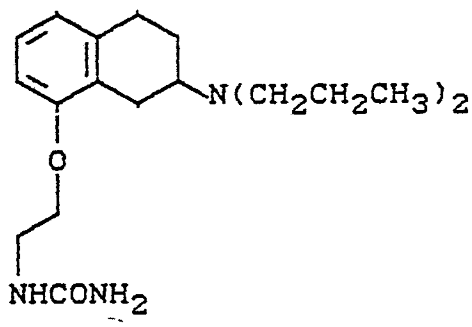

1,45 g (5 mmol) 2-Dipropylamino-8-(2-aminoethoxy)-1,2,3,4-tetrahydronaphthalin wurden in 10 ml Wasser und 10 ml 1N Salzsäure gelöst. Bei einer Temperatur von +60° C wurde dann eine Lösung aus 4,1 g (50 mmol) Kaliumcyanat und 20 ml Wasser zugetropft. Es wurde 3 h bei +60° C nachgerührt, wobei das Umsetzungsprodukt ausfiel. Man saugte ab und verrührte den Rückstand heiß mit Petrolether. Nach dem Abkühlen wurde abgesaugt.
Ausbeute: 83,8% der Theorie
Fp. : 107 - 108° C

Beispiel 31

2-Dipropylamino-8-[2-(3-methylureido)ethoxy]-1,2,3,4-tetrahydronaphthalin

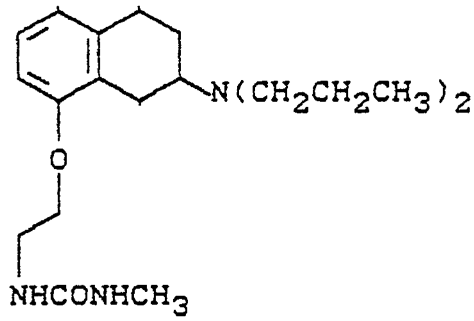

1,45 g (5 mmol) 2-Dipropylamino-8-(2-aminoethoxy)-1,2,3,4-tetrahydronaphthalin wurden in 20 ml Toluol vorgelegt. Man katalysierte mit 3 Tropfen Triethylamin. und tropfte dann 0,325 ml (5,5 mmol) Methylisocyanat zu. Man rührte 1 h bei +25° C nach. Man dampfte ein und verrührte den erhaltenen Rückstand heiß mit Diisopropylether. Es wurde abgekühlt und abgesaugt.
Ausbeute: 80,5% der Theorie
Fp. : 104 - 105° C

Beispiel 32

2-Dipropylamino-8-[2-(3-butylureido)ethoxy]-1,2,3,4-tetrahydronaphthalin

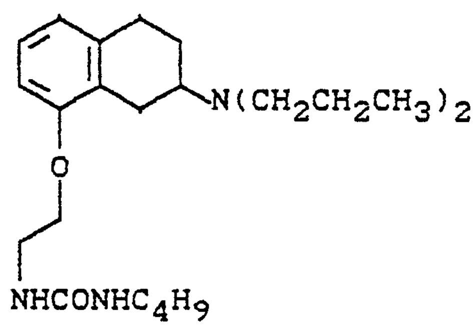

1,45 g (5 mmol) 2-Dipropylamino-8-(2-aminoethoxy)-1,2,3,4-tetrahydronaphthalin wurden in 25 ml Toluol vorgelegt. Man katalysierte mit 3 Tropfen Triethylamin und tropfte dann 0,63 ml (5,5 mmol) Butylisocyanat zu. Man rührte 2 h bei Raumtemperatur nach. Es wurde zur Trockene eingedampft und der Rückstand aus Petrolether/Diisopropylether kristallisiert.
Ausbeute: 82% der Theorie
Fp. : 90 - 91° C

Beispiel 33

2-Dipropylamino-8-(2-formylamido-ethoxy)-1.2.3.4-tetrahydronaphthalin

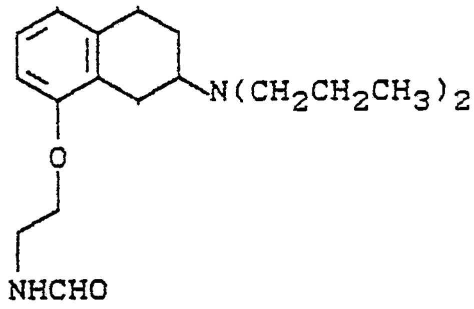

1,02 ml (10,8 mmol) Essigsäureanhydrid und 0,86 ml (22,8 mmol) Ameisensäure wurden 4 h auf +60° C erhitzt. Dann wurde auf Raumtemperatur abgekühlt und 1,45 g (5 mmol) 2-Dipropylamino-8-(2-amino-ethoxy)-1,2,3,4-tetrahydronaphthalin zugetropft. Nach Abklingen der exothermen Reaktion wurde dann noch 2 h bei Raumtemperatur nachgerührt. Dann wurde Essigester zugesetzt und der Ansatz mit 1 molarer Natronlauge auf pH 11 eingestellt. Die wäßrige Phase wurde noch 1 mal mit Essigester extrahiert. Die organischen Phasen wurden vereinigt, über Natriumsulfat getrocknet und eingedampft. Der Rückstand wurde aus Petrolether mit Aktivkohle umkristallisiert.
Ausbeute: 58,4% der Theorie
Fp. : 93° C

Beispiel 34

2-Dipropylamino-8-[2-(N,N-dimethylaminosulfonyl)ethenyl]-1,2,3,4-tetrahydronaphthalin

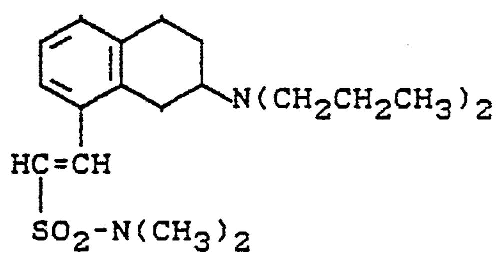

1,23 g (10 mmol) Methansulfonsäuredimethylamid und 150 mg (1 mmol) Benzyltriethylammoniumchlorid wurden mit 20 ml 50%iger Natronlauge in 20 ml Methylenchlorid bei Raumtemperatur 15 min kräftig gerührt. Anschließend tropfte man bei Raumtemperatur eine Lösung aus 7,8 g (30 mmol) 2-Dipropylamino-8-formyl-1,2,3,4-tetrahydronaphthalin in 5 ml Methylenchlorid zu, und rührte über Nacht nach. Dann wurde mit viel Wasser verdünnt und mehrfach mit Methylenchlorid extrahiert. Die organische Phase wurde über Natriumsulfat getrocknet und dann eingedampft. Man chromatographierte über Kieselgel 60, 63 - 200 µm, mit a) Cyclohexan/Essigester/Triethylamin : 80:19:1 und b) Cyclohexan/Essigester/Triethylamin: 50:49:1.
Ausbeute: 50,7% der Theorie
$R_f$ : 0,617 (Diisopropylether : Ethanol 3:2)

Beispiel 35

2-Dipropyiamino-8-[2-(N.N-dimethylaminosulfonyl)ethyl]-1.2.3.4-tetrahydronaphthalin

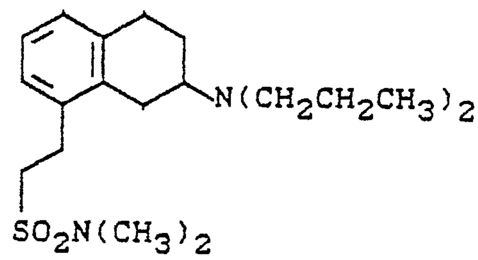

320 mg (8 mmol) Lithiumaluminiumhydrid wurden unter Argon in 20 ml absolutem Diethylether vorgelegt. Man tropfte dann eine Lösung aus 1,46 g (4 mmol) 2-Diisopropylamino-8-[2-(N,N-dimethylaminosulfonyl)ethenyl]-1,2,3,4-tetrahydronaphthalin in 10 ml absolutem Diethylether zu. Es wurde 2,5 h unter Rückfluß nachgerührt. Man zersetzte dann vorsichtig mit Wasser, saugte ab und wusch den Rückstand gründlich mit Essigester. Dann wurden die Phasen getrennt und die organische Phase über Natriumsulfat getrocknet und dann eingedampft. Man kristallisierte aus Isopropanol/Wasser um, saugte ab und trocknete im Hochvakuum bei +50° C.
Ausbeute: 55,2% der Theorie
Fp. : 84° C

Beispiel 36

2-Dipropylamino-8-(2-nitro-ethenyl)-1,2,3,4-tetrahydronaphthalin

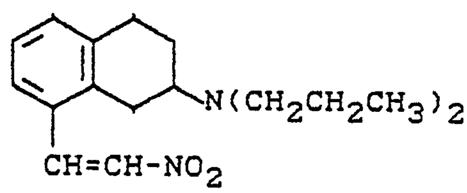

14 g (54 mmol) 2-Dipropylamino-8-formyl-1,2,3,4-tetrahydronaphthalin, 14 ml Nitromethan und 5,6 g (73 mmol) Ammoniumacetat wurden in 56 ml Eisessig 2 h lang unter Rückfluß nachgerührt. Nach Abkühlen wurde mit 5 molarer Natronlauge auf pH 12 gestellt und mit Essigester 3 mal extrahiert. Die organische Phase wurde mit Natriumsulfat getrocknet und dann eingedampft. Man chromatographierte mit Diisopropylether/Triethylamin 99,5:05, über Kieselgel 60, 63 - 200 µm.
Ausbeute: 52,1% der Theorie
$^1$H-NMR (300 MHz, $CDCl_3$): Protonen des Nitroethylenrestes:
δ = 7,38 ppm; J = 15 Hz, 1H,
δ = 8,25 ppm; J = 15 Hz, 1H

Beispiel 37

2-Dipropylamino-8-(2-amino-ethyl)-1.2.3.4-tetrahydronaphthalin

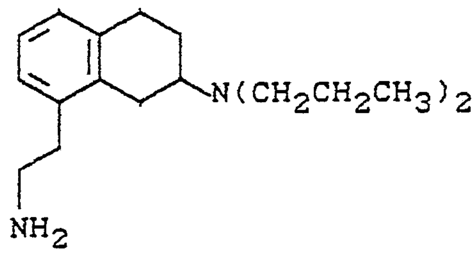

820 mg (20,4 mmol) Natriumborhydrid wurden unter Stickstoff in 30 ml absolutem Tetrahydrofuran bei 0° C vorgelegt. Bei dieser Temperatur tropfte man 3,24 ml (25,8 mmol) Bortrifluorid-Diethylether-Komplex zu, und rührte anschließend 15 min bei Raumtemperatur nach. Man tropfte dann eine Lösung aus 1,3 g (4,3 mmol) 2-Dipropylamino-8-(2-nitro-ethenyl)-1.2.3.4-tetrahydronaphthalin und 10 ml absolutem Tetrahydrofuran zu, und rührte dann 5,5 h unter Rückfluß nach. Nach Abkühlen auf Raumtemperatur tropfte man vorsichtig 55 ml Eiswasser zu und säuerte dann mit 55 ml 1 N Salzsäure an. Anschließend wurde 2 h bei +80° - +85° C nachgerührt. Die abgekühlte wäßrige Phase wurde 2 mal mit 35 ml Ether gewaschen, dann mit 5 molarer Natronlauge auf pH 12 eingestellt und das Produkt mit Methylenchlorid extrahiert. Man trocknete über Natriumsulfat und dampfte ein. Anschließend chromatographierte man mit Isopropanol/Triethylamin 95:5 über Kieselgel 60, 40 - 63 µm.
Ausbeute: 50,8% der Theorie
$R_f$ : 0,192

Beispiel 38

2-Dipropylamino-8-(2-methansulfonamido-ethyl)-1,2,3,4-tetrahydronaphthalin Hydrochlorid

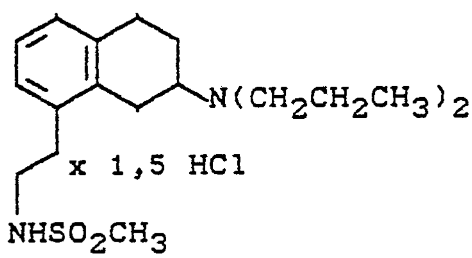

1,2 g (4.4 mmol) 2-Dipropylamino-8-(2-amino-ethyl)-1,2,3,4-tetrahydronaphthalin und 1,21 g (8,8 mmol) Kaliumcarbonat wurden in 20 ml Methylenchlorid vorgelegt. Bei 25° C wurden dann 550 mg (4.8 mmol) Methansulfonsäurechlorid zugetropft. Es wurde über Nacht bei Raumtemperatur nachgerüht. Dann wurde abfiltriert, 1 mal mit Wasser gewaschen, über Natriumsulfat getrocknet und eingedampft. Man versetzte den Rückstand mit Diethylether, gab soviel Methylenchlorid zu bis alles gelöst war, und fällte mit etherischer Salzsäure das Hydrochlorid. Man saugte ab und trocknete bei 40° C im Hochvakuum.
Ausbeute: 56,2% der Theorie
Fp. : 212° C (Zers.)

Beispiel 39

2-Dipropylamino-8-(2-butansulfonamido-ethyl)-1,2,3,4-tetrahydronaphthalin

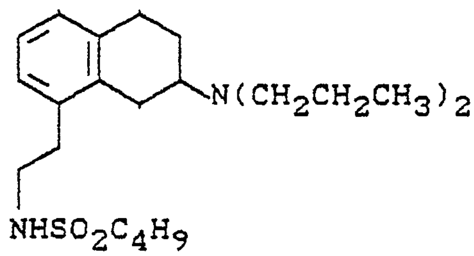

1,1 g (4 mmol) 2-Dipropylamino-8-(2-amino-ethyl)-1,2,3,4-tetrahydronaphthalin und 1,1 g (8 mmol) Kaliumcarbonat wurden in 20 ml Methylenchlorid vorgelegt. Man tropfte dann 690 mg (4,4 mmol) Butansulfonsäurechlorid zu, und rührte 5,5 h bei Raumtemperatur nach. Dann wurden 20 ml Wasser zugegeben und 10 min kräftig verrührt. Die Phasen wurden getrennt und die wäßrige Phase noch 1 mal mit Methylenchlorid extrahiert. Die organischen Phasen wurden vereinigt, über Natriumsulfat getrocknet und dann eingedampft. Man chromatographierte mit Essigester über Kieselgel 60, 40 - 63 µm.
Ausbeute: 72,9% der Theorie
Rf : 0.592 (Diisopropylether: Ethanol 3:2)

Beispiel 40

2-Dipropylamino-8-[2-(p-chlorbenzolsulfonamido)ethyl]-1,2,3,4-tetrahydronaphthalin

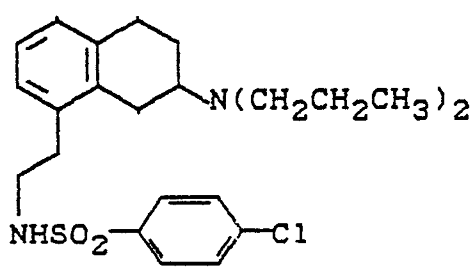

1,1 g (4 mmol) 2-Dipropylamino-8-(2-amino-ethyl)-1,2,3,4-tetrahydronaphthalin wurden mit 2 ml 50%iger Kaliumcarbonatlösung in 15 ml Methylenchlorid vorgelegt. Unter kräftigem Rühren wurde dann eine Lösung von 930 mg (4,4 mmol) p-Chlorbenzolsulfonsäurechlorid in 15 ml Methylenchlorid zugetropft. Es wurde 1 h bei Raumtemperatur nachgerührt. Es wurde mit 20 ml Wasser versetzt und kräftig verrührt. Die Phasen wurden getrennt und die wäßrige Phase noch 1 mal mit Methylenchlorid extrahiert. Die organischen Phasen wurden vereinigt, über Natriumsulfat getrocknet und eingedampft. Man chromatographierte mit Essigester über Kieselgel 60, 40 - 63 µm.
Ausbeute: 97,4% der Theorie
$R_f$ : 0,650 (Diisopropylether : Ethanol 3:2)

Beispiel 41

2-Dipropylamino-8-(2-ethoxycarbonylamido-ethyl)-1,2,3,4-tetrahydronaphthalin

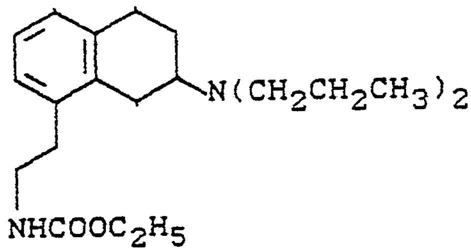

1,1 g (4 mmol) 2-Dipropylamino-8-(2-amino-ethyl)-1,2,3,4-tetrahydronaphthalin und 1,1 g (8 mmol) Kaliumcarbonat wurden in 20 ml Methylenchlorid vorgelegt. Bei +25° C tropfte man dann 0,45 ml (4,4 mmol) Chlorameisensäureethylester zu und rührte noch 1 h bei Raumtemperatur nach. Dann wurden 20 ml Wasser zugegeben und 10 min kräftig verrührt. Die Phasen wurden getrennt und die wäßrige Phase noch 1 mal mit Methylenchlorid extrahiert. Die organischen Phasen wurden vereinigt, über Natriumsulfat getrocknet und dann eingedampft. Man löste dann in 30 ml 3 N Salzsäure, extrahierte 3 mal mit Diethylether, stellte wieder auf pH 12 ein und extrahierte das Produkt mit Essigester, trocknete über Natriumsulfat und dampfte ein.
Ausbeute: 85,8% der Theorie
$R_f$ : 0,598 (Diisopropylether : Ethanol 3:2)

Beispiel 42

2-Dipropylamino-8-(2-benzyloxycarbonylamido-ethyl)-1,2,3,4-tetrahydronaphthalin

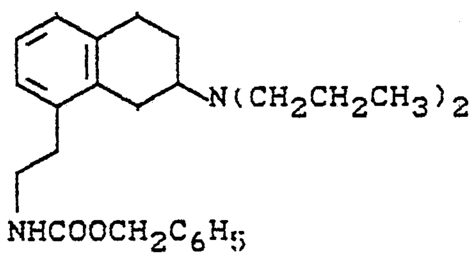

3,5 g (12,75 mmol) 2-Dipropylamino-8-(2-amino-ethyl)-1,2,3,4-tetrahydronaphthalin und 3,5 g (25,5 mmol) Kaliumcarbonat wurden in 50 ml Methylenchlorid vorgelegt. Bei 25° C tropfte man dann 4,7 ml (14 mmol) Chlorameisensäurebenzylester (50%ige Lösung in Toluol) zu und rührte noch 2 h bei Raumtemperatur nach. Anschließend gab man 50 ml Wasser zu und rührte 10 min kräftig durch. Die Phasen wurden getrennt und die wäßrige Phase noch 1 mal mit Methylenchlorid extrahiert. Die organischen Phasen wurden vereinigt, über Natriumsulfat getrocknet und dann eingedampft. Man chromatographierte mit Essigester über Kieselgel 60, 40 - 63 µm.
Ausbeute: 92,1% der Theorie
$R_f$ : 0,567 (Diisopropylether : Ethanol 3:2)

Beispiel 43

2-Dipropylamino-8-(2-ureido-ethyl)-1,2,3,4-tetrahydronaphthalin

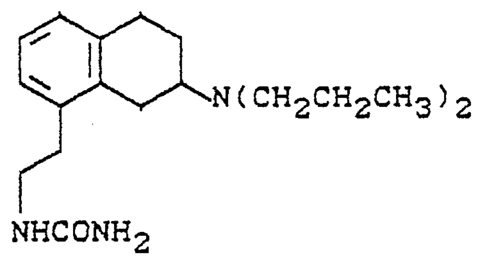

1,1 g (4 mmol) 2-Dipropylamino-8-(2-aminoethyl)-1,2,3,4-tetrahydronaphthalin wurden in 8 ml Wasser und 8 ml 1 N Salzsäure gelöst und auf +60°C erhitzt. Bei dieser Temperatur tropfte man eine Lösung aus 3,24 g (40 mmol) Kaliumcyanat und 15 ml Wasser zu. Es wurde 1 h bei +60° C nachgerührt. Dann wurde abgekühlt und abgesaugt. Man kristallisierte aus Petrolether/Diisopropylether mit Aktivkohle um.
Ausbeute: 43,4% der Theorie
Fp. : 123 - 124°C

Beispiel 44

2-Dipropylamino-8-[2-(3-methylureido)ethyl]-1,2,3,4-tetrahydronaphthalin

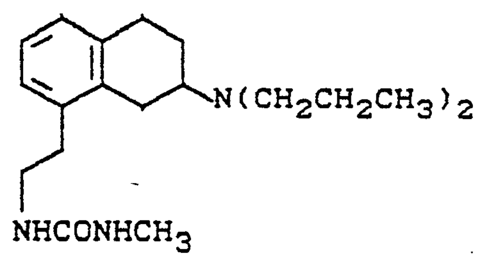

1.3 g (4,7 mmol) 2-Dipropylamino-8-(2-amino-ethyl)-1,2,3,4-tetrahydronaphthalin wurden in 20 ml Toluol vorgelegt. Man katalysierte mit 3 Tropfen Triethylamin und tropfte dann 0,31 ml (5,17 mmol) Methylisocyanat zu. Man rührte über Nacht bei Raumtemperatur nach und dampfte dann ein. Anschließend chromatographierte man mit Diisopropylether/Ethanol/Triethylamin über Kieselgel 60, 40 - 63 µm. Das Produkt wurde mit Petrolether und wenig Diisopropylether verrührt und abgesaugt.
Ausbeute: 21,2% der Theorie
Fp. : 70° C

Beispiel 45

2-Dipropylamino-8-(2-formylamido-ethyl)-1.2.3,4-tetrahydronaphthalin

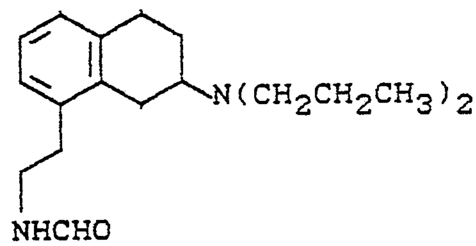

1,02 ml (10,8 mmol) Essigsäureanhydrid und 0,896 ml (22,8 mmol) Ameisensäure wurden 4 h auf +50 - +60° C erhitzt. Man kühlte auf Raumtemperatur ab und tropfte dann 1,4 g (5mmol) 2-Dipropylamino-8-(2-amino-ethyl)-1,2,3,4-tetrahydronaphthalin zu und hielt die Temperatur dabei unter +40° C. Anschließend wurde 2 h bei Raumtemperatur nachgerührt. Es wurde dann mit 5 molarer Natronlauge auf pH 12 eingestellt und mit Methylenchlorid extrahiert. Man trocknete über Natriumsulfat und dampfte dann ein. Es wurde mit Diisopropylether/Ethanol 3:2 über Kieselgel 60, 40 - 63 um chromatographiert.
Ausbeute: 56,3% der Theorie
$R_f$ : 0,242
(Alle DC's wurden auf DC-Alufolien, Kieselgel 60, F254 5 x 7,5 cm, Laufhöhe 6 cm durchgeführt).

Beispiel 46

2-Dipropylamino-8-[2-(N-methylaminosulphonyl)ethenyl]-1,2,3,4-tetrahydronaphthalin

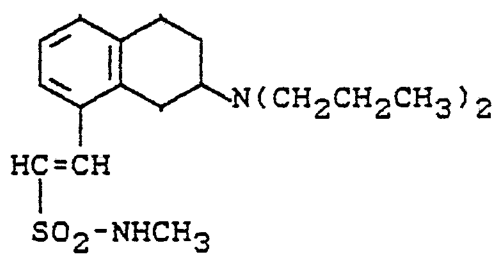

Diese Verbindung wurde in Analogie zu Beispiel 34 hergestellt. Als Startmaterial wurde N-Methylmethan Sulfonamid eingesetzt.
Ausbeute: 49,6 % der Theorie
$R_f$ : 0,383 (Diisopropylether:Ethanol 3:2)

Beispiel 47

2-Dipropylamino-8-[2-(N-methylaminosulphonyl)ethyl]-1,2,3,4-tetrahydronaphthalin

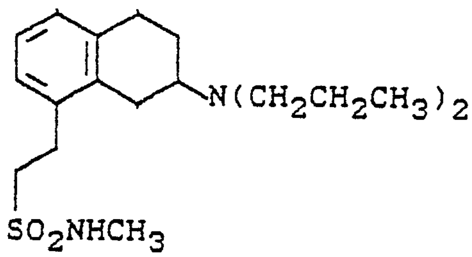

Diese Verbindung wurde in Analogie zu Beispiel 35 durch Reduktion der Verbindung des Beispiels 47 mit Lithiumaluminiumhydrid hergestellt.
Ausbeute: 33 % der Theorie
$R_f$ : 0,492 (Diisopropylether:Ethanol 3:2)

Beispiel 48

2-Dipropylamino-8-[2-(2-naphthyl)sulfonamido-ethyl]-1,2,3,4-tetrahydronaphthalin

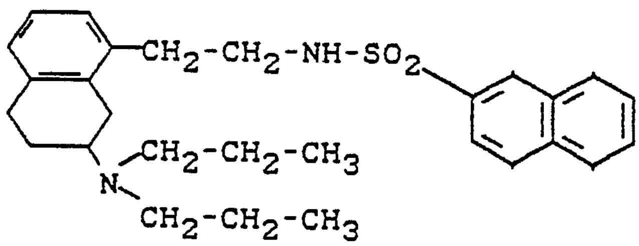

961 mg (3,5 mmol) 2-Dipropylamino-8-(2-amino-ethyl)-1,2,3,4-tetrahydronaphthalin wurden in 15 ml Methylenchlorid mit 1,75 ml 50 %iger Kaliumcarbonatlösung vorgelegt. Man tropft eine Lösung aus 873 mg (3,85 mmol) Naphthalin-2-sulfonsäurechlorid und 5 ml Methylenchlorid zu und rührte 20 h bei Raumtemperatur nach. Dann wurde mit Wassr und Methylenchlorid verdünnt. Die organische Phase wurde abgetrennt, über Natriumsulfat getrocknet und eingedampft. Anschließend chromatographierte man mit Cyclohexan/Essigester 1:1 über Kieselgel 60, 40-63 µm.
Ausbeute: 1,32 g = 81,2 % der Theorie
$R_f$ : 0,117 (Cyclohexan:Essigester 1:1)

Beispiel 49

2-Dipropylamino-8-[2-(3,4-dichlorphenyl)-sulfonamidoethyl]-1,2,3,4-tetrahydronaphthalin

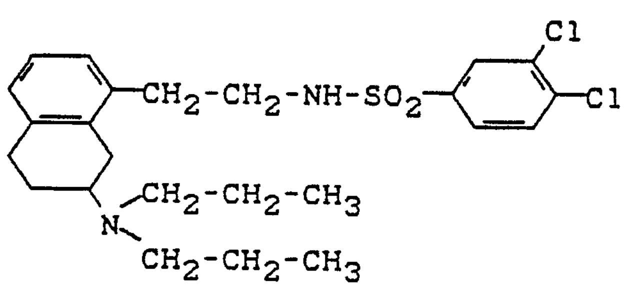

500 mg (1,82 mmol) 2-Dipropylamino-8-(2-amino-ethyl)-1,2,3,4-tetrahydronaphthalin wurden in 10 ml Methylenchlorid mit 1 ml 50 %iger Kaliumcarbonatlösung vorgelegt. Man tropfte eine Lösung aus 491 mg

(2,0 mmol) 3,4-Dichlorbenzolsulfonsäurechlorid und 5 ml Methylenchlorid zu und rührte 2 h bei Raumtemperatur nach. Dann wurde mit Wasser und Methylenchlorid verdünnt. Die organische Phase wurde abgetrennt, über Natriumsulfat getrocknet und eingedampft. Anschließend chromatographierte man mit Cyclohexan/Essigester 1:1 über Kieselgel 60, 40-63 µm.
Ausbeute: 3,82 mg = 43,4 % der Theorie
Rf : 0,121 (Cyclohexan:Essigester 1:1)

Beispiel 50

2-Dipropylamino-8-[2-N-(4-N-saccharinyl)n-butylaminoethyl]-1,2,3,4-tetrahydronaphthalin Hydrochlorid

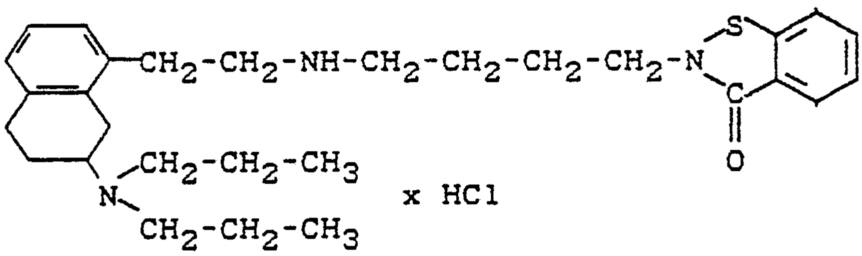

1,3 g (4,7 mmol) 2-Dipropylamino-8-(2-amino-ethyl)-1,2,3,4-tetrahydronaphthalin und 1,38 g (10 mmol) Kaliumcarbonat wurden in 10 ml Dimethylformamid vorgelegt. Man tropfte eine Lösung aus 1,49 g (4,7 mmol) 4-N-Saccharinyl-n-butylbromid und 5 ml Dimethylformamid zu und rührte 5 h bei 80° C nach. Anschließend wurde auf Wasser gegossen und mit Essigester extrahiert. Dann wurde zweimal mit Essigester und einmal mit Cyclohexan:Essigester:Triethylamin 50:49:1 über Kieselgel, 40-63 µm chromatographiert. Das zähe Öl wurde in Ether gelöst, mit etherischer Salzsäure versetzt, eingedampft und bei 50° C im Hochvakuum getrocknet.
Ausbeute: 300 mg = 11,6 % der Theorie
$R_f$ : 0,612 (Methanol:Triethylamin 95 + 5)

Anwendungsbeispiele

Beispiel 51

A.) Affinität zum 5-HT$_1$-Rezeptor

In Tabelle 1 wird beispielhaft die hohe Affinität der erfindungsgemäßen Verbindungen zu 5-Hydroxytryptamin-Rezeptoren vom Subtyp 1 dargestellt. Bei den angegebenen Werten handelt es sich um Daten, die aus Rezeptorbindungsstudien mit Kalbs-Hippocampus-Membranpräparationen ermittelt wurden. Als radioaktiv markierter Ligand wurde hierzu $^3$H-Serotonin verwendet.

Tabelle 1

| Verbindung des Beispiels-Nr. | Ki (nmol/l) |
|---|---|
| 1 | 20 |
| 2 | 13 |
| 6 | 7 |
| 25 | 13 |
| 26 | 17 |
| 28 | 4 |
| 30 | 30 |
| 33 | 19 |
| 42 | 5 |

Vergleich

In diesem Testmodell zeigen die in EP-A1-41 488 beschriebenen Verbindungen $R^1 = OCH_3$, $R^2 = R^3 = nC_3H_7$ und $R^1 = OH$, $R^2 = R^3 = nC_3H_7$ Ki-Werte von 3 bzw. 2 nmol/l

B.) Untersuchungen auf Serotonin-agonistische -antagonistische Wirkung.

Hierzu wird die Wirkung auf die Serotonin-vermittelte Kontraktion der arteria basilaris des Hundes untersucht [vgl. Peroutka et al., Brain Research 259, 327 (1983)].

Tabelle 2

| Verbindung des Beispiels-Nr. | Effekt antagonist. |
|---|---|
| 1 | - |
| 2 | - |
| 25 | + |
| 28 | - |
| 30 | + |
| 33 | + |

Vergleich aus EP-A1-41 488:

| | |
|---|---|
| $R^1$ =OH, $R^2 = nC_3H_7$, $R^3 = nC_3H_7$ | - |

## Ansprüche

1. 8-substituierte 2-Aminotetraline der allgemeinen Formel

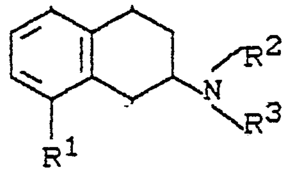

(I)

in welcher
$R^1$ -für Halogen, Cyano oder
-für eine Gruppe der Formel
$-NR^4R^5$, $-COR^6$, $-(CH_2)_a-X$, $-O-(CH_2)_a-X$ oder $-CH=CH-(CH_2)_b-X$ steht,
worin
$R^4$ und $R^5$ gleich oder verschieden sind und
-Wasserstoff oder
-eine Gruppe der Formel $-COR^7$ oder $-SO_2R^8$ bedeuten,
wobei
$R^7$ -für Wasserstoff steht, oder
-für eine Gruppe - $NHR^9$ steht, oder
-für Alkoxy steht, oder
-für Aryl, Aryloxy, Aralkyl, Aralkoxy oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
$R^8$ -für Cycloalkyl steht, oder
-für Alkyl steht, das durch Cyano, Halogen, Trifluormethyl, Trifluormethoxy oder Alkoxycarbonyl substituiert sein kann, oder
-für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
-für eine Gruppe - $NR^{10}R^{11}$ steht,
worin
$R^{10}$ und $R^{11}$ gleich oder verschieden sind und Wasserstoff, Alkyl, Aryl oder Aralkyl bedeuten
und
$R^9$ -für Wasserstoff steht, oder
-für Cycloalkyl steht, oder
-für gegebenenfalls durch Cyano, Halogen, Trifluormethyl oder Trifluormethoxy substituiertes Alkyl steht, oder
-für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
$R^6$ -Wasserstoff, Hydroxy, Amino, Alkoxy, Aryloxy oder Aralkoxy bedeutet,
a - eine Zahl 1 bis 10 bedeutet,
b - eine Zahl 0 bis 8 bedeutet
und
X - eine Gruppe der Formel $-NR^{12}R^{13}$, $-COR^{14}$, $-SO_2R^{15}$ oder $-OR^{16}$ bedeutet,
worin
$R^{12}$ und $R^{13}$ gleich oder verschieden sind und
-für Wasserstoff, Alkyl, Aryl oder Aralkyl stehen, wobei die Arylreste durch Halogen, Cyano, Alkyl, Alkoxy oder Trifluormethyl substituiert sein können, oder
-für eine Gruppe der Formel $-COR^{14}$, $-SO_2R^{15}$ oder $-(CH_2)_c-NR^{12}R^{13}$ stehen,
$R^{14}$ -Wasserstoff bedeutet, oder
-eine Gruppe - $NHR^{17}$ bedeutet, oder
-Alkyl oder Alkoxy bedeutet, oder
-Aryl, Aryloxy, Aralkyl, Aralkoxy oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können.

$R^{15}$ -Cycloalkyl bedeutet, oder

-Alkyl bedeutet, das durch Cyano, Halogen, Trifluormethyl, Trifluormethoxy oder Alkoxycarbonyl substituiert sein kann, oder

-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder

-eine Gruppe $-NR^{10}R^{11}$ bedeutet,

wobei

$R^{10}$ und $R^{11}$ die oben angegebene Bedeutung haben,

$R^{16}$ -Wasserstoff, Alkyl, Aryl, Aralkyl oder eine Gruppe der Formel $-CONR^{10}R^{11}$ bedeutet

$R^{17}$ -Wasserstoff bedeutet, oder

-Cycloalkyl bedeutet, oder

-gegebenenfalls durch Cyano, Halogen, Trifluormethyl oder Trifluormethoxy substituiertes Alkyl bedeutet, oder

-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, und

c - eine Zahl 1 bis 8 bedeutet

oder wobei

$R^{12}$ und $R^{13}$ zusammen mit dem Stickstoffatom einen Ring der Reihe

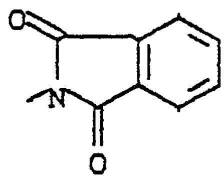 , 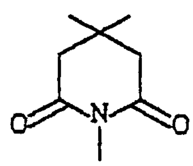 , 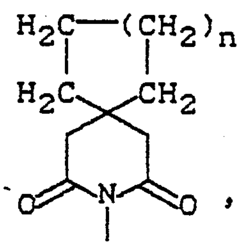 ,

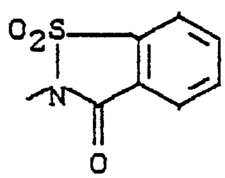 , 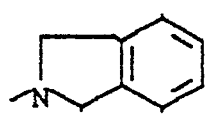 , 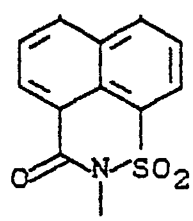 ,

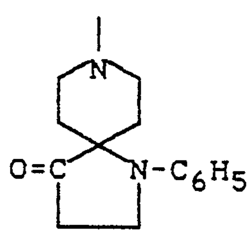 , 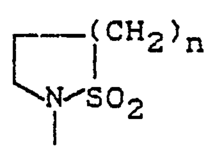 , 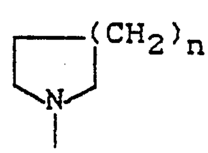

oder

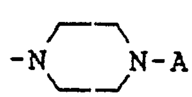

bilden,

worin

n - eine Zahl 1 oder 2 bedeutet

und

A - für Wasserstoff oder Cycloalkyl steht,

oder
-für Alkyl steht, das durch Halogen, Hydroxy, Amino, Alkylamino, Dialkylamino, Carbamoyl oder Sulfamoyl substituiert sein kann,
oder
-für Aryl, Heteroaryl, Aralkyl, Alkoxycarbonyl, Alkylsulfonyl, Phenylsulfonyl, Tolylsulfonyl, Benzylsulfonyl, Formyl, Carbamoyl oder Sulfamoyl steht,
$R^2$ -für Wasserstoff oder Alkyl steht
und
$R^3$ -für Alkyl steht,
wobei jedoch
$R^1$ nicht $NH_2$ bedeutet, wenn
$R^2$ und $R^3$ Propyl bedeuten
und deren Salze.

2. 8-substituierte 2-Aminotetraline nach Anspruch 1
worin
$R^1$ -für Fluor, Chlor, Brom oder Cyano steht, oder
-für eine Gruppe der Formel $-NR^4R^5$, $-COR^6$, $-(CH_2)_a-X$, $-O-(CH_2)_a-X$ oder $-CH=CH-(CH_2)_b-X$ steht,
worin
$R^4$ und $R^5$ gleich oder verschieden sind und
-Wasserstoff oder
-eine Gruppe der Formel - $COR^7$ oder $-SO_2R^8$ bedeuten,
wobei
$R^7$ -für Wasserstoff steht, oder
-für eine Gruppe $-NHR^9$ steht, oder
-für Niederalkoxy steht, oder
-für gegebenenfalls durch Niederalkyl, Niederalkoxy, Fluor, Chlor, Brom, Tri fluormethyl, Dimethylamino oder Diethylamino substituiertes Phenyl, Benzyl, Benzyloxy, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl steht,
$R^8$ -für Cyclopropyl, Cyclopentyl oder Cyclohexyl steht, oder
-für gegebenenfalls durch Cyano, Fluor, Chlor, Brom, Trifluormethyl oder Niederalkoxycarbonyl substituiertes Niederalkyl steht, oder
-für gegebenenfalls durch Niederalkyl, Niederalkoxy, Fluor, Chlor, Brom, Trifluormethyl, Dimethylamino oder Diethylamino substituiertes Phenyl, Benzyl, Thienyl, Furyl, Pyrimidyl, Pyridyl, Chinolyl, Isochinolyl, Benzothiazolyl, Benzoxazolyl, Thiazolyl, Oxazolyl, Isoxazolyl oder Isothiazolyl steht, oder
-für eine Gruppe der Formel $-NR^{10}R^{11}$ steht,
worin
$R^{10}$ und $R^{11}$ gleich oder verschieden sind und Wasserstoff, Niederalkyl oder Phenyl bedeuten
und
$R^9$ -für Wasserstoff steht, oder
-für gegebenenfalls durch Cyano, Fluor, Chlor oder Brom substituiertes Niederalkyl steht, oder
-für gegebenenfalls durch Niederalkyl, Niederalkoxy, Fluor, Chlor, Brom, Trifluormethyl, Dimethylamino oder Diethylamino substituiertes Phenyl, Benzyl, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl steht,
$R^6$ -Wasserstoff, Hydroxy, Amino, Niederalkoxy oder Benzyloxy bedeutet,
a - eine Zahl 1 bis 8 bedeutet,
b - eine Zahl 0 bis 6 bedeutet
und
X - eine Gruppe der Formel $-NR^{12}R^{13}$, $-COR^{14}$, $-SO_2R^{15}$ oder $-OR^{16}$ bedeutet,
worin
$R^{12}$ und $R^{13}$ gleich oder verschieden sind und
-für Wasserstoff, Niederalkyl, Phenyl oder Benzyl steht, wobei die genannten Reste durch Fluor, Chlor, Brom, Niederalkyl, Niederalkoxy oder Trifluormethyl substituiert sein können, oder
-für eine Gruppe der Formel $-COR^{14}$, $-SO_2R^{15}$ oder $-(CH_2)_c-NR^{12}R^{13}$ stehen,
$R^{14}$ -eine Gruppe $-NHR^{17}$ bedeutet, oder
-Niederalkyl oder Niederalkoxy bedeutet, oder
-gegebenenfalls durch Niederalkyl, Niederalkoxy, Fluor, Chlor, Brom, Trifluormethyl, Dimethylamino oder Diethylamino substituiertes Phenyl, Benzyl, Benzyloxy, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,

$R^{13}$ -Cyclopropyl. Cyclopentyl. Cyclohexyl, oder
-gegebenenfalls durch Cyano. Fluor. Chlor. Brom. Trifluormethyl oder Niederalkoxycarbonyl substituiertes Niederalkyl bedeutet. oder
-gegebenenfalls ein-oder mehrfach, gleich oder verschieden durch Niederalkyl. Niederalkoxy, Fluor. Chlor, Brom, Trifluormethyl, Dimethylamino oder Diethylamino substituiertes Phenyl. Naphthyl, Benzyl, Thienyl, Furyl, Pyrimidyl, Pyridyl. Chinolyl. Isochinolyl, Benzothiazolyl, Benzoxazolyl, Thiazolyl, Oxazolyl, Isoxazolyl oder Isothiazolyl bedeutet, oder
-eine Gruppe $-NR^{10}R^{11}$ bedeutet,
wobei
$R^{10}$ und $R^{11}$ die oben angegebene Bedeutung haben,
$R^{16}$ - Wasserstoff. Niederalkyl, Phenyl oder Benzyl bedeutet,
$R^{17}$ -Wasserstoff bedeutet, oder
-gegebenenfalls durch Cyano, Fluor, Chlor oder Brom substituiertes Niederalkyl bedeutet, oder
-gegebenenfalls durch Niederalkyl, Niederalkoxy, Fluor, Chlor. Brom, Trifluormethyl, Dimethylamino oder Diethylamino substituiertes Phenyl, Benzyl, Thienyl, Furyl, Pyridyl, Pyrimidyl, Chinolyl. Isochinolyl, Benzothiazolyl. Benzoxazolyl, Thiazolyl, Oxazolyl, Isoxazolyl oder Isothiazolyl bedeutet,
und
c - eine Zahl 1 bis 6 bedeutet,
oder wobei
$R^{12}$ und $R^{13}$ zusammen mit dem Stickstoffatom einen Ring der Reihe

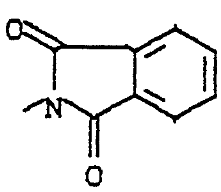 , 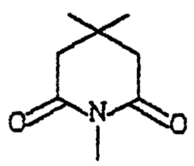 , 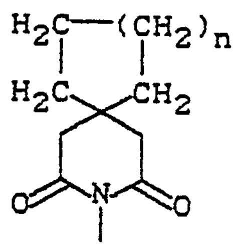 ,

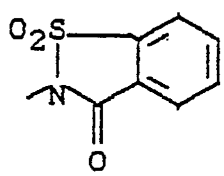 , 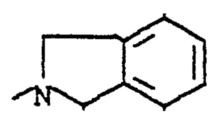 , 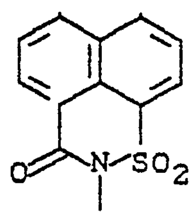 ,

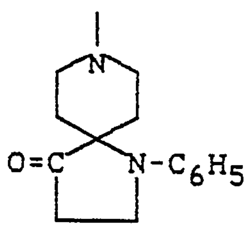 oder 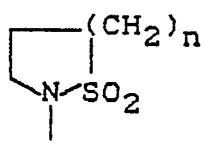

bilden,
worin
n - eine Zahl 1 oder 2 bedeutet,
$R^2$ -für Wasserstoff oder Niederalkyl steht
und
$R^3$ -für Niederalkyl steht,
wobei jedoch
$R^1$ -nicht für $NH_2$ steht, wenn
$R^2$ und $R^3$ Propyl bedeuten
und deren Salze.

3. 8-substituierte 2-Aminotetraline nach den Ansprüchen 1 und 2
worin
$R^1$ -für Chlor, Brom, Cyano oder
-für eine Gruppe der Formel $-NR^4R^5$, $-COR^6$, $-(CH_2)_a-X$, $-O-(CH_2)_a-X$ oder $-CH=CH-(CH_2)_b-X$ steht,

worin
$R^4$ und $R^5$ gleich oder verschieden sind und
-Wasserstoff oder
-eine Gruppe der Formel $-COR^7$ oder $-SO_2R^8$ bedeuten,
worin
$R^7$ -für Wasserstoff steht, oder
-für eine Gruppe $-NHR^9$ steht, oder
-für Methoxy, Ethoxy, Propoxy oder Isopropoxy steht, oder
-für gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl steht,
$R^8$ -für gegebenfalls durch Fluor, Chlor, Methoxycarbonyl, Ethoxycarbonyl, Propoxycarbonyl, Isopropoxycarbonyl, Butoxycarbonyl oder Isobutoxycarbonyl substituiertes Ethyl, Propyl, Isopropyl, Butyl oder Isobutyl steht, oder
- für gegebenenfalls durch Methyl, Ethyl, Propyl, Isopropyl, Methoxy, Ethoxy, Pro poxy oder Isopropoxy, Fluor oder Chlor substituiertes Phenyl, Thienyl, Furyl, Pyridyl, Pyrimidyl, Chinolyl oder Isochinolyl steht, oder
- für eine Gruppe $-NR^{10}R^{11}$ steht,
worin
$R^{10}$ und $R^{11}$ gleich oder verschieden sind und Wasserstoff, Methyl, Ethyl, Propyl, Isopropyl, Butyl oder Isobutyl bedeuten
und
$R^9$ -für Wasserstoff steht, oder
-für gegebenenfalls durch Fluor oder Chlor substituiertes methyl, Ethyl, Propyl, Isopropyl, Butyl, Isobutyl, Pentyl, Isopentyl, Hexyl oder Isohexyl steht, oder
-für Phenyl steht, das durch Fluor, Chlor, Methyl oder Methoxy substituiert sein kann,
$R^6$ -Wasserstoff, Hydroxy, Amino, Methoxy, Ethoxy, Propoxy, Butoxy, Isopropoxy oder Isobutoxy bedeutet,
a - eine Zahl 1 bis 6 bedeutet,
b - eine Zahl 0 bis 4 bedeutet
und
X - eine Gruppe der Formel $-NR^{12}R^{13}$, $-COR^{14}$, $-SO_2R^{15}$ oder $-OR^{16}$ bedeutet,
wobei
$R^{12}$ und $R^{13}$ gleich oder verschieden sind, und
-für Wasserstoff, Methyl, Ethyl, Propyl, Isopropyl, Butyl, Isobutyl, oder
-für gegebenenfalls durch Fluor, Chlor, Methyl oder Methoxy subsituiertes Phenyl, oder
-für eine Gruppe $-COR^{14}$, $-SO_2R^{15}$ oder

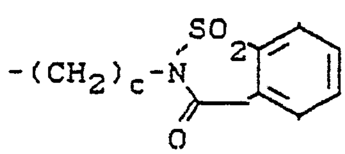

stehen,
$R^{14}$ -Wasserstoff bedeutet, oder
-eine Gruppe $-NHR^{17}$ bedeutet, oder
-Methyl, Ethyl, Propyl, Isopropyl, Methoxy, Ethoxy, Propoxy, Isopropoxy, oder
-gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl bedeutet,
$R^{15}$ -gegebenenfalls durch Fluor, Chlor, Methoxycarbonyl, Ethoxycarbonyl, Propoxycarbonyl, Isopropoxycarbonyl, Butoxycarbonyl oder Isobutoxycarbonyl substituiertes Methyl, Ethyl, Propyl, Isopropyl, Butyl oder Isobutyl bedeutet, oder
-gegebenenfalls ein-oder mehrfach, gleich oder verschieden durch Methyl, Ethyl, Propyl, Isopropyl, Methoxy, Fluor oder Chlor substituiertes Phenyl, Naphthyl, Thienyl, Furyl, Pyridyl, Pyrimidyl, Chinolyl oder Isochinolyl bedeutet, oder
-eine Gruppe $-NR^{10}R^{11}$ bedeutet,
wobei
$R^{10}$ und $R^{11}$ die oben angegebene Bedeutung haben,
$R^{16}$ -Wasserstoff, Methyl, Ethyl, Propyl, Isopropyl, Phenyl oder Benzyl bedeutet,

$R^{17}$ -Wasserstoff bedeutet, oder
-gegebenenfalls durch Fluor oder Chlor substituiertes Methyl, Ethyl, Propyl, Isopropyl, Butyl, Isobutyl, Pentyl, Isopentyl, Hexyl oder Isohexyl bedeutet, oder
-Phenyl bedeutet, das durch Fluor, Chlor, Methyl oder Methoxy substituiert sein kann,
und
n - eine Zahl 1 bis 4 bedeutet,
$R^2$ -für Wasserstoff, Methyl, Ethyl, Propyl oder Isopropyl steht
und
$R^3$ -für Methyl, Ethyl, Propyl oder Isopropyl steht,
wobei jedoch
$R^1$ -nicht $NH_2$ bedeutet, wenn
$R^2$ und $R^3$ Propyl bedeuten,
und deren Salze.

4. 8-substituierte 2-Aminotetraline nach den Ansprüchen 1, 2 und 3
worin
$R^1$ -für Chlor, Brom, Cyano oder
-für eine Gruppe der Formel $-NR^4R^5$, $-COR^6$, $-(CH_2)_a-X$, $-O-(CH_2)_a-X$ oder $-CH=CH-(CH_2)_b-X$ steht,
worin
$R^4$ -Wasserstoff bedeutet,
$R^5$ -eine Gruppe der Formel $-COR^7$ oder $-SO_2R^8$ bedeuten,
worin
$R^7$ -für Wasserstoff steht, oder
-für eine Gruppe $-NHR^9$ steht, oder
-für Methoxy oder Ethoxy steht,
$R^8$ -für Trifluormethyl, Phenyl, Tolyl steht, oder
-für eine Gruppe $-NR^{10}R^{11}$ steht,
worin
$R^{10}$ und $R^{11}$ gleich oder verschieden sind und Wasserstoff, Methyl oder Ethyl bedeuten,
und
$R^9$ -für Wasserstoff steht, oder
-für Methyl, Ethyl, Propyl, Isopropyl oder Butyl, oder
-für Phenyl steht,
$R^6$ -Wasserstoff, Hydroxy, Amino, Methoxy oder Ethoxy bedeutet,
a - eine Zahl 1 bis 4 bedeutet,
b - eine Zahl 0 bis 2 bedeutet
und
X - eine Gruppe der Formel $-NR^{12}R^{13}$, $-COR^{14}$, $-SO_2R^{15}$ oder $-OR^{16}$ bedeutet,
wobei
$R^{12}$ und $R^{13}$ gleich oder verschieden sind, und - für Wasserstoff, Methyl, Ethyl, Propyl, oder
-für eine Gruppe $-COR^{14}$, $-SO_2R^{15}$ oder

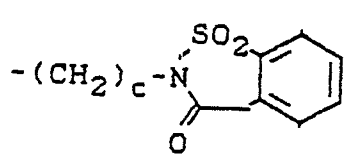

stehen,
$R^{14}$ -Wasserstoff bedeutet, oder
-eine Gruppe $-NHR^{17}$ bedeutet, oder
-Methyl, Ethyl, Propyl, Methoxy oder Ethoxy bedeutet,
$R^{15}$ - Trifluormethyl, Methyl, Ethyl, Propyl, Isopropyl, Butyl oder Isobutyl bedeutet, oder
-gegebenenfalls durch ein oder mehrere Methyl oder Chlor substituiertes Phenyl, oder Naphthyl bedeutet,
-eine Gruppe $-NR^{10}R^{11}$ bedeutet,
wobei
$R^{10}$ und $R^{11}$ die oben angegebene Bedeutung haben,
$R^{16}$ -Wasserstoff, Methyl, Ethyl oder Propyl, bedeutet,
$R^{17}$ -Wasserstoff bedeutet, oder

-Methyl, Ethyl, Propyl, isopropyl, Butyl oder Isobutyl bedeutet, oder
-Phenyl bedeutet
und
c - eine Zahl 2 bis 4 bedeutet,
und
$R^2$ und $R^3$ für Propyl stehen
und deren Salze.

5. 8-substituierte 2-Aminotetraline der Formel (I)

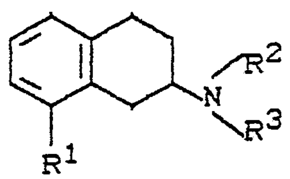

(I)

in welcher
$R^1$ -für Halogen, Cyano oder
-für eine Gruppe der Formel
$-NR^4R^5$, $-COR^6$, $-(CH_2)_a-X$, $-O-(CH_2)_a-X$ oder $-CH=CH-(CH_2)_b-X$ steht,
worin
$R^4$ und $R^5$ gleich oder verschieden sind und
- Wasserstoff oder
-eine Gruppe der Formel $-COR^7$ oder $-SO_2R^8$ bedeuten,
wobei
$R^7$ -für Wasserstoff steht, oder
-für eine Gruppe $-NHR^9$ steht, oder
-für Alkoxy steht, oder
-für Aryl, Aryloxy, Aralkyl, Aralkoxy oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
$R^8$ -für Cycloalkyl steht, oder
-für Alkyl steht, das durch Cyano, Halogen, Trifluormethyl, Trifluormethoxy oder Alkoxycarbonyl substituiert sein kann, oder
-für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
-für eine Gruppe $-NR^{10}R^{11}$ steht,
worin
$R^{10}$ und $R^{11}$ gleich oder verschieden sind und Wasserstoff, Alkyl, Aryl oder Aralkyl bedeuten
und
$R^9$ -für Wasserstoff steht, oder
-für Cycloalkyl steht, oder
-für gegebenenfalls durch Cyano, Halogen, Trifluormethyl oder Trifluormethoxy substituiertes Alkyl steht, oder
-für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
$R^6$ -Wasserstoff, Hydroxy, Amino, Alkoxy, Aryloxy oder Aralkoxy bedeutet,
a - eine Zahl 1 bis 10 bedeutet,
b - eine Zahl 0 bis 8 bedeutet
und
X - eine Gruppe der Formel $-NR^{12}R^{13}$, $-COR^{14}$, $-SO_2R^{15}$ oder $-OR^{16}$ bedeutet,
worin
$R^{12}$ und $R^{13}$ gleich oder verschieden sind und
-für Wasserstoff, Alkyl, Aryl oder Aralkyl stehen, wobei die Arylreste durch Halogen, Cyano, Alkyl, Alkoxy oder Trifluormethyl substituiert sein können, oder
-für eine Gruppe der Formel $-COR^{14}$, $-SO_2R^{15}$ oder $-(CH_2)_c-NR^{12}R^{13}$ stehen,

$R^{14}$ -Wasserstoff bedeutet, oder
-eine Gruppe $-NHR^{17}$ bedeutet, oder
-Alkyl oder Alkoxy bedeutet, oder
-Aryl, Aryloxy, Aralkyl, Aralkoxy oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
$R^{15}$ -Cycloalkyl bedeutet, oder
-Alkyl bedeutet, das durch Cyano, Halogen, Trifluormethyl, Trifluormethoxy oder Alkoxycarbonyl substituiert sein kann, oder
-Aryl, Aralkyl oder Heteroaryl bedeutet, wobei die genannten Reste bis zu 3-fach gleich oder verschieden durch Alkyl, Alkoxy, Alkylthio, Halogen, Cyano, Tri fluormethyl, Trifluormethoxy, Trifluormethylthio, Amino, Alkylamino oder Dialkylamino substituiert sein können, oder
-eine Gruppe $-NR^{10}R^{11}$ bedeutet,
wobei
$R^{10}$ und $R^{11}$ die oben angegebene Bedeutung haben,
$R^{16}$ - Wasserstoff, Alkyl, Aryl, Aralkyl oder eine Gruppe der Formel $-CONR^{10}R^{11}$ bedeutet,
$R^{17}$ -Wasserstoff bedeutet, oder
-Cycloalkyl bedeutet, oder
-gegebenenfalls durch Cyano, Halogen, Trifluormethyl oder Trifluormethoxy substituiertes Alkyl bedeutet, oder
-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
und
c - eine zahl 1 bis 8 bedeutet,
oder wobei
$R^{12}$ und $R^{13}$ zusammen mit dem Stickstoffatom einen Ring der Reihe

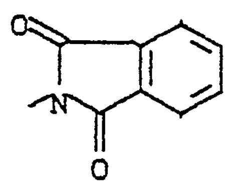 ,
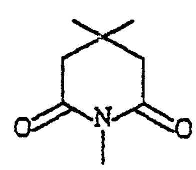 ,
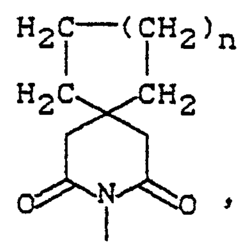 ,

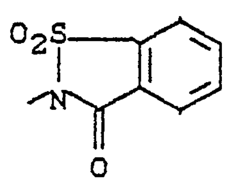 ,
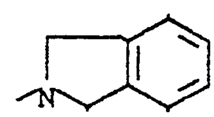 ,
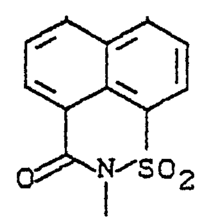 ,

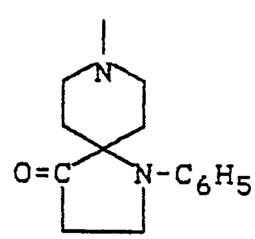 ,
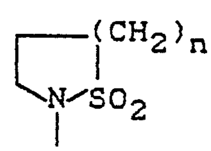 ,
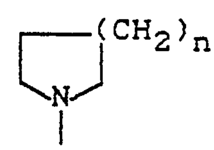

oder

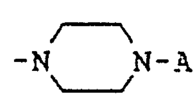

bilden.
worin
n - eine Zahl 1 oder 2 bedeutet
und
A - für Wasserstoff oder Cycloalkyl steht,
oder
-für Alkyl steht, das durch Halogen, Hydroxy, Amino, Alkylamino, Dialkylamino, Carbamoyl oder Sulfamoyl substituiert sein kann,
oder
-für Aryl, Heteroaryl, Aralkyl, Alkoxycarbonyl, Alkylsulfonyl, Phenylsulfonyl, Tolylsulfonyl, Benzylsulfonyl, Formyl, Carbamoyl oder Sulfamoyl steht,
$R^2$ -für Wasserstoff oder Alkyl steht
und
$R^3$ -für Alkyl steht,
wobei jedoch
$R^1$ nicht $NH_2$ bedeutet, wenn
$R^2$ und $R^3$ Propyl bedeuten
und deren Salze zur therapeutischen Anwendung.

6. Verfahren zur Herstellung von 8-Halogen-aminotetralinen der allgemeinen Formel

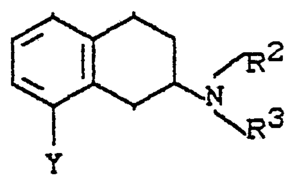

(Ia)

in welcher
Y - für Halogen oder Cyano steht,
$R^2$ -für Wasserstoff oder Alkyl steht,
und
$R^3$ -für Alkyl steht,
und deren Salze,
dadurch gekennzeichnet, daß man
[A] 8-Aminotetraline der allgemeinen Formel

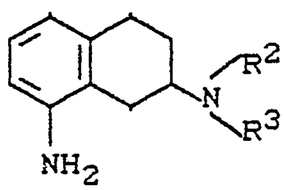

(II),

in welcher
$R^2$ und $R^3$ die angegebene Bedeutung haben,
in inerten Lösemitteln in Anwesenheit von Säuren mit Nitriten umsetzt,
dann die erhaltenen Diazoniumsalze mit Kupfersalzen der allgemeinen Formel
CuY (III),
in welcher
Y die angegebene Bedeutung hat,
gegebenenfalls in Anwesenheit von Hilfsstoffen umsetzt
und im Fall der Herstellung der Salze mit den entsprechenden Säuren umsetzt,
oder, daß man
[B] Tetralone der allgemeinen Formel

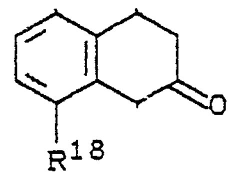

(IV),

in welcher
$R^{18}$ -für Chlor oder Brom steht,
zunächst mit Aminen der allgemeinen Formel

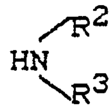

(V),

in welcher
$R^2$ und $R^3$ die angegebene Bedeutung haben,
in inerten Lösemitteln, gegebenenfalls in Anwesenheit von Hilfsstoffen umsetzt,
dann die Zwischenverbindungen in inerten Lösemitteln reduziert,
dann gegebenenfalls Halogen gegen Cyano austauscht
und dann im Fall der Herstellung der Salze mit den entsprechenden Säuren umsetzt.

7. Verfahren zur Herstellung von Diaminotetralinen der allgemeinen Formel

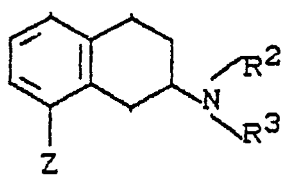

(Ib),

worin
Z - für eine Gruppe der Formel $-NR^4R^5$ steht,
worin
$R^4$ und $R^5$ gleich oder verschieden sind und
-für Wasserstoff, oder
-eine Gruppe der Formel $-COR^{7'}$ oder $-SO_2R^8$ bedeuten,
wobei
$R^{7'}$ -für Wasserstoff steht, oder
-für Alkoxy steht, oder
-für Aryl, Aryloxy, Aralkyl, Aralkoxy oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können
und
$R^8$ -für Cycloalkyl steht, oder
-für Alkyl steht, das durch Cyano, Halogen, Trifluormethyl, Trifluormethoxy oder Alkoxycarbonyl substituiert sein kann, oder
-für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
-für eine Gruppe $-NR^{10}R^{11}$ steht,
worin
$R^{10}$ und $R^{11}$ gleich oder verschieden sind und Wasserstoff, Alkyl, Aryl oder Aralkyl bedeuten,
$R^2$ -für Wasserstoff oder Alkyl steht,
und
$R^3$ -für Alkyl steht,
wobei jedoch
$R^1$ nicht $NH_2$ bedeutet, wenn
$R^2$ und $R^3$ Propyl bedeuten,

und deren Salze.
dadurch gekennzeichnet, daß man
8-Aminotetraline der allgemeinen Formel

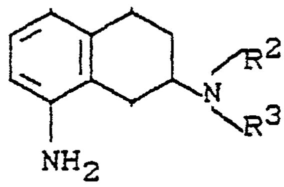

(II)

in welcher
$R^2$ und $R^3$ die oben angegebene Bedeutung haben.
mit Acylierungs-bzw. Sulfonierungsmitteln der allgemeinen Formel
V-$R^{19}$ (VI)
in welcher
$R^{19}$ für eine Gruppe der Formel $-COR^7$ oder $-SO_2R^8$ steht,
und
V-für Halogen, oder
-für den Rest $-OR^{20}$ steht,
worin
$R^{20}$ die gleiche Bedeutung hat wie $R^{19}$ und mit diesem gleich oder verschieden sein kann,
in inerten Lösemitteln gegebenenfalls in Anwesenheit von Basen umsetzt
und dann im Fall der Herstellung der Salze mit den entsprechenden Säuren umsetzt.

8. Verfahren zur Herstellung von 8-Ureido-aminotetraline der allgemeinen Formel

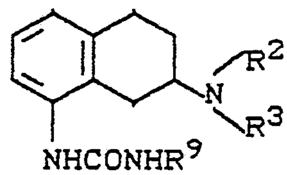

(Ic)

in welcher
$R^2$ -für Wasserstoff oder Alkyl steht,
$R^3$ -für Alkyl steht
und
$R^9$ -für Wasserstoff steht, oder
-für Cycloalkyl steht, oder
- für gegebenenfalls durch Cyano, Halogen, Trifluormethyl oder Trifluormethoxy substituiertes Alkyl steht, oder
-für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
und deren Salze,
dadurch gekennzeichnet, daß man
8-Aminotetraline der allgemeinen Formel

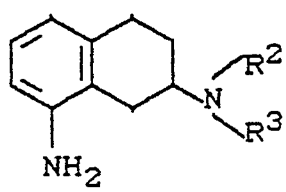

(II)

in welcher
$R^2$ und $R^3$ die angegebene Bedeutung haben,
in inerten Lösemitteln gegebenenfalls in Anwesenheit von Base mit Isocyanaten der allgemeinen Formel

$R^9N = C = O$ (VII)
in welcher
$R^9$ -die angegebene Bedeutung hat,
umsetzt.
und dann im Fall der Herstellung der Salze mit den entsprechenden Säuren umsetzt.

9. Verfahren zur Herstellung von 8-Acyl-aminotetralinen der allgemeinen Formel

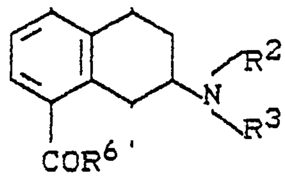

(Id),

in welcher
$R^2$ -für Wasserstoff oder Alkyl steht,
$R^3$ -für Alkyl steht
und
$R^{6'}$ für Hydroxy, Amino, Alkoxy, Aryloxy oder Aralkoxy steht,
und deren Salze,
dadurch gekennzeichnet, daß man
8-Cyanotetraline der allgemeinen Formel

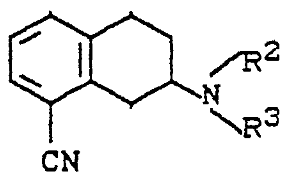

(VIII)

in welcher
$R^2$ und $R^3$ die oben angegebenen Bedeutung haben hydrolysiert,
im Fall der Herstellung der Carbonsäureester die erhaltenen Carbonsäuren verestert
und im Fall der Herstellung der Salze mit den entsprechenden Säuren umsetzt.

10. Verfahren zur Herstellung von 8-Formyl-aminotetraline der allgemeinen Formel

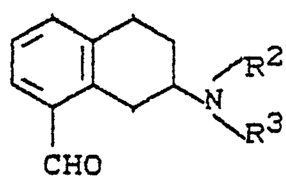

(Ie)

in welcher
$R^2$ -für Wasserstoff oder Alkyl steht,
und
$R^3$ -für Alkyl steht,
und deren Salze,
dadurch gekennzeichnet, daß man
8-Halogen-substituierte 2-Aminotetraline der allgemeinen Formel

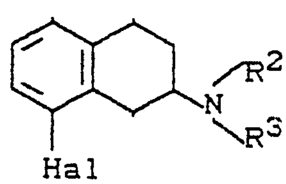

(IX)

in welcher
$R^2$ und $R^3$ die oben angegebene Bedeutung haben
und
Hal für Fluor, Chlor, Brom, Iod, bevorzugt für Chlor oder Brom steht,
in inerten Lösemitteln, gegebenenfalls in Anwesenheit von Hilfsstoffen mit Magnesium und Formamiden der allgemeinen Formel

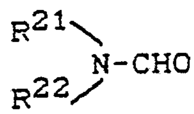

(X)

in welcher
$R^{21}$ und $R^{22}$ gleich oder verschieden sind und
-für Methyl, Ethyl, Propyl, Phenyl oder Pyridyl stehen, oder
$R^{21}$ und $R^{22}$ gemeinsam mit dem Stickstoffatom einen Piperidinring bilden,
umsetzt
und dann im Fall der Herstellung der Salze mit den entsprechenden Säuren umsetzt.

11. Verfahren zur Herstellung von 8-Methylen-aminotetraline der allgemeinen Formel

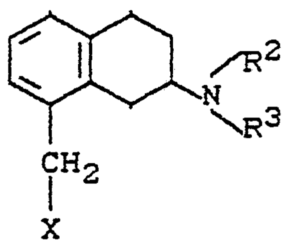

(If)

in welcher
$R^2$ -für Wasserstoff oder Alkyl steht,
$R^3$ -für Alkyl steht
und
X - eine Gruppe der Formel $-NR^{12}R^{13}$, $-COR^{14}$, $-SO_2R^{15}$ oder $-OR^{16}$ bedeutet,
worin
$R^{12}$ und $R^{13}$ gleich oder verschieden sind und
-für Wasserstoff, Alkyl, Aryl oder Aralkyl stehen, wobei die Arylreste durch Halogen, Cyano, Alkyl, Alkoxy der Trifluormethyl substituiert sein können, oder
-für eine Gruppe der Formel $-COR^{14}$, $-SO_2R^{15}$ oder $-(CH_2)_c-NR^{12}R^{13}$ stehen,
$R^{14}$ -Wasserstoff bedeutet, oder
-eine Gruppe $-NHR^{17}$ bedeutet, oder
-Alkyl oder Alkoxy bedeutet, oder
-Aryl, Aryloxy, Aralkyl, Aralkoxy oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
$R^{15}$ -Cycloalkyl bedeutet, oder
-Alkyl bedeutet, das durch Cyano, Halogen, Trifluormethyl, Trifluormethoxy oder Alkoxycarbonyl substituiert sein kann, oder
-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
-eine Gruppe $-NR^{10}R^{11}$ bedeutet,
wobei
$R^{10}$ und $R^{11}$ gleich oder verschieden sind und
-für Wasserstoff, Alkyl, Aryl oder Aralkyl stehen,
$R^{16}$ -Wasserstoff, Alkyl, Aryl, Aralkyl oder eine Gruppe $-CONR^{10}R^{11}$ bedeutet,
$R^{17}$ -Wasserstoff bedeutet, oder
-Cycloalkyl bedeutet, oder
-gegebenenfalls durch Cyano, Halogen, Trifluormethyl oder Trifluormethoxy substituiertes Alkyl bedeutet,

oder
-Aryl, Aralkyl oder Heteroaryl bedeutet, wobei die Arylrest bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
und
n eine Zahl 1 bis 8 bedeutet,
oder wobei
$R^{12}$ und $R^{13}$ zusammen mit dem Stickstoffatom einen Ring der Reihe

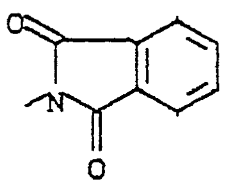 , 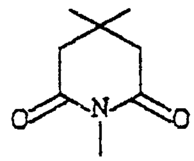 , 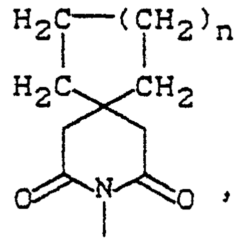 ,

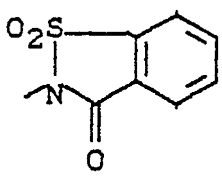 , 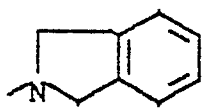 , 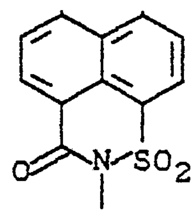 ,

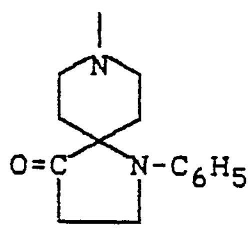 , 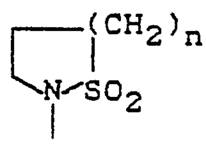 , 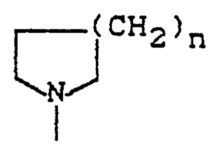

oder

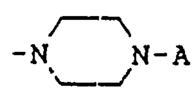

bilden,
worin
n - eine Zahl 1 oder 2 bedeutet,
und
A - für Wasserstoff oder Cycloalkyl steht,
oder
-für Alkyl steht, das durch Halogen, Hydroxy, Amino, Alkylamino, Dialkylamino, Carbamoyl oder Sulfamoyl substituiert sein kann,
oder
-für Aryl, Heteroaryl, Aralkyl, Alkoxycarbonyl, Alkylsulfonyl, Phenylsulfonyl, Tolylsulfonyl, Benzylsulfonyl, Formyl, Carbamoyl oder Sulfamoyl steht,
$R^2$ -für Wasserstoff oder Alkyl steht,
und
$R^3$ -für Alkyl steht,
wobei jedoch

$R^1$ nicht $NH_2$ bedeutet, wenn
$R^2$ und $R^3$ Propyl bedeuten,
und deren Salze,
dadurch gekennzeichnet, daß man
Tetraline der allgemeinen Formel

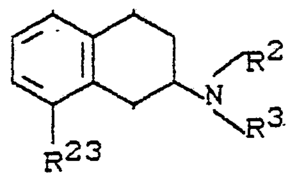

(XI)

in welcher
$R^2$ -für Wasserstoff oder Alkyl steht,
$R^3$ -für Alkyl steht
und
$R^{23}$ für eine Gruppe der Formel $-COR^{24}$ oder -CN steht,
worin
$R^{24}$ - Wasserstoff, Hydroxy, Alkoxy oder Amino bedeutet,
in inerten Lösemitteln reduziert,
dann gegebenenfalls funktionelle Gruppen durch Reduktion, Hydrolyse, Oxidation oder Umsetzung mit elektrophilen Reagenzien in andere funktionelle Gruppen überführt,
und dann im Fall der Herstellung der Salze mit den entsprechenden Säuren umsetzt.

12. Verfahren zur Herstellung von 8-Alkylen-aminotetralinen der allgemeinen Formel

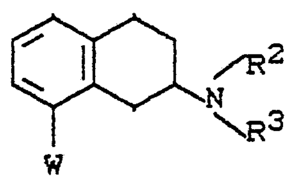

(Ig)

in welcher
$R^2$ -für Wasserstoff oder Alkyl steht,
$R^3$ -für Alkyl steht
und
W-für eine gruppe der Formel $-(CH_2)_{a'}-X$ oder $-CH=CH-(CH_2)_b-X$ steht,
worin
X - eine Gruppe der Formel $-NR^{12}R^{13}$, $-COR^{14}$, $-SO_2R^{15}$ oder $-OR^{16}$ bedeutet,
worin
$R^{12}$ und $R^{13}$ gleich oder verschieden sind und
-für Wasserstoff, Alkyl, Aryl oder Aralkyl stehen, wobei die Arylreste durch Halogen, Cyano, Alkyl, Alkoxy oder Trifluormethyl substituiert sein können, oder
-für eine Gruppe der Formel $-COR^{14}$, $-SO_2R^{15}$ oder $-(CH_2)_c-NR^{12}R^{13}$ stehen,
$R^{14}$ -Wasserstoff bedeutet, oder
-eine Gruppe $-NHR^{17}$ bedeutet, oder
-Alkyl oder Alkoxy bedeutet, oder
-Aryl, Aryloxy, Aralkyl, Aralkoxy oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
$R^{15}$ -Cycloalkyl bedeutet, oder
-Alkyl bedeutet, das durch Cyano, Halogen, Trifluormethyl, Trifluormethoxy oder Alkoxycarbonyl substituiert sein kann, oder
-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
-eine Gruppe $-NR^{10}R^{11}$ bedeutet,

wobei

$R^{14}$ und $R^{15}$ die oben angegebene Bedeutung haben.

$R^{16}$ - Wasserstoff. Alkyl. Aryl. Aralkyl oder eine Gruppe der Formel $-CONR^{14}R^{15}$ bedeutet,

$R^{17}$ -Wasserstoff bedeutet. oder

-Cycloalkyl bedeutet. oder

-gegebenenfalls durch Cyano. Halogen. Trifluormethyl oder Trifluormethoxy substituiertes Alkyl bedeutet, oder

-Aryl. Aralkyl oder Heteroaryl bedeutet, wobei die genannten Reste bis zu 3-fach gleich oder verschieden durch Alkyl. Alkoxy. Alkylthio, Halogen, Cyano. Trifluormethyl, Trifluormethoxy. Trifluormethylthio. Amino, Alkylamino oder Dialkylamino substituiert sein können,

und

c - eine Zahl 1 bis 8 bedeutet,

oder wobei

$R^{12}$ und $R^{13}$ zusammen mit dem Stickstoffatom einen Ring der Reihe

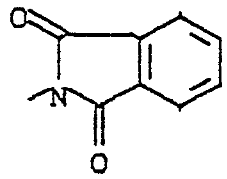 , 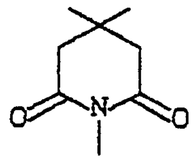 , 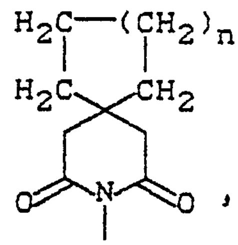 ,

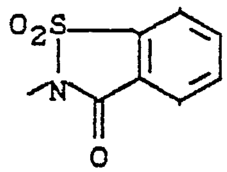 , 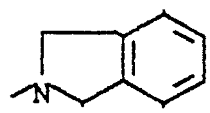 , 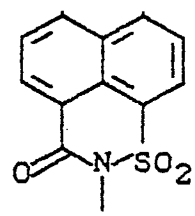 ,

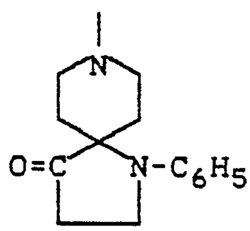 , 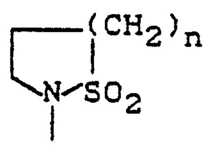 , 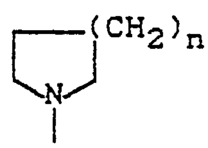

oder

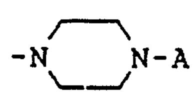

bilden,

worin

n - eine Zahl 1 oder 2 bedeutet,

und

A - für Wasserstoff oder Cycloalkyl steht,

oder

-für Alkyl steht, das durch Halogen, Hydroxy, Amino, Alkylamino, Dialkylamino, Carbamoyl oder Sulfamoyl substituiert sein kann,

oder

-für Aryl, Heteroaryl, Aralkyl, Alkoxycarbonyl, Alkylsulfonyl, Phenylsulfonyl, Tolylsulfonyl, Benzylsulfonyl, Formyl, Carbamoyl oder Sulfamoyl steht,

a' -eine Zahl 2 bis 10 bedeutet

und

b - eine Zahl 0 bis 8 bedeutet

und deren Salze, dadurch gekennzeichnet, daß man 8-Formyl-aminotetraline der allgemeinen Formel

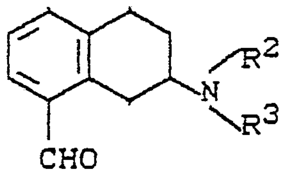

(Ie)

in welcher

$R^2$ und $R^3$ die angegebenen Bedeutung haben,

in inerten Lösemitteln in Anwesenheit von Basen mit Phosphorverbindungen der allgemeinen Formel

$U\text{-}(CH_2)_{a'}\text{-}1\text{-}X'$ (XII)

in welcher

X' -die für X angegebene Bedeutung hat, oder

-für Nitro steht,

a' -die oben angegebene Bedeutung hat

und

U - für eine Gruppe der Formel

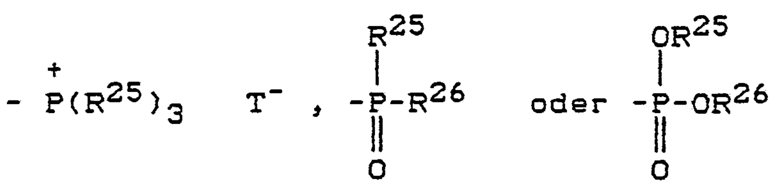

steht,

wobei

$R^{25}$ und $R^{26}$ gleich oder verschieden sind und Alkyl oder Phenyl bedeuten

und

T - ein Halogenidanion, bevorzugt Chlorid, Bromid oder Iodid bedeutet,

umsetzt,

dann gegebenenfalls funktionelle Gruppen durch Reduktion, Hydrolyse, Oxidation oder Umsetzung mit elektrophilen Reagenzien in andere funktionelle Gruppen überführt

und dann im Fall der Herstellung der Salze mit den entsprechenden Säuren umsetzt.

13. Verfahren zur Herstellung von 8-Ethylen-aminotetralinen der allgemeinen Formel

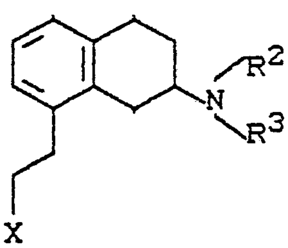

(Ih)

in welcher

$R^2$ -für Wasserstoff oder Alkyl steht,

$R^3$ -für Wasserstoff oder Alkyl steht

und

X - eine Gruppe der Formel $-NR'^{2}R'^{3}$, $-COR'^{4}$, $-SO_2R'^{5}$ oder $-OR'^{6}$ bedeutet,

worin

$R'^{2}$ und $R'^{3}$ gleich oder verschieden sind und

-für Wasserstoff, Alkyl, Aryl oder Aralkyl stehen, wobei die Arylreste durch Halogen, Cyano, Alkyl, Alkoxy oder Trifluormethyl substituiert sein können,

oder

-für eine Gruppe der Formel $-COR'^{4}$, $-SO_2R'^{5}$ oder $-(CH_2)_c-NR'^{2}R'^{3}$ stehen,

$R^{14}$ -Wasserstoff bedeutet, oder
-eine Gruppe $-NHR^{17}$ bedeutet, oder
- Alkyl oder Alkoxy bedeutet, oder
-Aryl, Aryloxy, Aralkyl, Aralkoxy oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
$R^{15}$ -Cycloalkyl bedeutet, oder
-Alkyl bedeutet, das durch Cyano, Halogen, Trifluormethyl, Trifluormethoxy oder Alkoxycarbonyl substituiert sein kann, oder
-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
-eine Gruppe $-NR^{12}R^{13}$ bedeutet,
wobei
$R^{12}$ und $R^{13}$ die oben angegebene Bedeutung haben,
$R^{16}$ -Wasserstoff, Alkyl, Aryl, Aralkyl oder eine Gruppe $-CONR^{12}R^{13}$ bedeutet,
$R^{17}$ -Wasserstoff bedeutet, oder
-Cycloalkyl bedeutet, oder
-gegebenenfalls durch Cyano, Halogen, Trifluormethyl oder Trifluormethoxy substituiertes Alkyl bedeutet, oder
-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
und
c - eine Zahl 1 bis 8 bedeutet,
oder wobei
$R^{12}$ und $R^{13}$ zusammen mit dem Stickstoffatom einen Ring der Reihe

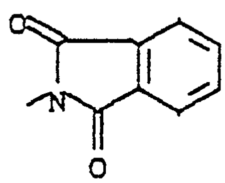 , 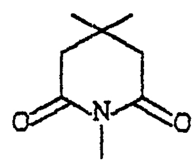 , 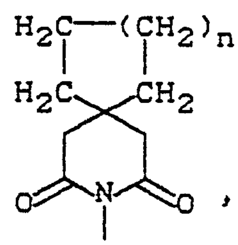 ,

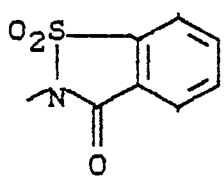 , 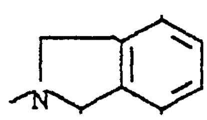 , 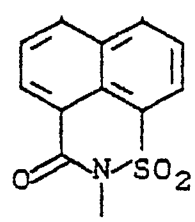 ,

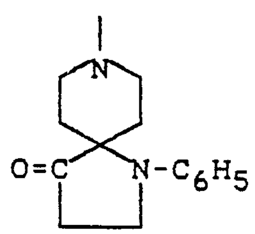 , 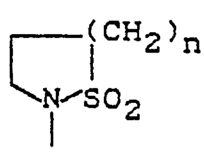 , 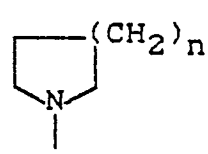

oder

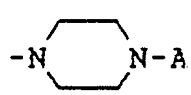 bilden,

worin

n - eine Zahl 1 oder 2 bedeutet.
und
A - für Wasserstoff oder Cycloalkyl steht, oder
-für Alkyl steht, das durch Halogen, Hydroxy, Amino, Alkylamino, Dialkylamino, Carbamoyl oder Sulfamoyl substituiert sein kann,
oder
-für Aryl, Heteroaryl, Aralkyl, Alkoxycarbonyl, Alkylsulfonyl, Phenylsulfonyl, Tolylsulfonyl, Benzylsulfonyl, Formyl, Carbamoyl oder Sulfamoyl steht
und deren Salze,
dadurch gekennzeichnet, daß man
8-Formyl-aminotetraline der allgemeinen Formel

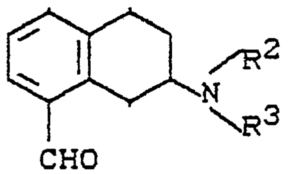

(Ie),

in welcher
$R^2$ und $R^3$ die oben angegebene Bedeutung haben,
in inerten Lösemitteln, gegebenenfalls in Anwesenheit von Kondensationsmitteln mit CH-aciden Verbindungen der allgemeinen Formel
$H_3C$-X' (XIII)
in welcher
X' -die für X angegebene Bedeutung hat, oder
-für Nitro steht,
umsetzt,
dann gegebenenfalls funktionelle Gruppen durch Reduktion, Hydrolyse, Oxidation oder Umsetzung mit elektrophilen Reagenzien in andere funktionelle Gruppen überführt
und dann im Fall der Herstellung der Salze mit den entsprechenden Säuren umsetzt.

14.Azneimitteln, enthaltend 8-substituierte 2-Aminotetraline der Formel

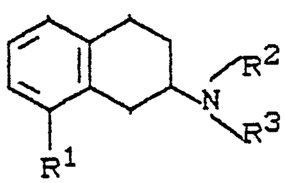

(I)

in welcher
$R^1$ -für Halogen, Cyano oder
-für eine Gruppe der Formel
$-NR^4R^5$, $-COR^6$, $-(CH_2)_a-X$, $-O-(CH_2)_a-X$ oder $-CH=CH-(CH_2)_b-X$ steht,
worin
$R^4$ und $R^5$ gleich oder verschieden sind und
-Wasserstoff oder
-eine Gruppe der Formel $-COR^7$ oder $-SO_2R^8$ bedeuten,
wobei
$R^7$ -für Wasserstoff steht, oder
-für eine Gruppe $-NHR^9$ steht, oder
-für Alkoxy steht, oder
-für Aryl, Aryloxy, Aralkyl, Aralkoxy oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
$R^8$ -für Cycloalkyl steht, oder
-für Alkyl steht, das durch Cyano, Halogen, Trifluormethyl, Trifluormethoxy oder Alkoxycarbonyl substituiert sein kann, oder

-für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluor methylthio, Amino, Alkylamino oder Dialkylamino substituiert sein können,
oder
-für eine Gruppe $-NR^{10}R^{11}$ steht,
worin
$R^{10}$ und $R^{11}$ gleich oder verschieden sind und Wasserstoff, Alkyl, Aryl oder Aralkyl bedeuten
und
$R^9$ -für Wasserstoff steht, oder
-für Cycloalkyl steht, oder
-für gegebenenfalls durch Cyano, Halogen, Trifluormethyl oder Trifluormethoxy substituiertes Alkyl steht, oder
-für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
$R^6$ -Wasserstoff, Hydroxy, Amino, Alkoxy, Aryloxy oder Aralkoxy bedeutet,
a - eine Zahl 1 bis 10 bedeutet,
b - eine Zahl 0 bis 8 bedeutet
und
X - eine Gruppe der Formel $-NR^{12}R^{13}$, $-COR^{14}$, $-SO_2R^{15}$ oder $-OR^{16}$ bedeutet,
worin
$R^{12}$ und $R^{13}$ gleich oder verschieden sind und
-für Wasserstoff, Alkyl, Aryl oder Aralkyl stehen, wobei die Arylreste durch Halogen, Cyano, Alkyl, Alkoxy oder Trifluormethyl substituiert sein können, oder
-für eine Gruppe der Formel $-COR^{14}$, $-SO_2R^{15}$ oder $-(CH_2)_c-NR^{12}R^{13}$ stehen,
$R^{14}$ -Wasserstoff bedeutet, oder
-eine Gruppe $-NHR^{17}$ bedeutet, oder
-Alkyl oder Alkoxy bedeutet, oder
-Aryl, Aryloxy, Aralkyl, Aralkoxy oder Heteroaryl bedeutet, wobei die genannten Reste bis zu 3-fach gleich oder verschieden durch Alkyl, Alkoxy, Alkylthio, Halogen, Cyano, Trifluormethylthio, Amino, Alkylamino oder Dialkylamino substituiert sein können,
$R^{15}$ -Cycloalkyl bedeutet, oder
-Alkyl bedeutet, das durch Cyano, Halogen, Trifluormethyl, Trifluormethoxy oder Alkoxycarbonyl substituiert sein kann, oder
-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
-eine Gruppe $-NR^{10}R^{11}$ bedeutet,
wobei
$R^{10}$ und $R^{11}$ die oben angegebene Bedeutung haben,
$R^{16}$ -Wasserstoff, Alkyl, Aryl, Aralkyl oder eine Gruppe $-CONR^{10}R^{11}$ bedeutet,
$R^{17}$ -Wasserstoff bedeutet, oder
-Cycloalkyl bedeutet oder
-gegebenenfalls durch Cyano, Halogen, Trifluormethyl oder Trifluormethoxy substituiertes Alkyl bedeutet, oder
-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluor methylthio, Amino, Alkylamino oder Dialkylamino substituiert sein können,
und
C - eine Zahl 1 bis 8 bedeutet,
oder wobei
$R^{12}$ und $R^{13}$ zusammen mit dem Stickstoffatom einen Ring der Reihe

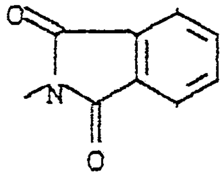,
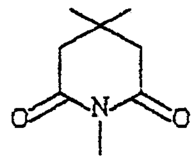,
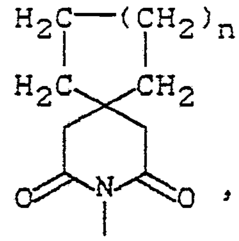,

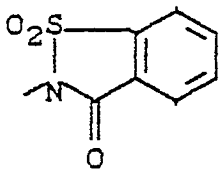,
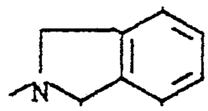,
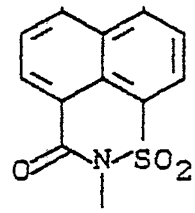,

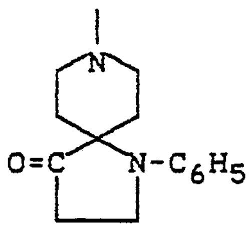,
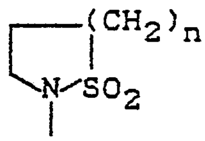,
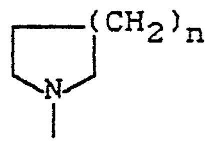

oder

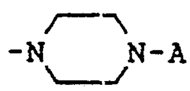

bilden,
worin
n - eine Zahl 1 oder 2 bedeutet
und
A - für Wasserstoff oder Cycloalkyl steht,
oder
-für Alkyl steht, das durch Halogen, Hydroxy, Amino, Alkylamino, Dialkylamino, Carbamoyl oder Sulfamoyl substituiert sein kann,
oder
-für Aryl, Heteroaryl, Aralkyl, Alkoxycarbonyl, Alkylsulfonyl, Phenylsulfonyl, Tolylsulfonyl, Benzylsulfonyl, Formyl, Carbamoyl oder Sulfamoyl steht,
$R^2$ -für Wasserstoff oder Alkyl steht
und
$R^3$ -für Alkyl steht,
wobei jedoch
$R^1$ nicht $NH_2$ bedeutet, wenn
$R^2$ und $R^3$ Propyl bedeuten
und/oder deren Salze.

15.Arzneimittel nach Anspruch 14, enthaltend 0,5 bis 90 Gew.-% der 8-substituierten 2-Aminotetraline und/oder deren Salze bezogen auf die Gesamtmenge.

16.Verwendung von 8-substituierten 2-Aminotetralinen der allgemeinen Formel

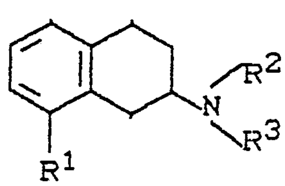

(I)

in welcher
$R^1$ -für Halogen, Cyano oder
-für eine Gruppe der Formel
$-NR^4R^5$, $-COR^6$, $-(CH_2)_a-X$, $-O-(CH_2)_a-X$ oder $-CH=CH-(CH_2)_b-X$ steht,
worin
$R^4$ und $R^5$ gleich oder verschieden sind und
-Wasserstoff oder
-eine Gruppe der Formel $-COR^7$ oder $-SO_2R^8$ bedeuten,
wobei
$R^7$ -für Wasserstoff steht, oder
-für eine Gruppe $-NHR^9$ steht, oder
-für Alkoxy steht, oder
-für Aryl, Aryloxy, Aralkyl, Aralkoxy oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
$R^9$ für Cycloalkyl steht, oder
-für Alkyl steht, das durch Cyano, Halogen, Trifluormethyl, Trifluormethoxy oder Alkoxycarbonyl substituiert sein kann, oder
-für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
-für eine Gruppe $-NR^{10}R^{11}$ steht,
worin
$R^{10}$ und $R^{11}$ gleich oder verschieden sind und Wasserstoff, Alkyl, Aryl oder Aralkyl bedeuten
und
$R^9$ -für Wasserstoff steht, oder
-für Cycloalkyl steht, oder
-für gegebenenfalls durch Cyano, Halogen, Trifluormethyl oder Trifluormethoxy substituiertes Alkyl steht, oder
-für Aryl, Aralkyl oder Heteroaryl steht, wobei die genannten Reste bis zu 3-fach gleich oder verschieden durch Alkyl,- Alkoxy, Alkylthio, Halogen, Cyano, Trifluormethyl, Trifluormethoxy, Trifluormethylthio, Amino,- Alkylamino oder Dialkylamino substituiert sein können,
$R^6$ -Wasserstoff, Hydroxy, Amino, Alkoxy, Aryloxy oder Aralkoxy bedeutet,
a - eine Zahl 1 bis 10 bedeutet,
b - eine Zahl 0 bis 8 bedeutet
und
X - eine Gruppe der Formel $-NR^{12}R^{13}$, $-COR^{14}$, $-SO_2R^{15}$ oder $-OR^{16}$ bedeutet,
worin
$R^{12}$ und $R^{13}$ gleich oder verschieden sind und
-für Wasserstoff, Alkyl, Aryl oder Aralkyl stehen, wobei die Arylreste durch Halogen, Cyano Alkyl, Alkoxy oder Trifluormethyl substituiert sein können, oder
-für eine Gruppe der Formel $-COR^{14}$, $-SO_2R^{15}$ oder $-(CH_2)_c-NR^{12}R^{13}$ stehen,
$R^{14}$ -Wasserstoff bedeutet, oder
-eine Gruppe $-NHR^{17}$ bedeutet, oder
-Alkyl oder Alkoxy bedeutet, oder
-Aryl, Aryloxy, Aralkyl, Aralkoxy oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
$R^{15}$ -Cycloalkyl bedeutet, oder
-Alkyl bedeutet, das durch Cyano, Halogen, Trifluormethyl, Trifluormethoxy oder Alkoxycarbonyl substituiert sein kann, oder
-Aryl, Aralkyl oder Heteroaryl bedeutet, wobei die genannten Reste bis zu 3-fach gleich oder verschieden durch Alkyl, Alkoxy, Alkylthio, Halogen, Cyano, Tri fluormethyl, Trifluormethoxy, Trifluormethylthio, Amino, Alkylamino oder Dialkylamino substituiert sein können, oder
-eine Gruppe $-NR^{10}R^{11}$ bedeutet,
wobei
$R^{10}$ und $R^{11}$ die oben angegebene Bedeutung haben,
$R^{16}$ - Wasserstoff, Alkyl, Aryl, Aralkyl oder die Gruppe $-CONR^{10}R^{11}$ bedeutet,

$R^{12}$ -Wasserstoff bedeutet, oder
-Cycloalkyl bedeutet, oder
-gegebenenfalls durch Cyano, Halogen, Trifluormethyl oder Trifluormethoxy substituiertes Alkyl bedeutet, oder
-Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
und
c - eine Zahl 1 bis 8 bedeutet,
oder wobei
$R^{12}$ und $R^{13}$ zusammen mit dem Stickstoffatom einen Ring der Reihe

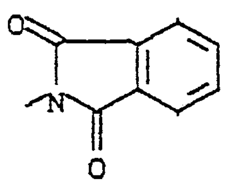 , 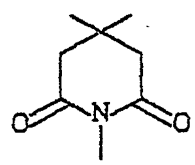 , 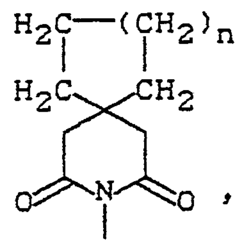 ,

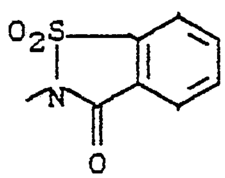 , 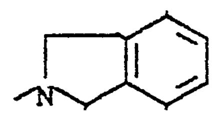 , 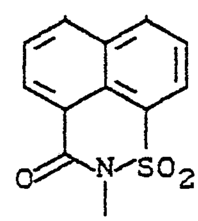 ,

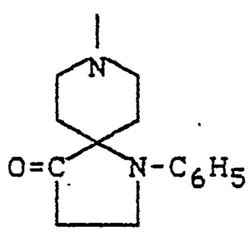 , 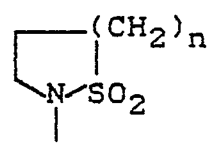 , 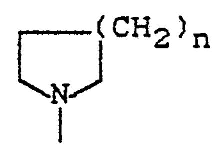

oder

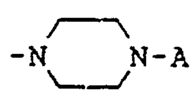

bilden,
worin
n - eine Zahl 1 oder 2 bedeutet
und
A - für Wasserstoff oder Cycloalkyl steht,
oder
-für Alkyl steht, das durch Halogen, Hydroxy, Amino, Alkylamino, Dialkylamino, Carbamoyl oder Sulfamoyl substituiert sein kann,
oder
-für Aryl, Heteroaryl, Aralkyl, Alkoxycarbonyl, Alkylsulfonyl, Phenylsulfonyl, Tolylsulfonyl, Benzylsulfonyl, Formyl, Carbamoyl oder Sulfamoyl steht,
$R^2$ -für Wasserstoff oder Alkyl steht
und
$R^3$ -für Alkyl steht,
wobei jedoch
$R^1$ nicht $NH_2$ bedeutet, wenn
$R^2$ und $R^3$ Propyl bedeuten

und/oder deren Salze zur Herstellung von Arzneimitteln.

17. Verwendung von 8-substituierten 2-Aminotetralinen nach Anspruch 16 zur Herstellung von Arzneimitteln zur Behandlung von Erkrankungen des Zentralnervensystems.

18. Verwendung von 8-substituierten 2-Aminotetralinen nach Anspruch 16 zur Herstellung von Arzneimitteln zur Modulierung des cardiovaskulären Systems.

19. Verwendung von 8-substituierten 2-Aminotetralinen nach Anspruch 16 zur Herstellung von Arzneimitteln zur Behandlung von Erkrankungen des Gastrointestinaltraktes.